(19) Europäisches Patentamt
European Patent Office
Office européen des brevets

(11) **EP 3 993 826 B1**

(12) **EUROPEAN PATENT SPECIFICATION**

(45) Date of publication and mention
of the grant of the patent:
**29.04.2026 Bulletin 2026/18**

(21) Application number: **20737269.9**

(22) Date of filing: **03.07.2020**

(51) International Patent Classification (IPC):
*A61K 40/11* (2025.01)      *A61K 40/17* (2025.01)
*A61K 40/24* (2025.01)      *A61K 40/32* (2025.01)
*A61K 40/34* (2025.01)      *A61K 40/42* (2025.01)
*A61P 17/00* (2006.01)      *A61P 35/00* (2006.01)
*C12N 5/0784* (2010.01)      *A61K 39/12* (2006.01)
*A61K 39/00* (2006.01)      *C12N 5/0783* (2010.01)

(52) Cooperative Patent Classification (CPC):
**A61K 39/12; A61K 40/11; A61K 40/17;
A61K 40/24; A61K 40/32; A61K 40/34;
A61K 40/4201; A61K 40/4271; A61P 17/00;
A61P 35/00; C12N 5/0638; C12N 5/0639;**
A61K 2039/53; A61K 2039/876; C12N 2740/00034

(86) International application number:
**PCT/GB2020/051592**

(87) International publication number:
**WO 2021/005338 (14.01.2021 Gazette 2021/02)**

(54) **NOVEL CANCER ANTIGENS AND METHODS**

NEUARTIGE KREBSANTIGENE UND VERFAHREN

NOUVEAUX ANTIGÈNES DU CANCER ET PROCÉDÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **05.07.2019 EP 19184680
17.04.2020 EP 20170224**

(43) Date of publication of application:
**11.05.2022 Bulletin 2022/19**

(73) Proprietors:
• **The Francis Crick Institute Limited**
**London NW1 1AT (GB)**
• **Enara Bio Limited**
**Oxford, OX4 4GA (GB)**

(72) Inventors:
• **KASSIOTIS, George**
**London NW1 1AT (GB)**
• **YOUNG, George**
**London NW1 1AT (GB)**

• **ATTIG, Jan**
**London NW1 1AT (GB)**
• **MARINO, Fabio**
**Oxford OX4 4GA (GB)**

(74) Representative: **Sagittarius IP
Marlow International
Parkway
Marlow SL7 1YL (GB)**

(56) References cited:
**WO-A1-2005/099750      WO-A2-2006/119527
US-A1- 2010 136 518**

• **P.F. FERRUCCI ET AL: "Newly Identified Tumor
Antigens as Promising Cancer Vaccine Targets
for Malignant Melanoma Treatment", CURRENT
TOPICS IN MEDICINAL CHEMISTRY, vol. 12, no.
1, 1 January 2012 (2012-01-01), NL, pages 11 - 31,
XP055652022, ISSN: 1568-0266, DOI: 10.2174/
156802612798919213**

- NORBERT BANNERT ET AL: "HERVs New Role in Cancer: From Accused Perpetrators to Cheerful Protectors", FRONTIERS IN MICROBIOLOGY, vol. 9, 13 February 2018 (2018-02-13), XP055652215, ISSN: 1664-302X, DOI: 10.3389/fmicb.2018.00178
- ESCUDIER BERNARD ET AL: "Vaccination of metastatic melanoma patients with autologous dendritic cell (DC) derived-exosomes: results of thefirst phase I clinical trial", JOURNAL OF TRANSLATIONAL MEDICINE, BIOMED CENTRAL, vol. 3, no. 1, 2 March 2005 (2005-03-02), pages 10, XP021009856, ISSN: 1479-5876, DOI: 10.1186/1479-5876-3-10
- WEIFAN YIN ET AL: "Immature Dendritic Cell-Derived Exosomes: a Promise Subcellular Vaccine for Autoimmunity", INFLAMMATION, KLUWER ACADEMIC PUBLISHERS-PLENUM PUBLISHERS, NE, vol. 36, no. 1, 7 September 2012 (2012-09-07), pages 232 - 240, XP035170032, ISSN: 1573-2576, DOI: 10.1007/S10753-012-9539-1

Remarks:

The complete document including Reference Table(s) and the Sequence Listing(s) can be downloaded from the EPO website

**Description**

**Field of the Invention**

The present invention relates to antigenic polypeptides and corresponding polynucleotides for use in the treatment or prevention of cancer, in particular for use in treating or preventing melanoma (e.g. cutaneous melanoma or uveal melanoma). The present invention further relates *inter alia* to pharmaceutical and immunogenic compositions comprising said nucleic acids and polypeptides, immune cells loaded with and/or stimulated by said polypeptides and polynucleotides, antibodies specific for said polypeptides and cells (autologous or otherwise) genetically engineered with molecules that recognize said polypeptides.

**Background of the invention**

As part of normal immunosurveillance for pathogenic microbes, all cells degrade intracellular proteins to produce peptides that are loaded onto Major Histocompatibility Complex (MHC) Class I molecules that are expressed on the surface of all cells. Most of these peptides, which are derived from the host cell, are recognized as self, and remain invisible to the adaptive immune system. However, peptides that are foreign (non-self), are capable of stimulating the expansion of naive CD8+ T cells that encode a T cell receptor (TCR) that tightly binds the MHC I-peptide complex. This expanded T cell population can produce effector CD8+ T cells (including cytotoxic T-lymphocytes - CTLs) that can eliminate the foreign antigen-tagged cells, as well as memory CD8+ T cells that can be re-amplified when the foreign antigen-tagged cells appear later in the animal's life.

MHC Class II molecules, whose expression is normally limited to professional antigen-presenting cells (APCs) such as dendritic cells (DCs), are usually loaded with peptides which have been internalised from the extracellular environment. Binding of a complementary TCR from a naive CD4+ T cell to the MHC II-peptide complex, in the presence of various factors, including T-cell adhesion molecules (CD54, CD48) and co-stimulatory molecules (CD40, CD80, CD86), induces the maturation of CD4+ T-cells into effector cells (e.g., $T_H1$, $T_H2$, $T_H17$, $T_{FH}$, $T_{reg}$ cells). These effector CD4+ T cells can promote B-cell differentiation to antibody-secreting plasma cells as well as facilitate the differentiation of antigen-specific CD8+ CTLs, thereby helping induce the adaptive immune response to foreign antigens, that include both short-term effector functions and longer-term immunological memory. DCs can perform the process of cross-presentation of peptide antigens by delivering exogenously-derived antigens (such as a peptide or protein released from a pathogen or a tumor cell) onto their MHC I molecules, contributing to the generation of immunological memory by providing an alternative pathway to stimulating the expansion of naïve CD8+ T-cells.

Immunological memory (specifically antigen-specific B cells/antibodies and antigen-specific CTLs) are critical players in controlling microbial infections, and immunological memory has been exploited to develop numerous vaccines that prevent the diseases caused by important pathogenic microbes. Immunological memory is also known to play a key role in controlling tumor formation, but very few efficacious cancer vaccines have been developed.

Cancer is the second leading cause of morbidity, accounting for nearly 1 in 6 of all deaths globally. Of the 8.8 million deaths caused by cancer in 2015, the cancers which claimed the most lives were from lung (1.69 million), liver (788,000), colorectal (774,000), stomach (754,000) and breast (571,000) carcinomas. The economic impact of cancer in 2010 was estimated to be USD1.16 Trillion, and the number of new cases is expected to rise by approximately 70% over the next two decades (World Health Organisation Cancer Facts 2017).

Current therapies for cutaneous melanoma are varied and are highly dependent on the location of the tumor and stage of the disease. The main treatment for a non-metastatic melanoma is surgery to remove the tumor and surrounding tissue. Later stage melanomas may require treatment comprising lymph node dissection, radiotherapy, or chemotherapy. Immune checkpoint blockade strategies, including the use of antibodies targeting negative immune regulators such as PD-1/PD-L1 and CTLA4, have recently revolutionised treatments to a variety of malignancies, including melanoma (Ribas, A., & Wolchok, J. D. (2018) Science, 359:1350-1355.). The extraordinary value of checkpoint blockade therapies, and the well-recognized association of their clinical benefit with patient's adaptive immune responses (specifically T cell based immune responses) to their own cancer antigens has reinvigorated the search for effective cancer vaccines, vaccine modalities, and cancer vaccine antigens.

Human endogenous retroviruses (HERVs) are remnants of ancestral germline integrations of exogenous infectious retroviruses. HERVs belong to the group of endogenous retroelements that are characterised by the presence of Long Terminal Repeats (LTRs) flanking the viral genome. This group also includes the Mammalian apparent LTR Retrotransposons (MaLRs) and are therefore collectively known as LTR elements (here referred to collectively as ERV to mean all LTR elements). ERVs constitute a considerable proportion of the mammalian genome (8%), and can be grouped into approximately 100 families based on sequence homology. Many ERV sequences encode defective proviruses which share the prototypical retroviral genomic structure consisting of *gag*, *pro*, *pol* and *env* genes flanked by LTRs. Some intact ERV ORFs produce retroviral proteins which share features with proteins encoded by exogenous infectious retroviruses such as HIV-1. Such proteins may serve as antigens to induce a potent immune response (Hurst & Magiorkinis, 2015, J. Gen. Virol 96:1207-1218), suggesting that polypeptides encoded by ERVs can escape T and B-cell receptor selection processes and central and peripheral tolerance. Immune reactivity to ERV products may occur spontaneously in infection or cancer, and ERV products have been implicated as a cause of some autoimmune diseases (Kassiotis & Stoye, 2016, Nat. Rev. Immunol. 16:207-219).

Due to the accumulation of mutations and recombination events during evolution, most ERVs have lost functional open reading frames for some or all of their genes and therefore their ability to produce infectious virus. However, these ERV elements are maintained in germline DNA like other genes and still have the potential to produce proteins from at least some of their genes. Indeed, HERV-encoded proteins have been detected in a variety of human cancers. For example, splice variants of the HERV-K env gene, Rec and Np9, are found exclusively in malignant testicular germ cells and not in healthy cells (Ruprecht et. al, 2008, Cell Mol Life Sci 65:3366-3382). Increased levels of HERV transcripts have also been observed in cancers such as those of the prostate, as compared to healthy tissue (Wang-Johanning, 2003, Cancer 98:187-197; Andersson et al., 1998, Int. J. Oncol, 12:309-313). Additionally, overexpression of HERV-E and HERV-H has been demonstrated to be immunosuppressive, which could also contribute to the development of cancer (Mangeney et al., 2001, J. Gen. Virol. 82:2515-2518). However, the exact mechanism(s) by which HERVs could contribute to the development or pathogenicity of cancer remains unknown.

In addition to deregulating the expression of surrounding neighbouring host genes, the activity and transposition of ERV regulatory elements to new genomic sites may lead to the production of novel transcripts, some of which may have oncogenic properties (Babaian & Mager, Mob. DNA, 2016, , Lock et al., PNAS, 2014, 111:3534-3543).

A wide range of vaccine modalities are known. One well-described approach involves directly delivering an antigenic polypeptide to a subject with a view to raising an immune response (including B- and T-cell responses) and stimulating immunological memory. Alternatively, a polynucleotide may be administered to the subject by means of a vector such that the polynucleotide-encoded immunogenic polypeptide is expressed in vivo. The use of viral vectors, for example adenovirus vectors, has been well explored for the delivery of antigens in both prophylactic vaccination and therapeutic treatment strategies against cancer (Wold et al. Current Gene Therapy, 2013, Adenovirus Vectors for Gene Therapy, Vaccination and Cancer Gene Therapy, 13:421-433). Immunogenic peptides, polypeptides, or polynucleotides encoding them, can also be used to load patient-derived antigen presenting cells (APCs), that can then be infused into the subject as a vaccine that elicits a therapeutic or prophylactic immune response. An example of this approach is Provenge, which is presently the only FDA-approved anti-cancer vaccine.

Cancer antigens, may also be exploited in the treatment and prevention of cancer by using them to create a variety of non-vaccine therapeutic modalities. These therapies fall into two different classes: 1) antigen-binding biologics, 2) adoptive cell therapies.

Antigen-binding biologics typically consist of multivalent engineered polypeptides that recognize antigen-decorated cancer cells and facilitate their destruction. The antigen-binding components of these biologics may consist of TCR-based biologicals, including, but not limited to TCRs, high-affinity TCRs, and TCR mimetics produced by various technologies (including those based on monoclonal antibody technologies). Cytolytic moieties of these types of multivalent biologics may consist of cytotoxic chemicals, biological toxins, targeting motifs and/or immune stimulating motifs that facilitate targeting and activation of immune cells, any of which facilitate the therapeutic destruction of tumor cells.

Adoptive cell therapies may be based on a patient's own T cells that are removed and stimulated ex vivo with vaccine antigen preparations (cultivated with T cells in the presence or absence of other factors, including cellular and acellular components) (Yossef et al., JCI Insight. 2018 Oct 4;3(19). pii: 122467. doi: 10.1172/jci.insight.122467). Alternatively, adoptive cell therapies can be based on cells (including patient- or non-patient-derived cells) that have been deliberately engineered to express antigen-binding polypeptides that recognize cancer antigens. These antigen-binding polypeptides fall into the same classes as those described above for antigen-binding biologics. Thus, lymphocytes (autologous or non-autologous), that have been genetically manipulated to express cancer antigen-binding polypeptides can be administered to a patient as adoptive cell therapies to treat their cancer.

Use of ERV-derived antigens in raising an effective immune response to cancer has shown promising results in promoting tumor regression and a more favourable prognosis in murine models of cancer (Kershaw et al., 2001, Cancer Res. 61:7920-7924; Slansky et al., 2000, Immunity 13:529-538). Thus, HERV antigen-centric immunotherapy trials have been contemplated in humans (Sacha et al.,2012, J.Immunol 189:1467-1479), although progress has been restricted, in part, due to a severe limitation of identified tumor-specific ERV antigens.

WO 2005/099750 identifies anchored sequences in existing vaccines against infectious pathogens, which are common in raising cross-reactive immune responses against the HERV-K Mel tumor antigen and confers protection to melanoma.

WO 00/06598 relates to the identification of HERV-AVL3-B tumor associated genes which are preferentially expressed in melanomas, and methods and products for diagnosing and treating conditions characterised by expression of said genes.

WO 2006/119527 provides antigenic polypeptides derived from the melanoma-associated endogenous retrovirus (MERV), and their use for the detection and diagnosis of melanoma as well as prognosis of the disease. The use of antigenic polypeptides as anticancer vaccines is also disclosed.

WO 2007/137279 discloses methods and compositions for detecting, preventing and treating HERV-K+ cancers, for example with use of a HERV-K+ binding antibody to prevent or inhibit cancer cell proliferation.

WO 2006/103562 discloses a method for treating or preventing cancers in which the immunosuppressive Np9 protein from the env gene of HERV-K is expressed. The invention also relates to pharmaceutical compositions comprising nucleic acid or antibodies capable of inhibiting the activity of said protein, or immunogen or vaccinal composition capable of inducing an immune response directed against said protein.

WO 2007/109583 provides compositions and methods for preventing or treating neoplastic disease in a mammalian subject, by providing a composition comprising an enriched immune cell population reactive to a HERV-E antigen on a tumor cell.

Humer J, et al., 2006, Canc. Res., 66:1658-63 identifies a melanoma marker derived from melanoma-associated endogenous retroviruses.

Ferrucci et al., 2012, Curr. Top. Med. Chem., 12(1):11-31 describes antigen-targeted therapy strategies, mainly directed to Cancer Testis and Heat Shock Proteins, leading to a possible active immunization against melanoma through T-cell specific activation

Bannert et al., 2018 Front. Microbiol., 9:178 discusses the role of HERV reactivation in terms of its implications for cancer.

Escudier et al., 2005 J. Transl. Med., 3:10 describes a Phase I clinical trial using autologous exosomes pulsed with MAGE 3 peptides for the immunization of stage III/IV melanoma patients.

Yin et al., 2013, Inflammation. 36(1):232-40 discusses immature DC-derived exosomes as a subcellular vaccine in autoimmunity

US 2010/136518 discloses antibodies, or fragments thereof, for isolating and/or identifying epitopes of an endogenous retrovirus, preferably of a melanoma associated endogenous retrovirus, and hybridoma cells producing said antibodies

There is a need to identify further HERV-associated antigenic sequences which can be used in immunotherapy of cancer, particularly melanoma, especially cutaneous and uveal melanoma.

## Summary of the Invention

The inventors have surprisingly discovered certain RNA transcripts which comprise LTR elements which are found at high levels in cutaneous melanoma cells, but are undetectable or found at very low levels in normal, healthy tissues (see Example 1). Such transcripts are herein referred to as cancer-specific LTR-element spanning transcripts (CLTs). Further, the inventors have shown that a subset of the potential polypeptide sequences (i.e., open reading frames (ORFs)) encoded by these CLTs are translated in cancer cells, processed by components of the antigen-processing apparatus, and presented on the surface of cells found in tumor tissue in association with the class I human leukocyte antigen (HLA Class I) molecules (see Example 2). These findings demonstrate that these polypeptides (herein referred to as CLT antigens) are, *ipso facto*, antigenic. Thus, cancer cell presentation of CLT antigens is expected to render these cells susceptible to elimination by T cells that bear cognate T cell receptors (TCRs) for the CLT antigens, and CLT antigen-based vaccination methods/regimens that amplify T cells bearing these cognate TCRs are expected to elicit immune responses against cancer cells (and tumors containing them), particularly melanoma particularly cutaneous melanoma tumors. T-cells from melanoma subjects are indeed reactive to peptides derived from CLT antigens disclosed herein (see Example 3). The inventors have confirmed that T-cells specific for CLT antigens have not been deleted from normal subject's T-cell repertoire by central tolerance (see Example 4). Finally, qRT-PCR studies have confirmed that CLTs are specifically expressed in RNA extracted from melanoma tumour tissue as compared to a non-melanoma control cell line (see Example 5).

The inventors have also surprisingly discovered that certain CLT antigen-encoding CLTs as well as being overexpressed in cutaneous melanoma are also overexpressed in uveal melanoma. The CLT antigen polypeptide sequences encoded by these CLTs are expected to elicit immune responses against uveal melanoma cells and tumors containing them.

The CLTs and the CLT antigens that are the subject of the present invention are not canonical sequences which can be readily derived from known tumor genome sequences found in the cancer genome atlas. The CLTs are transcripts resulting from complex transcription and splicing events driven by transcription control sequences of ERV origin. Since the CLTs are expressed at high level and since CLT antigen polypeptide sequences are not sequences of normal human proteins, it is expected that they will be capable of eliciting strong, specific immune responses and thus suitable for therapeutic use in a cancer immunotherapy setting.

The CLT antigens discovered in the highly expressed transcripts that characterize tumor cells, which prior to the present invention were not known to exist and produce protein products in man, can be used in several formats. First, CLT antigen polypeptides of the invention can be directly delivered to a subject as a vaccine that elicits a therapeutic or prophylactic immune response to tumor cells. Second, nucleic acids of the invention, which may be codon optimised to enhance the expression of their encoded CLT antigens, can be directly administered or else inserted into vectors for delivery *in vivo* to produce the encoded protein products in a subject as a vaccine that elicits a therapeutic or prophylactic immune response to tumor cells. Third, polynucleotides and/or polypeptides of the invention can be used to load patient-derived antigen presenting cells (APCs), that can then be infused into the subject as a vaccine that elicits a therapeutic or prophylactic immune response to tumor cells. Fourth, polynucleotides and/or polypeptides of the invention can be used for *ex vivo* stimulation of a subject's T cells, producing a stimulated T cell preparation that can be administered to a subject as a therapy to treat cancer. Fifth, biological molecules such as T cell receptors (TCRs) or TCR mimetics that recognize CLT antigens complexed to MHC I molecules and have been further modified to permit them to kill (or facilitate killing) of cancer cells may be administered to a subject as a therapy to treat cancer. Sixth, chimeric versions of biological molecules that recognize CLT antigens complexed to MHC cells may be introduced into T cells (autologous our non-autologous), and the resulting cells may be administered to a subject as a therapy to treat cancer. These and other applications are described in greater detail below.

Thus, the invention provides *inter alia* an isolated polypeptide comprising a sequence selected from:

(a) the sequence of SEQ ID NO. 2; and
(b) an immunogenic variant of the sequence of (a) which is at least 80% identical to SEQ ID NO. 2; and
(c) an immunogenic fragment of the sequence of (a) comprising at least 9 contiguous amino acids of SEQ ID NO. 2
(hereinafter referred to as "a polypeptide of the invention").

The invention also provides a nucleic acid molecule which encodes a polypeptide of the invention (hereinafter referred to as "a nucleic acid of the invention").

The polypeptides of the invention and the nucleic acids of the invention, as well as related aspects of the invention, are expected to be useful in a range of embodiments in cancer immunotherapy and prophylaxis, particularly immunotherapy and prophylaxis of melanoma, as discussed in more detail below.

## Description of the Figures

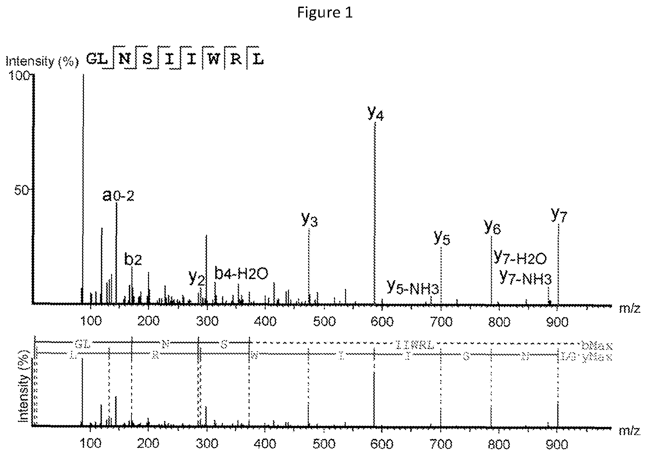

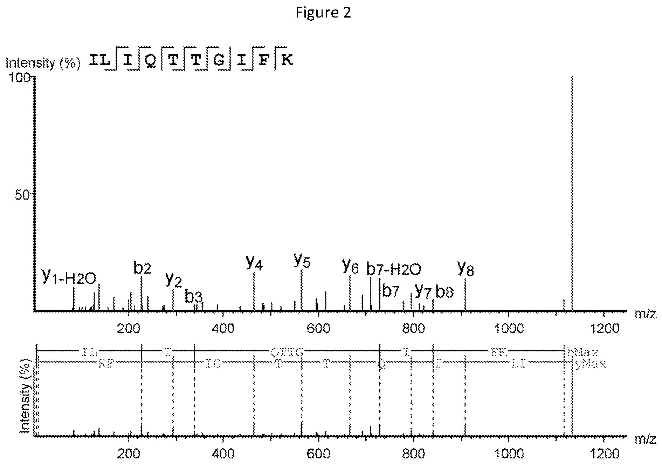

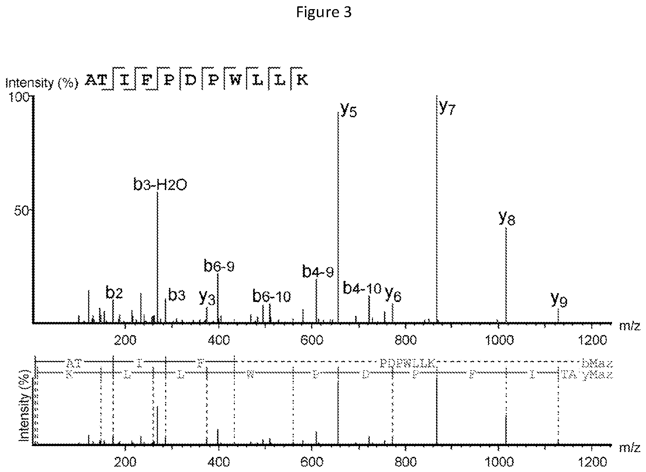

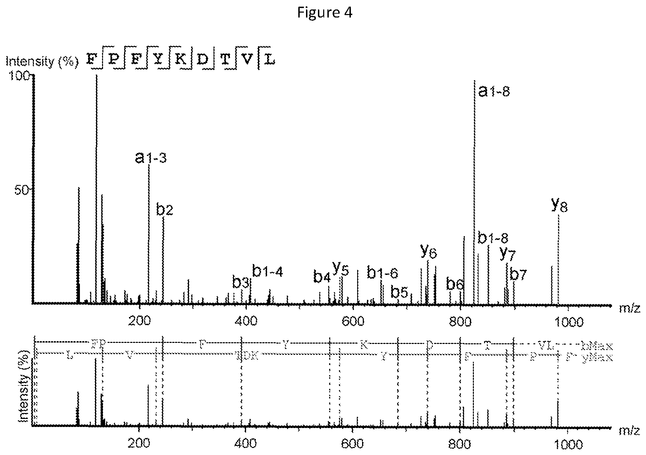

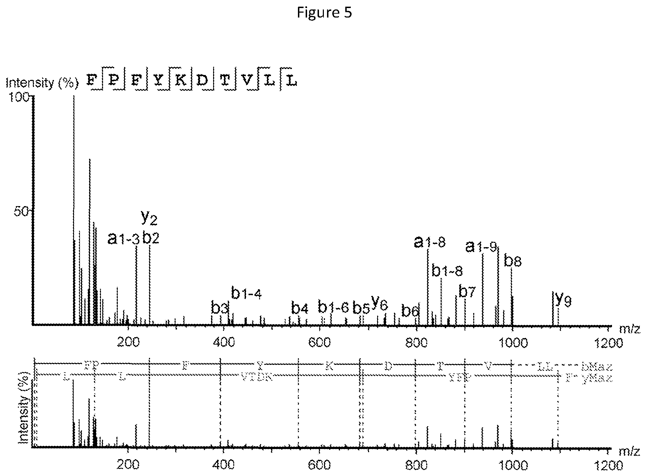

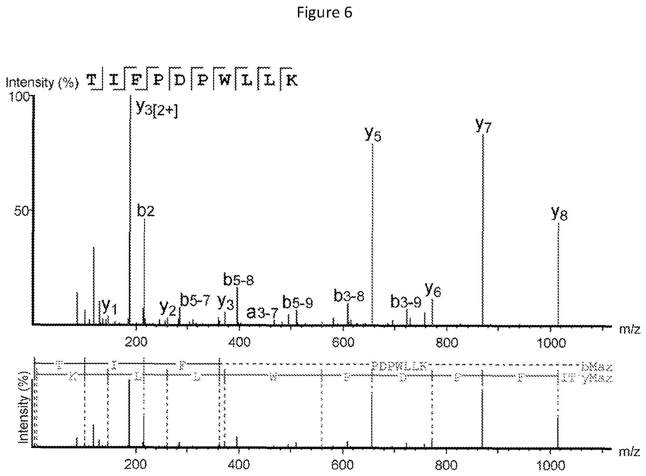

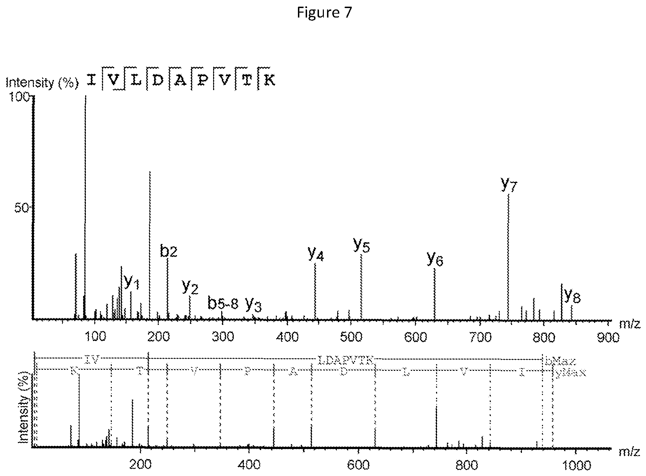

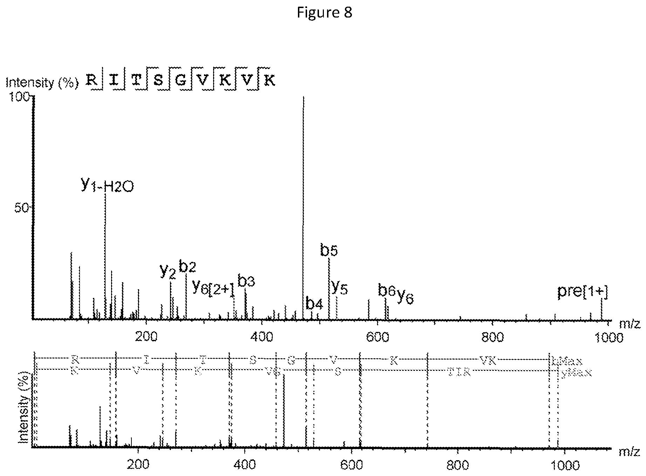

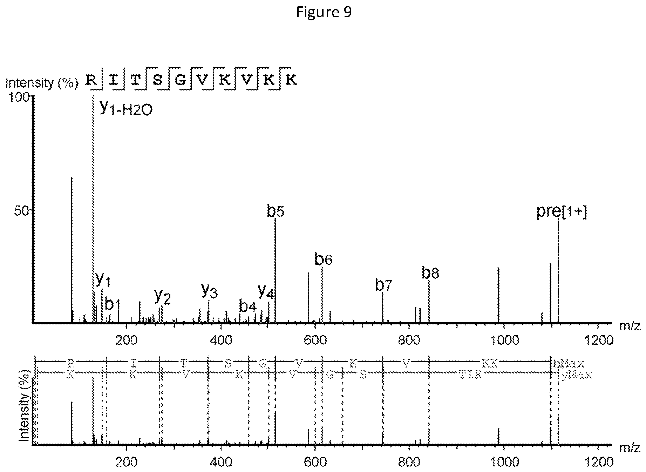

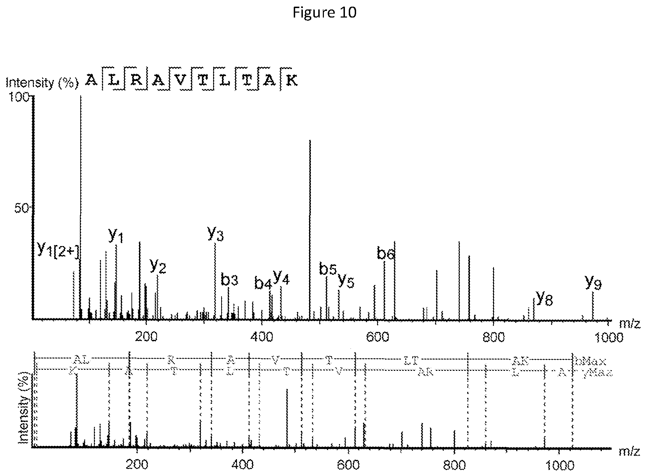

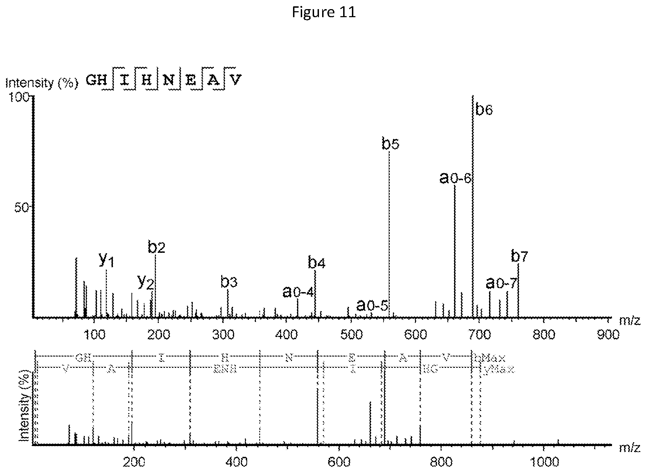

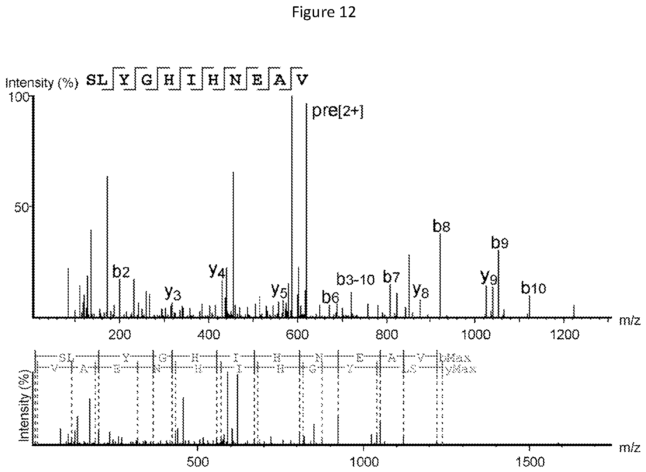

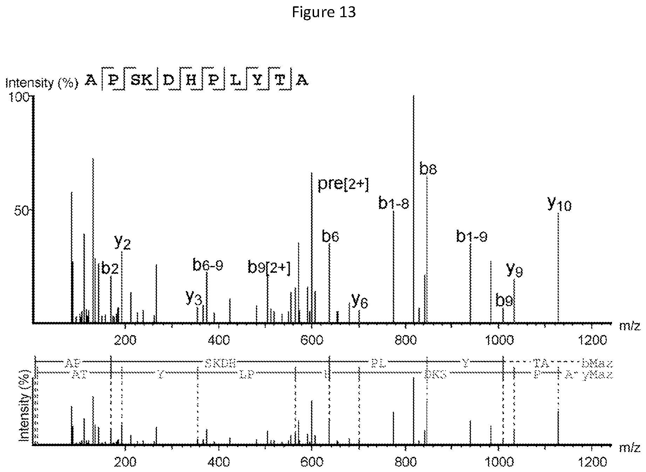

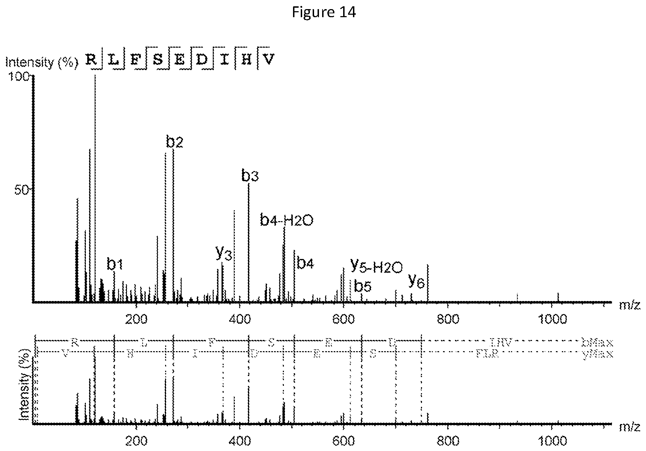

For each of Figures 1-14, the top panel shows an extracted MS/MS spectrum (with assigned fragment ions) of a peptide obtained from a tumor sample of a patient and the bottom panel shows a rendering of the spectrum indicating the positions of the linear peptide sequences that have been mapped to the fragment ions.

Figure 1. Spectra for the peptide of SEQ ID NO. 9 obtained from a tumor sample of patient Mel-27.
Figure 2. Spectra for the peptide of SEQ ID NO. 10 obtained from a tumor sample of patient Mel-21.
Figure 3. Spectra for the peptide of SEQ ID NO. 11 obtained from a tumor sample of patient Mel-41.
Figure 4. Spectra for the peptide of SEQ ID NO. 12 obtained from a tumor sample of patient Mel-41.
Figure 5. Spectra for the peptide of SEQ ID NO. 13 obtained from a tumor sample of patient Mel-41.
Figure 6. Spectra for the peptide of SEQ ID NO. 14 obtained from a tumor sample of patient Mel-41.
Figure 7. Spectra for the peptide of SEQ ID NO. 15 obtained from a tumor sample of patient Mel-21.
Figure 8. Spectra for the peptide of SEQ ID NO. 16 obtained from a tumor sample of patient Mel-21.
Figure 9. Spectra for the peptide of SEQ ID NO. 17 obtained from a tumor sample of patient Mel-21.
Figure 10. Spectra for the peptide of SEQ ID NO. 18 obtained from a tumor sample of patient Mel-15.
Figure 11. Spectra for the peptide of SEQ ID NO. 19 obtained from a tumor sample of patient Mel-27.
Figure 12. Spectra for the peptide of SEQ ID NO. 20 obtained from a tumor sample of patient Mel-27.
Figure 13. Spectra for the peptide of SEQ ID NO. 21 obtained from a tumor sample of patient Mel-25.
Figure 14. Spectra for the peptide of SEQ ID NO. 22 obtained from a tumor sample of patient Mel-25.

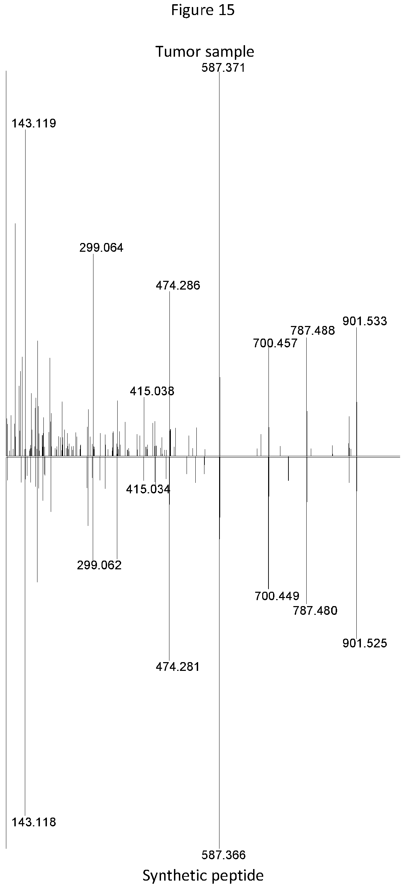

Each of Figures 15-28 shows an alignment of a native MS/MS spectrum of a peptide obtained from a patient tumor sample to the native spectrum of a synthetic peptide corresponding to the same sequence.

Figure 15. Spectra for the peptide of SEQ ID NO. 9 obtained from a tumor sample of patient Mel-27.

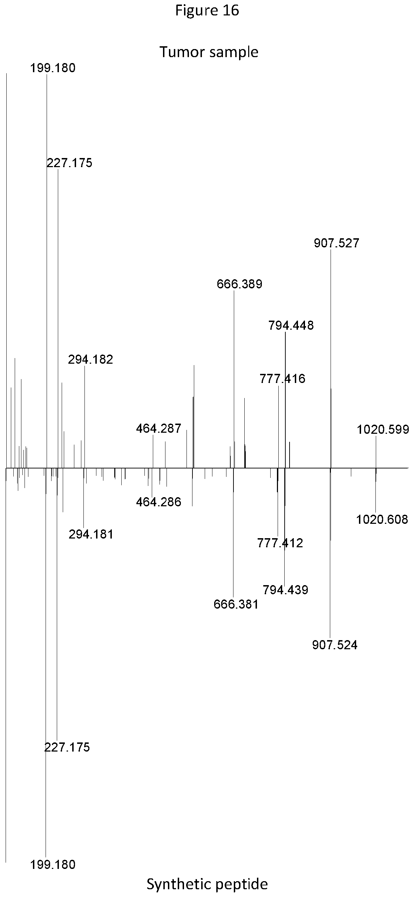

Figure 16. Spectra for the peptide of SEQ ID NO. 10 obtained from a tumor sample of patient Mel-20.

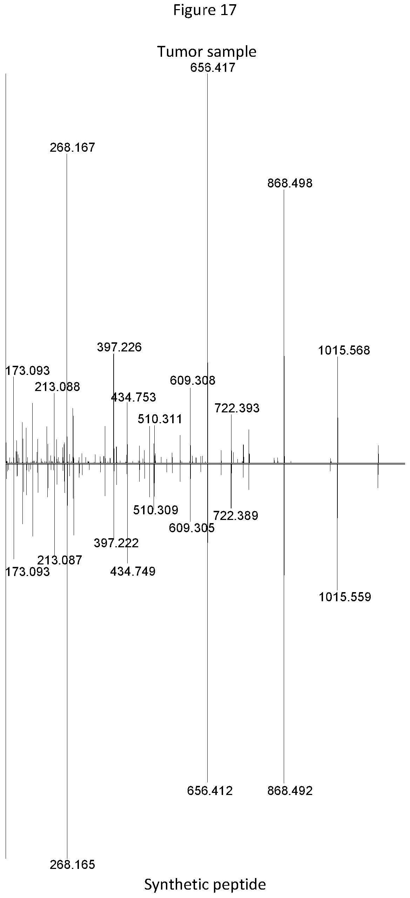

Figure 17. Spectra for the peptide of SEQ ID NO. 11 obtained from a tumor sample of patient Mel-41.

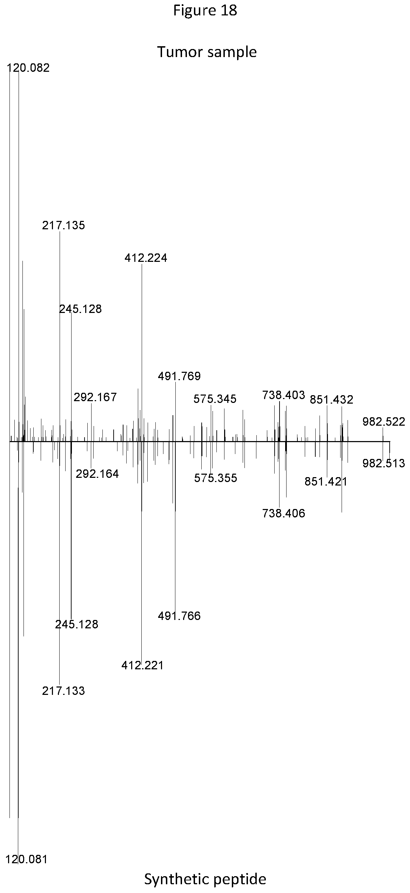

Figure 18. Spectra for the peptide of SEQ ID NO. 12 obtained from a tumor sample of patient Mel-41.

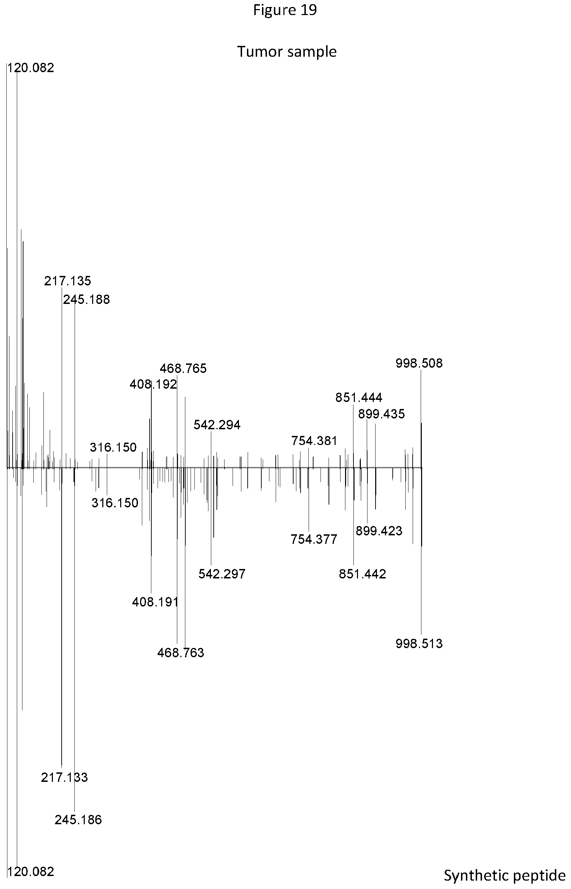

Figure 19. Spectra for the peptide of SEQ ID NO. 13 obtained from a tumor sample of patient Mel-41.

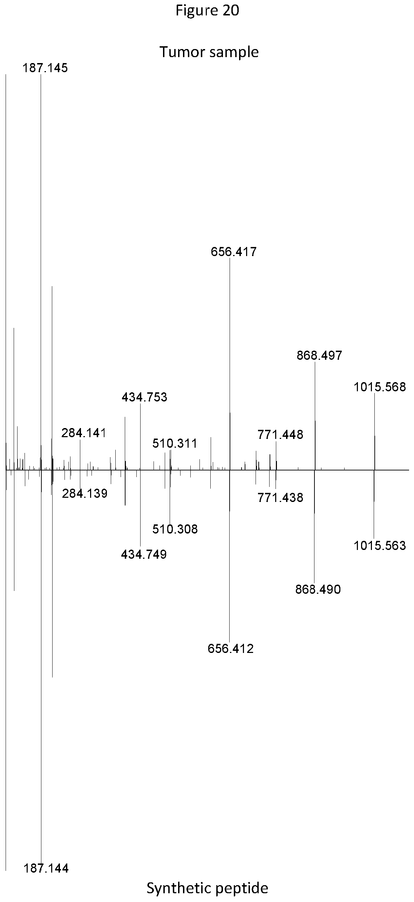

Figure 20. Spectra for the peptide of SEQ ID NO. 14 obtained from a tumor sample of patient Mel-41.

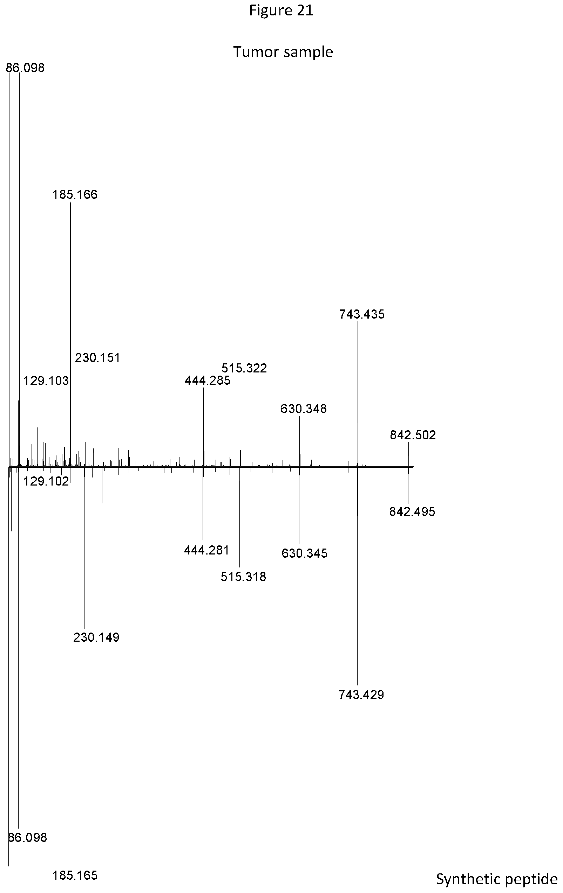

Figure 21. Spectra for the peptide of SEQ ID NO. 15 obtained from a tumor sample of patient Mel-21.

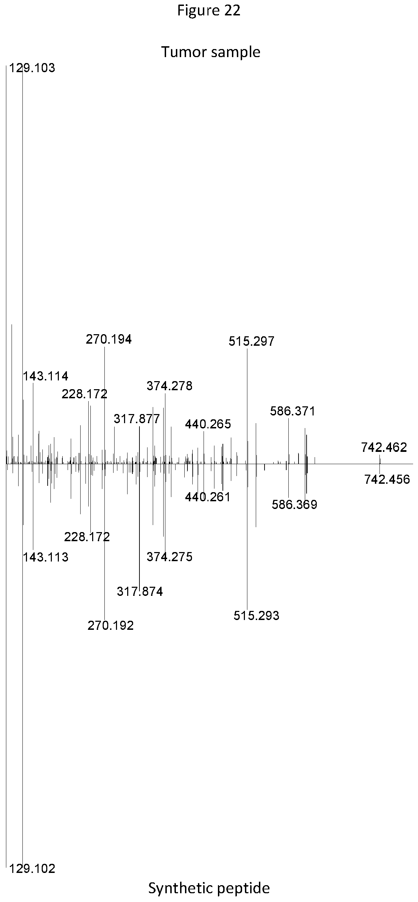

Figure 22. Spectra for the peptide of SEQ ID NO. 16 obtained from a tumor sample of patient Mel-21.

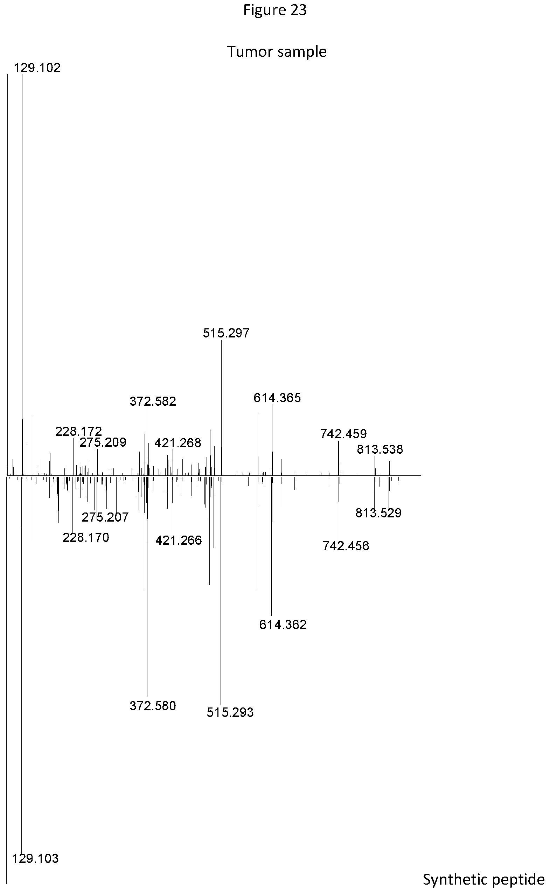

Figure 23. Spectra for the peptide of SEQ ID NO. 17 obtained from a tumor sample of patient Mel-21.

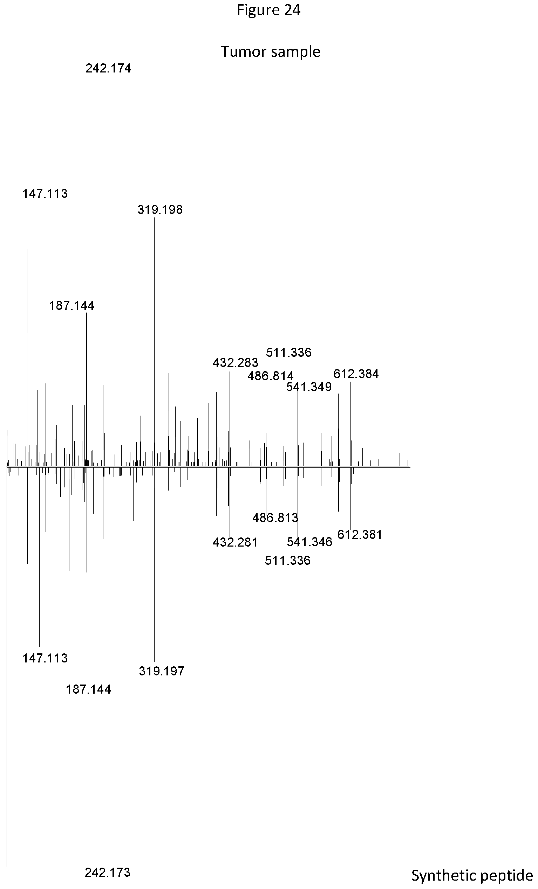

Figure 24. Spectra for the peptide of SEQ ID NO. 18 obtained from a tumor sample of patient Mel-15.

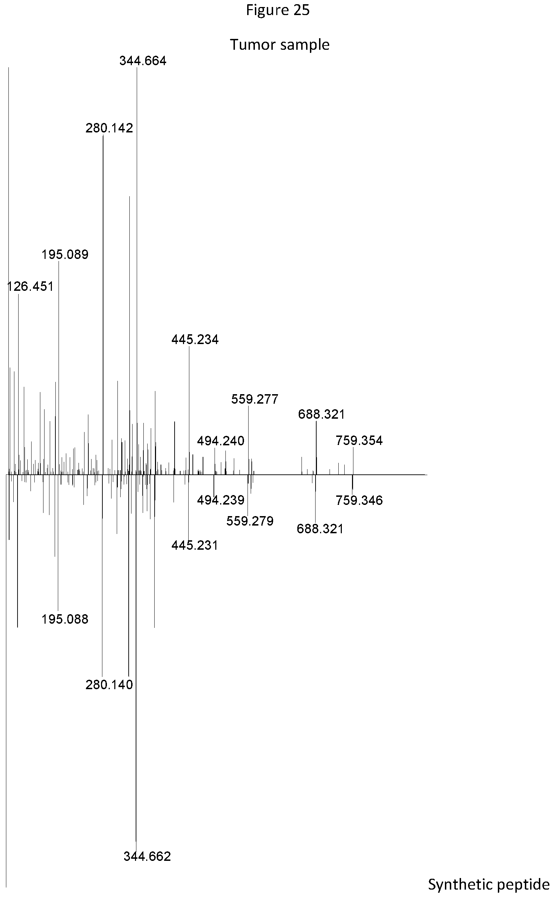

Figure 25. Spectra for the peptide of SEQ ID NO. 19 obtained from a tumor sample of patient Mel-27.

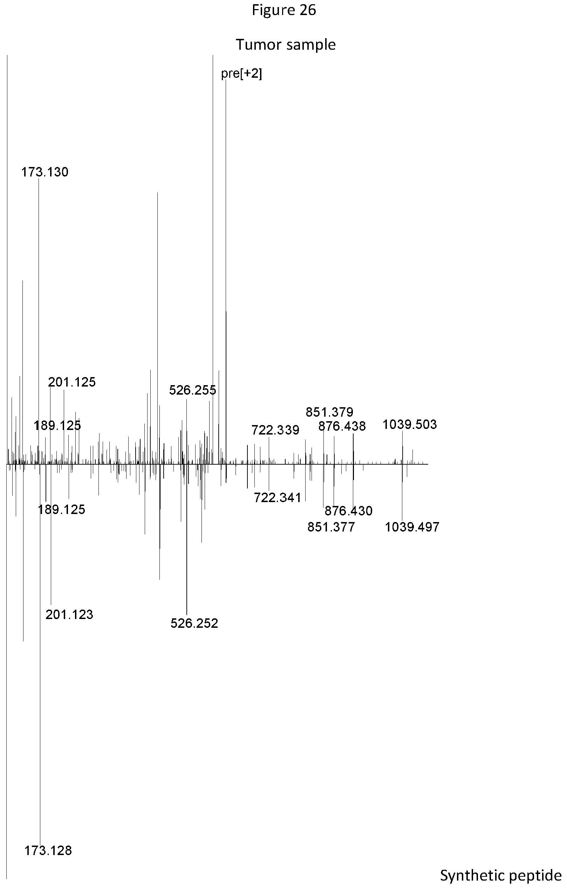

Figure 26. Spectra for the peptide of SEQ ID NO. 20 obtained from a tumor sample of patient Mel-27.

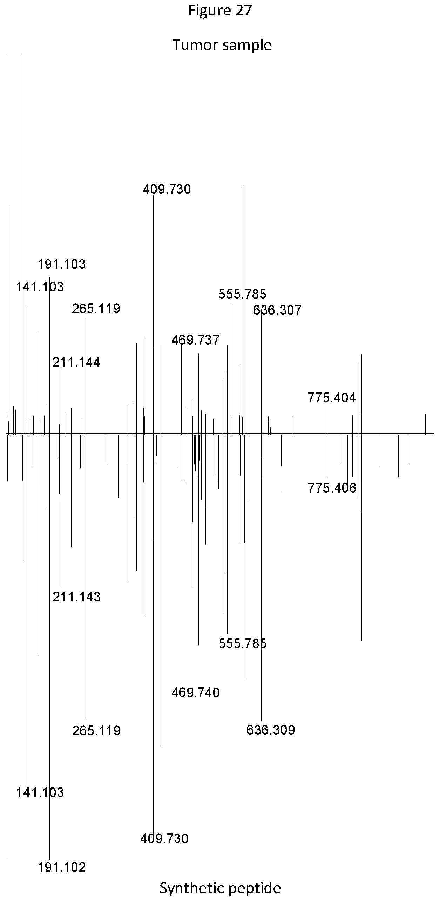

Figure 27. Spectra for the peptide of SEQ ID NO. 21 obtained from a tumor sample of patient Mel-25.

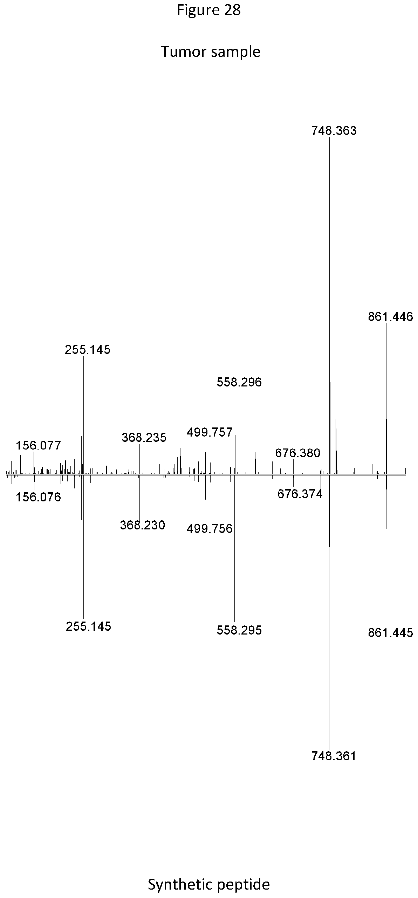

Figure 28. Spectra for the peptide of SEQ ID NO. 22 obtained from a tumor sample of patient Mel-25.

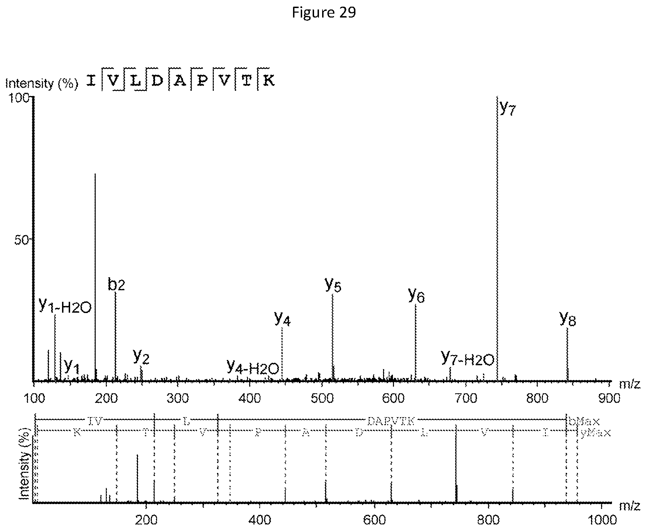

Figure 29. Spectra for the peptide of SEQ ID NO. 15 obtained from a tumor sample of patient 2MT3.

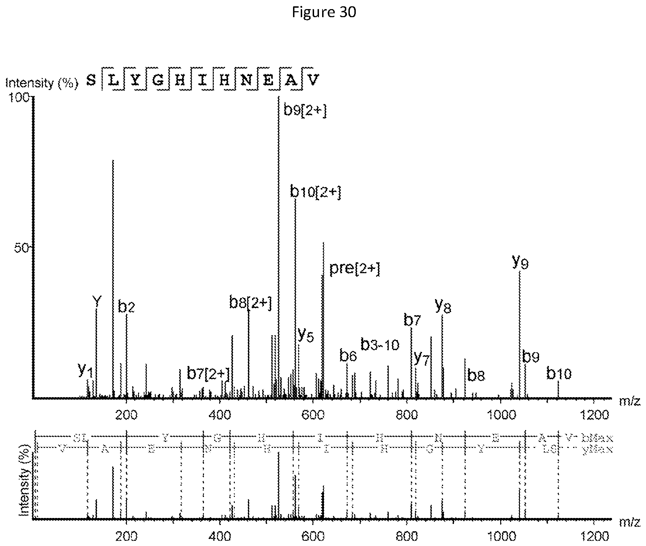

Figure 30. Spectra for the peptide of SEQ ID NO. 20 obtained from a tumor sample of patient 2MT4.

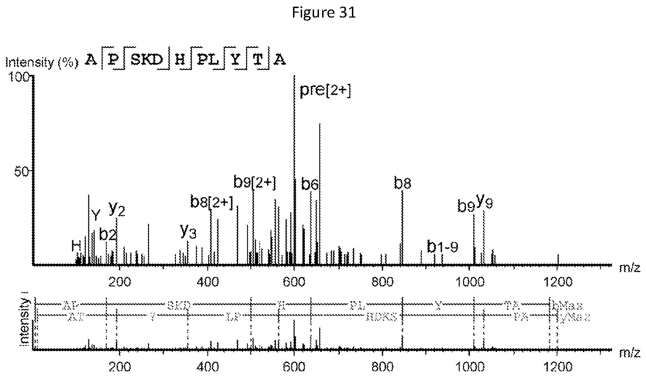

Figure 31. Spectra for the peptide of SEQ ID NO. 21 obtained from a tumor sample of patient 2MT4.

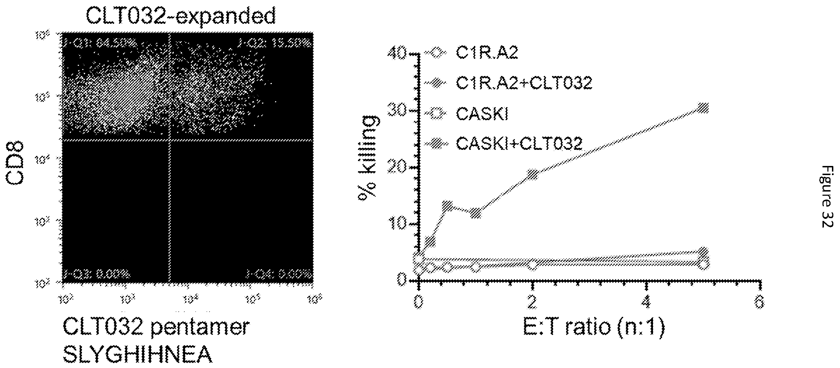

Figure 32 shows expanded, pentamer-sorted CD8 T-cells killing of CaSki cells transfected with the open reading frame of CLT Antigen 6 (SEQ ID NO.6).

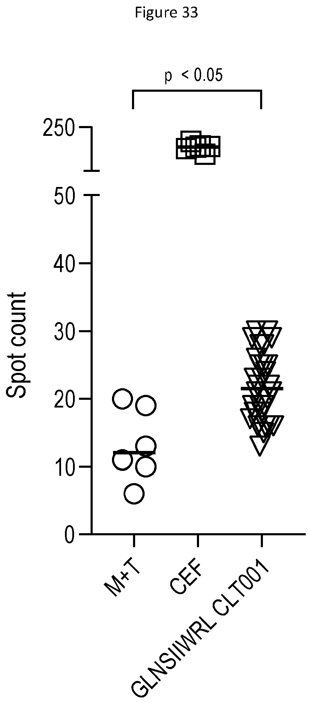

Figure 33 shows CD8 T-cell responses from a normal blood donor to HLA-A *02:01-restricted peptide (SEQ ID NO. 9) from CLT Antigen 1.

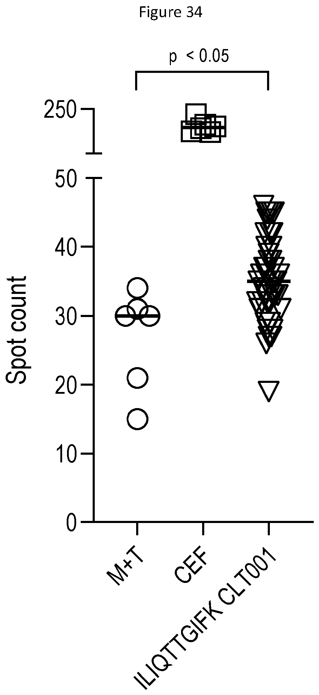

Figure 34 shows CD8 T-cell responses from a normal blood donor to HLA-A *03:01-restricted peptide (SEQ ID NO. 10) from CLT Antigen 1.

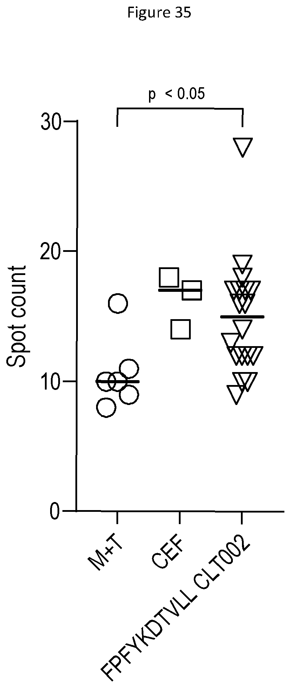

Figure 35 shows CD8 T-cell responses from a normal blood donor to HLA-B *07:02-restricted peptide (SEQ ID NO. 13) from CLT Antigen 2.

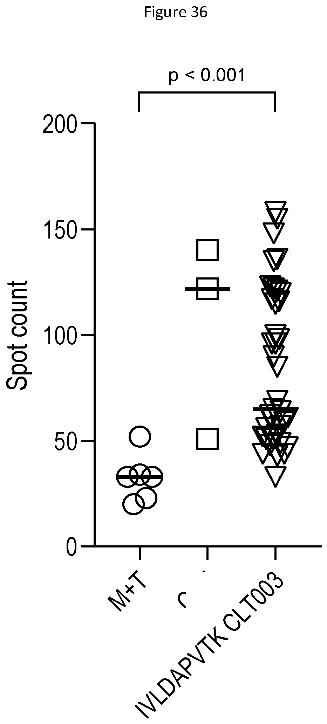

Figure 36 shows CD8 T-cell responses from a normal blood donor to HLA-A*03:01-restricted peptide (SEQ ID NO. 15) from CLT Antigen 3.

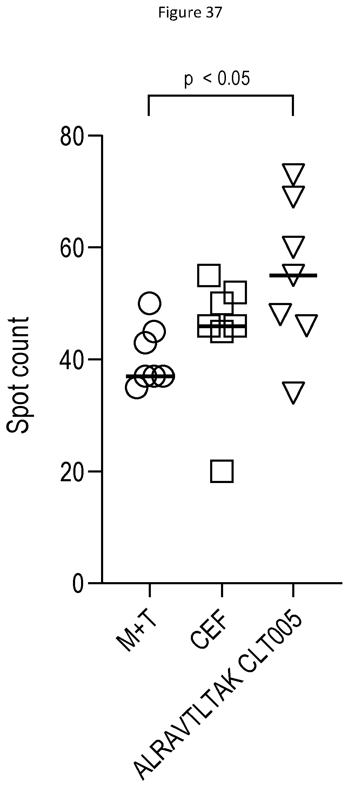

Figure 37 shows CD8 T-cell responses from a normal blood donor to HLA-A*03:01-restricted peptide (SEQ ID NO. 18) from CLT Antigen 5.

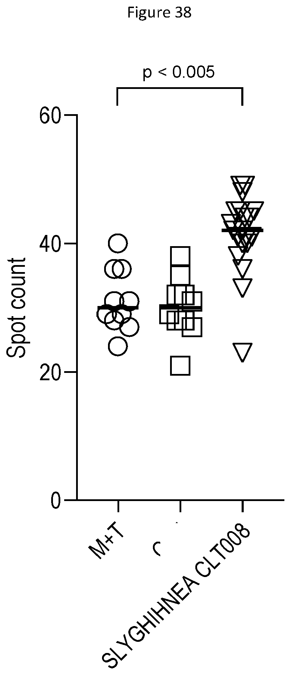

Figure 38 shows CD8 T-cell responses from a normal blood donor to HLA-A*02:01-restricted peptide (SEQ ID NO. 39) from CLT Antigen 6.

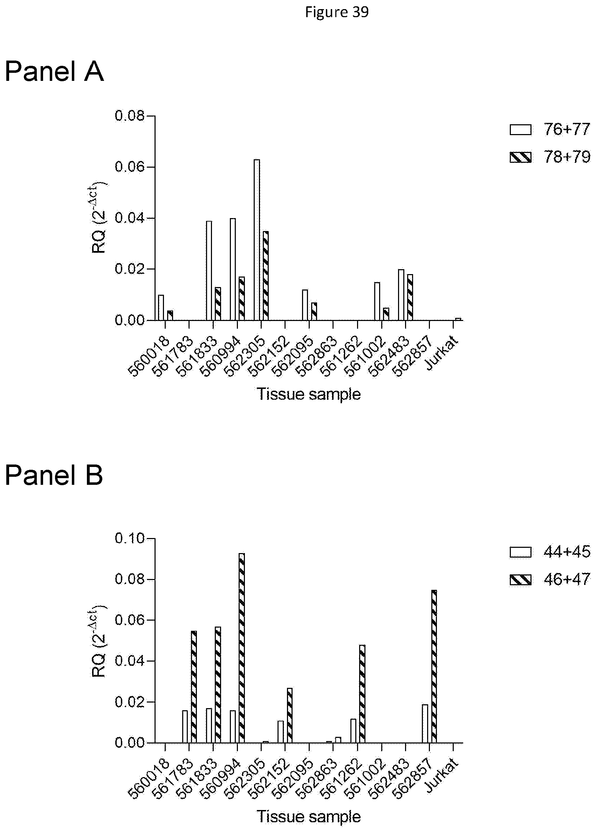

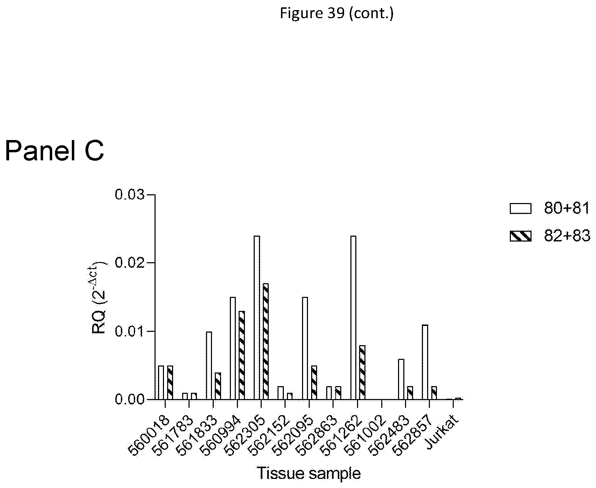

Figure 39 panels A to C shows qRT-PCR assay results to verify the transcription of the CLT encoding CLT Antigen 2 (SEQ ID NO. 24), the CLT encoding CLT Antigen 3 (SEQ ID NO. 25) and the CLT encoding CLT Antigen 6 (SEQ ID NO. 28) in melanoma cancer cell lines.

## Description of the Sequences

SEQ ID NO. 1 is the polypeptide sequence of CLT Antigen 1
SEQ ID NO. 2 is the polypeptide sequence of CLT Antigen 2
SEQ ID NO. 3 is the polypeptide sequence of CLT Antigen 3
SEQ ID NO. 4 is the polypeptide sequence of CLT Antigen 4
SEQ ID NO. 5 is the polypeptide sequence of CLT Antigen 5
SEQ ID NO. 6 is the polypeptide sequence of CLT Antigen 6
SEQ ID NO. 7 is the polypeptide sequence of CLT Antigen 7
SEQ ID NO. 8 is the polypeptide sequence of CLT Antigen 8
SEQ ID NOs. 9 and 10 are peptide sequences derived from CLT Antigen 1
SEQ ID NOs. 11-14 are peptide sequences derived from CLT Antigen 2
SEQ ID NO. 15 is a peptide sequence derived from CLT Antigen 3
SEQ ID NOs. 16 and 17 are peptide sequences derived from CLT Antigen 4
SEQ ID NO. 18 is a peptide sequence derived from CLT Antigen 5
SEQ ID NOs. 19 and 20 are peptide sequences derived from CLT Antigen 6
SEQ ID NO. 21 is a peptide sequence derived from CLT Antigen 7
SEQ ID NO. 22 is a peptide sequence derived from CLT Antigen 8
SEQ ID NO. 23 is the cDNA sequence of the CLT encoding CLT Antigen 1
SEQ ID NO. 24 is the cDNA sequence of the CLT encoding CLT Antigen 2
SEQ ID NO. 25 is the cDNA sequence of the CLT encoding CLT Antigen 3
SEQ ID NO. 26 is the cDNA sequence of the CLT encoding CLT Antigen 4
SEQ ID NO. 27 is the cDNA sequence of the CLT encoding CLT Antigen 5
SEQ ID NO. 28 is the cDNA sequence of the CLT encoding CLT Antigen 6
SEQ ID NO. 29 is the cDNA sequence of the CLT encoding CLT Antigen 7
SEQ ID NO. 30 is the cDNA sequence of the CLT encoding CLT Antigen 8
SEQ ID NO. 31 is a cDNA sequence encoding CLT Antigen 1
SEQ ID NO. 32 is a cDNA sequence encoding CLT Antigen 2

SEQ ID NO. 33 is a cDNA sequence encoding CLT Antigen 3
SEQ ID NO. 34 is a cDNA sequence encoding CLT Antigen 4
SEQ ID NO. 35 is a cDNA sequence encoding CLT Antigen 5
SEQ ID NO. 36 is a cDNA sequence encoding CLT Antigen 6
SEQ ID NO. 37 is a cDNA sequence encoding CLT Antigen 7
SEQ ID NO. 38 is a cDNA sequence encoding CLT Antigen 8
SEQ ID NO. 39 is a peptide sequence derived from CLT Antigen 6

## Detailed Description of the Invention

*Polypeptides*

The terms "protein", "polypeptide" and "peptide" are used interchangeably herein and refer to any peptide-linked chain of amino acids, regardless of length, co-translational or post-translational modification.

The term "amino acid" refers to any one of the naturally occurring amino acids, as well as amino acid analogs and amino acid mimetics that function in a manner which is similar to the naturally occurring amino acids. Naturally occurring amino acids are those 20 L-amino acids encoded by the genetic code, as well as those amino acids that are later modified, e.g., hydroxyproline, $\gamma$-carboxyglutamate, and O-phosphoserine. The term "amino acid analogue" refers to a compound that has the same basic chemical structure as a naturally occurring amino acid, i.e., an $\alpha$ carbon that is bound to a hydrogen, a carboxyl group, an amino group, and an R group but has a modified R group or a modified peptide backbone as compared with a natural amino acid. Examples include homoserine, norleucine, methionine sulfoxide, methionine methyl sulfonium and norleucine. Amino acid mimetics refers to chemical compounds that have a structure that is different from the general chemical structure of an amino acid, but that functions in a manner similar to a naturally occurring amino acid. Suitably an amino acid is a naturally occurring amino acid or an amino acid analogue, especially a naturally occurring amino acid and in particular one of those 20 L-amino acids encoded by the genetic code.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

Thus, the invention provides an isolated polypeptide comprising a sequence selected from:

(a) the sequence of SEQ ID NO. 2; and
(b) an immunogenic variant of the sequence of (a) which is at least 80% identical to SEQ ID NO. 2; and
(c) an immunogenic fragment of the sequence of (a) comprising at least 9 contiguous amino acids of SEQ ID NO. 2In general, variants of polypeptide sequences include sequences having a high degree of sequence identity thereto. For example, immunogenic variants of the invention have at least about 80% identity, more preferably at least about 85% identity and most preferably at least about 90% identity (such as at least about 95%, at least about 98% or at least about 99%) to the associated reference sequence over their whole length.

Variants of polypeptide sequences of the invention are immunogenic variants. A variant is considered to be an immunogenic variant where it elicits a response which is at least 20%, suitably at least 50% and especially at least 75% (such as at least 90%) of the activity of the reference sequence (i.e. the sequence of which the variant is a variant) e.g., in an *in vitro* restimulation assay of PBMC or whole blood with the polypeptide as antigen (e.g., restimulation for a period of between several hours to up to 1 year, such as up to 6 months, 1 day to 1 month or 1 to 2 weeks), that measures the activation of the cells via lymphoproliferation (e.g., T-cell proliferation), production of cytokines (e.g., IFN-gamma) in the supernatant of culture (measured by ELISA etc.) or characterisation of T cell responses by intra- and extracellular staining (e.g., using antibodies specific to immune markers, such as CD3, CD4, CD8, IL2, TNF-alpha, IFNg, Type 1 IFN, CD40L, CD69 etc.) followed by analysis with a flow cytometer.

The immunogenic variant may, for example, be a conservatively modified variant. A "conservatively modified variant" is one where the alteration(s) results in the substitution of an amino acid with a functionally similar amino acid or the substitution/deletion/addition of residues which do not substantially impact the biological function of the variant. Typically, such biological function of the variants will be to induce an immune response against a melanoma e.g. a cutaneous melanoma cancer antigen.

Conservative substitution tables providing functionally similar amino acids are well known in the art. Variants can include homologues of polypeptides found in other species.

A variant of a polypeptide may contain a number of substitutions, for example, conservative substitutions (for example, 1-25, such as 1-10, in particular 1-5, and especially 1 amino acid residue(s) may be altered) when compared to the reference sequence. The number of substitutions, for example, conservative substitutions, may be up to 20% e.g., up to 10% e.g., up to 5% e.g., up to 1% of the number of residues of the reference sequence. In general, conservative substitutions will fall within one of the amino-acid groupings specified below, though in some circumstances other substitutions may be possible without substantially affecting the immunogenic properties of the antigen. The following eight groups each contain amino acids that are typically conservative substitutions for one another:

1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)

(see, e.g., Creighton, Proteins 1984).

Suitably such substitutions do not alter the immunological structure of an epitope (e.g., they do not occur within the epitope region as mapped in the primary sequence), and do not therefore have a significant impact on the immunogenic properties of the antigen.

Polypeptide variants also include those wherein additional amino acids are inserted compared to the reference sequence, for example, such insertions may occur at 1-10 locations (such as 1-5 locations, suitably 1 or 2 locations, in particular 1 location) and may, for example, involve the addition of 50 or fewer amino acids at each location (such as 20 or fewer, in particular 10 or fewer, especially 5 or fewer). Suitably such insertions do not occur in the region of an epitope, and do not therefore have a significant impact on the immunogenic properties of the antigen. One example of insertions includes a short stretch of histidine residues (e.g., 2-6 residues) to aid expression and/or purification of the antigen in question.

Polypeptide variants include those wherein amino acids have been deleted compared to the reference sequence, for example, such deletions may occur at 1-10 locations (such as 1-5 locations, suitably 1 or 2 locations, in particular 1 location) and may, for example, involve the deletion of 50 or fewer amino acids at each location (such as 20 or fewer, in particular 10 or fewer, especially 5 or fewer). Suitably such deletions do not occur in the region of an epitope, and do not therefore have a significant impact on the immunogenic properties of the antigen.

The skilled person will recognise that a particular protein variant may comprise substitutions, deletions and additions (or any combination thereof). For example, substitutions/deletions/additions might enhance (or have neutral effects) on binding to desired patient HLA molecules, potentially increasing immunogenicity (or leaving immunogenicity unchanged).

Immunogenic fragments according to the present invention will comprise at least 9 contiguous amino acids from the full-length polypeptide sequence (e.g., at least 9 or 10), such as at least 12 contiguous amino acids (e.g., at least 15 or at least 20 contiguous amino acids), in particular at least 50 contiguous amino acids, such as at least 100 contiguous amino acids (for example at least 200 contiguous amino acids) depending on the length of the CLT antigen. Suitably the immunogenic fragments will be at least 10%, such as at least 20%, such as at least 50%, such as at least 70% or at least 80% of the length of the full-length polypeptide sequence.

Immunogenic fragments typically comprise at least one epitope. Epitopes include B cell and T cell epitopes and suitably immunogenic fragments comprise at least one T-cell epitope such as a CD4+ or a CD8+ T-cell epitope.

T cell epitopes are short contiguous stretches of amino acids which are recognised by T cells (e.g., CD4+ or CD8+ T cells) when bound to HLA molecules. Identification of T cell epitopes may be achieved through epitope mapping experiments which are well known to the person skilled in the art (see, for example, Paul, Fundamental Immunology, 3rd ed., 243-247 (1993); Beiβbarth et al., 2005, Bioinformatics,21(Suppl. 1):i29-i37).

As a result of the crucial involvement of the T cell response in cancer, it is readily apparent that fragments of the full-length polypeptides of SEQ ID NOs. 1-8 which contain at least one T cell epitope may be immunogenic and may contribute to immunoprotection.

It will be understood that in a diverse outbred population, such as humans, different HLA types mean that specific epitopes may not be recognised by all members of the population. Consequently, to maximise the level of recognition and scale of immune response to a polypeptide, it is generally desirable that an immunogenic fragment contains a plurality of the epitopes from the full-length sequence (suitably all epitopes within a CLT antigen).

Particular fragments of the polypeptides of SEQ ID NOs. 1-8 which may be of use include those containing at least one CD8+ T-cell epitope, suitably at least two CD8+ T-cell epitopes and especially all CD8+ T-cell epitopes, particularly those associated with a plurality of HLA Class I alleles, e.g., those associated with 2, 3, 4, 5 or more alleles). Particular fragments of the polypeptides of SEQ ID NOs. 1-8 which may be of use include those containing at least one CD4+ T-cell epitope, suitably at least two CD4+ T-cell epitopes and especially all CD4+ T-cell epitopes (particularly those associated with a plurality of HLA Class II alleles, e.g., those associated with 2, 3, 4, 5 or more alleles). However, a person skilled in design of vaccines could combine exogenous CD4+ T-cell epitopes with CD8+ T cells epitopes of this invention and achieve desired responses to the invention's CD8+ T cell epitopes.

Where an individual fragment of the full-length polypeptide is used, such a fragment is considered to be immunogenic where it elicits a response which is at least 20%, suitably at least 50% and especially at least 75% (such as at least 90%) of the activity of the reference sequence (i.e., the sequence of which the fragment is a fragment) e.g., activity in an *in vitro* restimulation assay of PBMC or whole blood with the polypeptide as antigen (e.g., restimulation for a period of between several hours to up to 1 year, such as up to 6 months, 1 day to 1 month or 1 to 2 weeks,) that measures the activation of the cells via lymphoproliferation (e.g., T-cell proliferation), production of cytokines (e.g., IFN-gamma) in the supernatant of culture (measured by ELISA etc.) or characterisation of T cell responses by intra and extracellular staining (e.g., using antibodies specific to immune markers, such as CD3, CD4, CD8, IL2, TNF-alpha, IFN-gamma, Type 1 IFN, CD40L, CD69 etc.) followed by analysis with a flow cytometer.

In some circumstances a plurality of fragments of the full-length polypeptide (which may or may not be overlapping and may or may not cover the entirety of the full-length sequence) may be used to obtain an equivalent biological response to the full-length sequence itself. For example, at least two immunogenic fragments (such as three, four or five) as described above, which in combination provide at least 50%, suitably at least 75% and especially at least 90% activity of the reference sequence in an *in vitro* restimulation assay of PBMC or whole blood (e.g., a T cell proliferation and/or IFN-gamma production assay).

Example immunogenic fragments of polypeptides of SEQ ID NOs. 1-8 include polypeptides which comprise or consist of the sequences of SEQ ID NOs. 9-22 and 39. The sequences of SEQ ID NOs. 9-22 were identified as being bound to HLA Class I molecules from immunopeptidomic analysis (see Example 2). The sequence of SEQ ID NO. 39 was predicted by NetMHC software as being bound to an HLA Class I molecule and was used in immunological validation assays (see Example 4).

*Nucleic acids*

The invention provides an isolated nucleic acid encoding a polypeptide of the invention (referred to as a nucleic acid of the invention). For example, the nucleic acid of the invention comprises or consists of a sequence selected from SEQ ID NOs. 24 and 32.

The terms "nucleic acid" and "polynucleotide" are used interchangeably herein and refer to a polymeric macromolecule made from nucleotide monomers particularly deoxyribonucleotide or ribonucleotide monomers. The term encompasses nucleic acids containing known nucleotide analogs or modified backbone residues or linkages, which are naturally occurring and non-naturally occurring, which have similar properties as the reference nucleic acid, and which are intended to be metabolized in a manner similar to the reference nucleotides or are intended to have extended half-life in the system. Examples of such analogs include, without limitation, phosphorothioates, phosphoramidates, methyl phosphonates, chiral-methyl phosphonates, 2-O-methyl ribonucleotides, peptide-nucleic acids (PNAs). Suitably the term "nucleic acid" refers to naturally occurring polymers of deoxyribonucleotide or ribonucleotide monomers. Suitably the nucleic acid molecules of the invention are recombinant. Recombinant means that the nucleic acid molecule is the product of at least one of cloning, restriction or ligation steps, or other procedures that result in a nucleic acid molecule that is distinct from a nucleic acid molecule found in nature (e.g., in the case of cDNA). In an embodiment the nucleic acid of the invention is an artificial nucleic acid sequence (e.g., a cDNA sequence or nucleic acid sequence with non-naturally occurring codon usage). In one embodiment, the nucleic acids of the invention are DNA. Alternatively, the nucleic acids of the invention are RNA.

DNA (deoxyribonucleic acid) and RNA (ribounucleic acid) refer to nucleic acid molecules having a backbone of sugar moieties which are deoxyribosyl and ribosyl moieties respectively. The sugar moieties may be linked to bases which are the 4 natural bases (adenine (A), guanine (G), cytosine (C) and thymine (T) in DNA and adenine (A), guanine (G), cytosine (C) and uracil (U) in RNA). As used herein, a "corresponding RNA" is an RNA having the same sequence as a reference DNA but for the substitution of thymine (T) in the DNA with uracil (U) in the RNA. The sugar moieties may also be linked to unnatural bases such as inosine, xanthosine, 7-methylguanosine, dihydrouridine and 5-methylcytidine. Natural phosphodiester linkages between sugar (deoxyribosyl/ribosyl) moieties may optionally be replaced with phosphorothioates linkages. Suitably nucleic acids of the invention consist of the natural bases attached to a deoxyribosyl or ribosyl sugar backbone with phosphodiester linkages between the sugar moieties.

In an embodiment the nucleic acid of the invention is a DNA. For example the nucleic acid comprises or consists of a sequence selected from SEQ ID NOs. 24 and 32. Also provided is a nucleic acid which comprises or consists of a variant of sequence selected from SEQ ID NOs. 24 and 32 which variant encodes the same amino acid sequence but has a different nucleic acid based on the degeneracy of the genetic code.

Thus, due to the degeneracy of the genetic code, a large number of different, but functionally identical nucleic acids can encode any given polypeptide. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations lead to "silent" (sometimes referred to as "degenerate" or "synonymous") variants, which are one species of conservatively modified variations. Every nucleic acid sequence disclosed herein which encodes a polypeptide also enables every possible silent variation of the nucleic acid. One of skill will recognise that each codon in a nucleic acid (except AUG, which is ordinarily the only codon for methionine, and UGG, which is ordinarily the only codon for tryptophan) can be modified to yield a functionally identical molecule. Accordingly, each silent variation of a nucleic acid that encodes a polypeptide is implicit in each described sequence and is provided as an aspect of the invention.

Degenerate codon substitutions may also be achieved by generating sequences in which the third position of one or more selected (or all) codons is substituted with mixed-base and/or deoxyinosine residues (Batzer et al., 1991, Nucleic Acid Res. 19:5081; Ohtsuka et al., 1985, J. Biol. Chem. 260:2605-2608; Rossolini et al., 1994, Mol. Cell. Probes 8:91-98).

A nucleic acid of the invention which comprises or consists of a sequence selected from SEQ ID NOs. 24 and 32 may contain a number of silent variations (for example, 1-50, such as 1-25, in particular 1-5, and especially 1 codon(s) may be altered) when compared to the reference sequence.

A nucleic acid of the invention may comprise or consist of the sequence of SEQ ID NO. 32 without the initial codon for methionine (i.e. ATG or AUG), or a variant thereof as described above.

In an embodiment the nucleic acid of the invention is an RNA. RNA sequences are provided which correspond to a DNA sequence provided herein and have a ribonucleotide backbone instead of a deoxyribonucleotide backbone and have the sidechain base uracil (U) in place of thymine (T).

Thus a nucleic acid of the invention comprises or consists of the RNA equivalent of a cDNA sequence selected from SEQ ID NOs. 24 and 32 and may contain a number of silent variations (for example, 1-50, such as 1-25, in particular 1-5, and especially 1 codon(s) may be altered) when compared to the reference sequence. By "RNA equivalent" is meant an RNA sequence which contains the same genetic information as the reference cDNA sequence (i.e. contains the same codons with a ribonucleotide backbone instead of a deoxyribonucleotide backbone and having the sidechain base uracil (U) in place of thymine (T)).

The invention also comprises sequences which are complementary to the aforementioned cDNA and RNA sequences.

In an embodiment, the nucleic acids of the invention are codon optimised for expression in a human host cell.

The nucleic acids of the invention are capable of being transcribed and translated into polypeptides of the invention in the case of DNA nucleic acids, and translated into polypeptides of the invention in the case of RNA nucleic acids.

*Polypeptides and Nucleic acids*

The polypeptides and nucleic acids used in the present invention are isolated. An "isolated" polypeptide or nucleic acid is one that is removed from its original environment. For example, a naturally-occurring polypeptide or nucleic acid is isolated if it is separated from some or all of the coexisting materials in the natural system. A nucleic acid is considered to be isolated if, for example, it is cloned into a vector that is not a part of its natural environment.

"Naturally occurring" when used with reference to a polypeptide or nucleic acid sequence means a sequence found in nature and not synthetically modified.

"Artificial" when used with reference to a polypeptide or nucleic acid sequence means a sequence not found in nature which is, for example, a synthetic modification of a natural sequence, or contains an unnatural sequence.

The term "heterologous" when used with reference to the relationship of one nucleic acid or polypeptide to another nucleic acid or polypeptide indicates that the two or more sequences are not found in the same relationship to each other in nature. A "heterologous" sequence can also mean a sequence which is not isolated from, derived from, or based upon a naturally occurring nucleic acid or polypeptide sequence found in the host organism.

As noted above, polypeptide variants have at least about 80% identity, more preferably at least about 85% identity and most preferably at least about 90% identity (such as at least about 95%, at least about 98% or at least about 99%) to the associated reference sequence over their whole length.

For the purposes of comparing two closely-related polypeptide or polynucleotide sequences, the "% sequence identity" between a first sequence and a second sequence may be calculated. Polypeptide sequences are said to be the same as or identical to other polypeptide sequences, if they share 100% sequence identity over their entire length. Residues in sequences are numbered from left to right, i.e. from N- to C- terminus for polypeptides. The terms "identical" or percentage "identity", in the context of two or more polypeptide sequences, refer to two or more sequences or subsequences that are the same or have a specified percentage of amino acid residues that are the same (i.e., 70% identity, optionally 75%, 80%, 85%, 90%, 95%, 98% or 99% identity over a specified region), when compared and aligned for maximum correspondence over a comparison window. Suitably, the comparison is performed over a window corresponding to the entire length of the reference sequence.

For sequence comparison, one sequence acts as the reference sequence, to which the test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percentage sequence identities for the test sequences relative to the reference sequence, based on the program parameters.

A "comparison window", as used herein, refers to a segment in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, 1981, Adv. Appl. Math. 2:482, by the homology alignment algorithm of Needleman & Wunsch, 1970, J. Mol. Biol. 48:443, by the search for similarity method of Pearson & Lipman, 1988, Proc. Nat'l. Acad. Sci. USA 85:2444, by computerised implementations of these algorithms (GAP, BESTFIT, FASTA, and TFASTA in the Wisconsin Genetics Software Package, Genetics Computer Group, 575 Science Dr., Madison, WI), or by manual alignment and visual inspection (*see*, *e.g.*, Current Protocols in Molecular Biology (Ausubel et al., eds. 1995 supplement)).

One example of a useful algorithm is PILEUP. PILEUP creates a multiple sequence alignment from a group of related sequences using progressive, pairwise alignments to show relationship and percent sequence identity. It also plots a tree or dendogram showing the clustering relationships used to create the alignment. PILEUP uses a simplification of the progressive alignment method of Feng & Doolittle, 1987, J. Mol. Evol. 35:351-360. The method used is similar to the method described by Higgins & Sharp, 1989, CABIOS 5:151-153. The program can align up to 300 sequences, each of a maximum length of 5,000 nucleotides or amino acids. The multiple alignment procedure begins with the pairwise alignment of the two most similar sequences, producing a cluster of two aligned sequences. This cluster is then aligned to the next most related sequence or cluster of aligned sequences. Two clusters of sequences are aligned by a simple extension of the pairwise alignment of two individual sequences. The final alignment is achieved by a series of progressive, pairwise alignments. The program is run by designating specific sequences and their amino acid coordinates for regions of sequence comparison and by designating the program parameters. Using PILEUP, a reference sequence is compared to other test sequences to determine the percent sequence identity relationship using the following parameters: default gap weight (3.00), default gap length weight (0.10), and weighted end gaps. PILEUP can be obtained from the GCG sequence analysis software package, e.g., version 7.0 (Devereaux et al., 1984, Nuc. Acids Res. 12:387-395).

Another example of algorithm that is suitable for determining percent sequence identity and sequence similarity are the BLAST and BLAST 2.0 algorithms, which are described in Altschul et al., 1977, Nuc. Acids Res. 25:3389-3402 and Altschul et al., 1990, J. Mol. Biol. 215:403-410, respectively. Software for performing BLAST analyses is publicly available through the National Center for Biotechnology Information (website at www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighbourhood word score threshold (Altschul *et al., supra*). These initial neighbourhood word hits act as seeds for initiating searches to find longer HSPs containing them. The word hits are extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. For amino acid sequences, the BLASTP program uses as defaults a wordlength of 3, and expectation (E) of 10, and the BLOSUM62 scoring matrix (see Henikoff & Henikoff, 1989, Proc. Natl. Acad. Sci. USA 89:10915) alignments (B) of 50, expectation (E) of 10, M=5, N=-4, and a comparison of both strands.

The BLAST algorithm also performs a statistical analysis of the similarity between two sequences (see, *e.g.*, Karlin & Altschul, 1993, Proc. Nat'l. Acad. Sci. USA 90:5873-5787). One measure of similarity provided by the BLAST algorithm is the smallest sum probability (P(N)), which provides an indication of the probability by which a match between two nucleotide or amino acid sequences would occur by chance.

A "difference" between sequences refers to an insertion, deletion or substitution of a single residue in a position of the second sequence, compared to the first sequence. Two sequences can contain one, two or more such differences. Insertions, deletions or substitutions in a second sequence which is otherwise identical (100% sequence identity) to a first sequence result in reduced % sequence identity. For example, if the identical sequences are 9 residues long, one substitution in the second sequence results in a sequence identity of 88.9%. If the identical sequences are 17 amino acid residues long, two substitutions in the second sequence results in a sequence identity of 88.2%.

Alternatively, for the purposes of comparing a first, reference sequence to a second, comparison sequence, the number of additions, substitutions and/or deletions made to the first sequence to produce the second sequence may be ascertained. An addition is the addition of one residue into the first sequence (including addition at either terminus of the first sequence). A substitution is the substitution of one residue in the first sequence with one different residue. A deletion is the deletion of one residue from the first sequence (including deletion at either terminus of the first sequence).

*Production of polypeptides of the invention*

Polypeptides of the invention can be obtained and manipulated using the techniques disclosed for example in Green and Sambrook 2012 Molecular Cloning: A Laboratory Manual 4th Edition Cold Spring Harbour Laboratory Press. In particular, artificial gene synthesis may be used to produce polynucleotides (Nambiar et al., 1984, Science, 223:1299-1301, Sakamar and Khorana, 1988, Nucl. Acids Res., 14:6361-6372, Wells et al., 1985, Gene, 34:315-323 and Grundstrom et al., 1985, Nucl. Acids Res., 13:3305-3316) followed by expression in a suitable organism to produce polypeptides. A gene encoding a polypeptide of the invention can be synthetically produced by, for example, solid-phase DNA synthesis. Entire genes may be synthesized *de novo*, without the need for precursor template DNA. To obtain the desired oligonucleotide, the building blocks are sequentially coupled to the growing oligonucleotide chain in the order required by the sequence of the product. Upon the completion of the chain assembly, the product is released from the solid phase to solution, deprotected, and collected. Products can be isolated by high-performance liquid chromatography (HPLC) to obtain the desired oligonucleotides in high purity (Verma and Eckstein, 1998, Annu. Rev. Biochem. 67:99-134). These relatively short segments are readily assembled by using a variety of gene amplification methods (Methods Mol Biol., 2012; 834:93-109) into longer DNA molecules, suitable for use in innumerable recombinant DNA-based expression systems. In the context of this invention one skilled in the art would understand that the polynucleotide sequences encoding the polypeptide antigens described in this invention could be readily used in a variety of vaccine production systems, including, for example, viral vectors.

For the purposes of production of polypeptides of the invention in a microbiological host (e.g., bacterial or fungal), nucleic acids of the invention will comprise suitable regulatory and control sequences (including promoters, termination signals etc) and sequences to promote polypeptide secretion suitable for protein production in the host. Similarly, polypeptides of the invention could be produced by transducing cultures of eukaryotic cells (e.g., Chinese hamster ovary cells or drosophila S2 cells) with nucleic acids of the invention which have been combined with suitable regulatory and control sequences (including promoters, termination signals etc) and sequences to promote polypeptide secretion suitable for protein production in these cells.

Improved isolation of the polypeptides of the invention produced by recombinant means may optionally be facilitated through the addition of a stretch of histidine residues (commonly known as a His-tag) towards one end of the polypeptide.

Polypeptides may also be produced synthetically.

*Vectors*

In additional embodiments, genetic constructs comprising one or more of the nucleic acids of the invention are introduced into cells *in vivo* such that a polypeptide of the invention is produced *in vivo* eliciting an immune response. The nucleic acid (e.g., DNA) may be present within any of a variety of delivery systems known to those of ordinary skill in the art, including nucleic acid expression systems, bacteria and some viral expression systems. Numerous gene delivery techniques are well known in the art, such as those described by Rolland, 1998, Crit. Rev. Therap. Drug Carrier Systems 15:143-198, and references cited therein. Several of these approaches are outlined below for the purpose of illustration.

Accordingly, there is provided a vector (also referred to herein as a 'DNA expression construct' or 'construct') comprising a nucleic acid molecule of the invention.

Suitably, the vector comprises nucleic acid encoding regulatory elements (such as a suitable promoter and terminating signal) suitable for permitting transcription of a translationally active RNA molecule in a human host cell. A "translationally active RNA molecule" is an RNA molecule capable of being translated into a protein by a human cell's translation apparatus.

Accordingly, there is provided a vector comprising a nucleic acid of the invention (herein after a "vector of the invention").

In particular, the vector may be a viral vector. The viral vector may be an adenovirus, adeno-associated virus (AAV) (e.g., AAV type 5 and type 2), alphavirus (e.g., Venezuelan equine encephalitis virus (VEEV), Sindbis virus (SIN), Semliki Forest virus (SFV)), herpes virus, arenavirus (e.g., lymphocytic choriomeningitis virus (LCMV)), measles virus, poxvirus (such as modified vaccinia Ankara (MVA)), paramyxovirus, lentivirus, or rhabdovirus (such as vesicular stomatitis virus (VSV)) vector i.e. the vector may be derived from any of the aforementioned viruses. Adenoviruses are particularly suitable for use as a gene transfer vector because of its mid-sized genome, ease of manipulation, high titre, wide target-cell range and high infectivity. Both ends of the viral genome contain 100-200 base pair inverted repeats (ITRs), which are cis elements necessary for viral DNA replication and packaging. The early (E) and late (L) regions of the genome contain different transcription units that are divided by the onset of viral DNA replication. The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression and host cell shut-off (Renan, 1990). The products of the late genes, including the majority of the viral capsid proteins, are expressed only after significant processing of a single primary transcript issued by the major late promoter (MLP). The MLP is particularly efficient during the late phase of infection, and all the mRNAs trasncribed from this promoter possess a 5'-tripartite leader (TPL) sequence which makes them preferred mRNAs for translation. Replication-deficient adenovirus, which are created by from viral genomes that are deleted for one or more of the early genes are particularly useful, since they have limited replication and less possibility of pathogenic spread within a vaccinated host and to contacts of the vaccinated host.

*Other polynucleotide delivery*

In certain embodiments of the invention, the expression construct comprising one or more polynucleotide sequences may simply consist of naked recombinant DNA plasmids. See Ulmer et al., 1993, Science 259:1745-1749 and reviewed by Cohen, 1993, Science 259:1691-1692. Transfer of the construct may be performed, for example, by any method which physically or chemically permeabilises the cell membrane. This is particularly applicable for transfer *in vitro* but it may be applied to *in vivo* use as well. It is envisioned that DNA encoding a gene of interest may also be transferred in a similar manner *in vivo* and express the gene product. Multiple delivery systems have been used to deliver DNA molecules into animal models and into man. Some products based on this technology have been licensed for use in animals, and others are in phase 2 and 3 clinical trials in man.

*RNA delivery*

In other embodiments of the invention, the expression construct comprising one or more polynucleotide sequences may consist of naked, recombinant DNA-derived RNA molecules (Ulmer et al., 2012, Vaccine 30:4414-4418). As for DNA-based expression constructs, a variety of methods can be utilized to introduce RNA molecules into cells in vitro or in vivo. The RNA-based constructs can be designed to mimic simple messenger RNA (mRNA) molecules, such that the introduced biological molecule is directly translated by the host cell's translation machinery to produce its encoded polypeptide in the cells to which it has been introduced. Alternatively, RNA molecules may be designed in a manner that allows them to self-amplify within cells they are introduced into, by incorporating into their structure genes for viral RNA-dependent RNA polymerases. Thus, these types of RNA molecules, known as self-amplifying mRNA (SAM™) molecules (Geall et al. 2012, PNAS, 109:14604-14609), share properties with some RNA-based viral vectors. Either mRNA-based or SAM™ RNAs may be further modified (e.g., by alteration of their sequences, or by use of modified nucleotides) to enhance stability and translation (Schlake et al., RNA Biology, 9: 1319-1330), and both types of RNAs may be formulated (e.g., in emulsions (Brito et al., Molecular Therapy, 2014 22:2118-2129) or lipid nanoparticles (Kranz et al., 2006, Nature, 534:396-401)) to facilitate stability and/or entry into cells in vitro or in vivo. Myriad formulations of modified (and non-modified) RNAs have been tested as vaccines in animal models and in man, and multiple RNA-based vaccines are being used in ongoing clinical trials.

*Pharmaceutical Compositions*

The polypeptides, nucleic acids and vectors of the invention may be formulated for delivery in pharmaceutical compositions which are immunogenic pharmaceutical compositions and vaccine compositions (all hereinafter "compositions of the invention"). Compositions of the invention comprise a polypeptide, nucleic acid or vector of the invention together with a pharmaceutically acceptable carrier.

Thus, in an embodiment, there is provided an immunogenic pharmaceutical composition comprising a polypeptide, nucleic acid or vector of the invention together with a pharmaceutically acceptable carrier.

In another embodiment there is provided a vaccine composition comprising a polypeptide, nucleic acid or vector of the invention together with a pharmaceutically acceptable carrier. Preparation of pharmaceutical compositions is generally described in, for example, Powell & Newman, eds., Vaccine Design (the subunit and adjuvant approach), 1995. Compositions of the invention may also contain other compounds, which may be biologically active or inactive. Suitably, the composition of the invention is a sterile composition suitable for parenteral administration.

In certain preferred embodiments of the present invention, pharmaceutical compositions of the invention are provided which comprise one or more (e.g., one) polypeptides of the invention in combination with a pharmaceutically acceptable carrier.

In certain preferred embodiments of the present invention, compositions of the invention are provided which comprise one or more (e.g., one) nucleic acids of the invention or one or more (e.g., one) vectors of the invention in combination with a pharmaceutically acceptable carrier.

In an embodiment, the compositions of the invention may comprise one or more (e.g., one) polynucleotide and one or more (e.g., one) polypeptide components. Alternatively, the compositions may comprise one or more (e.g., one) vector and one or more (e.g., one) polypeptide components. Alternatively, the compositions may comprise one or more (e.g., one) vector and one or more (e.g., one) polynucleotide components. Such compositions may provide for an enhanced immune response.

*Pharmaceutically acceptable salts*

It will be apparent that a composition of the invention may contain pharmaceutically acceptable salts of the nucleic acids or polypeptides provided herein. Such salts may be prepared from pharmaceutically acceptable non-toxic bases, including organic bases (e.g., salts of primary, secondary and tertiary amines and basic amino acids) and inorganic bases (e.g., sodium, potassium, lithium, ammonium, calcium and magnesium salts).

*Pharmaceutically acceptable carriers*

While many pharmaceutically acceptable carriers known to those of ordinary skill in the art may be employed in the compositions of the invention, the optimal type of carrier used will vary depending on the mode of administration. Compositions of the present invention may be formulated for any appropriate manner of administration, including for example, parenteral, topical, oral, nasal, intravenous, intracranial, intraperitoneal, subcutaneous or intramuscular administration, preferably parenteral e.g., intramuscular, subcutaneous or intravenous administration. For parenteral administration, the carrier preferably comprises water and may contain buffers for pH control, stabilising agents e.g., surfactants and amino acids and tonicity modifying agents e.g., salts and sugars. If the composition is intended to be provided in lyophilised form for dilution at the point of use, the formulation may contain a lyoprotectant e.g., sugars such as trehalose. For oral administration, any of the above carriers or a solid carrier, such as mannitol, lactose, starch, magnesium stearate, sodium saccharine, talcum, cellulose, glucose, sucrose, and magnesium carbonate, may be employed.

Thus, compositions of the invention may comprise buffers (e.g., neutral buffered saline or phosphate buffered saline), carbohydrates (e.g., glucose, mannose, sucrose or dextrans), mannitol, proteins, polypeptides or amino acids such as glycine, antioxidants, bacteriostats, chelating agents such as EDTA or glutathione, solutes that render the formulation isotonic, hypotonic or weakly hypertonic with the blood of a recipient, suspending agents, thickening agents and/or preservatives. Alternatively, compositions of the invention may be formulated as a lyophilizate.

*Immunostimulants*

Compositions of the invention may also comprise one or more immunostimulants. An immunostimulant may be any substance that enhances or potentiates an immune response (antibody and/or cell-mediated) to an exogenous antigen. Examples of immunostimulants, which are often referred to as adjuvants in the context of vaccine formulations, include aluminium salts such as aluminium hydroxide gel (alum) or aluminium phosphate, saponins including QS21, immunostimulatory oligonucleotides such as CPG, oil-in-water emulsion (e.g., where the oil is squalene), aminoalkyl glucosaminide 4-phosphates, lipopolysaccharide or a derivative thereof e.g., 3-de-O-acylated monophosphoryl lipid A (3D-MPL®) and other TLR4 ligands, TLR7 ligands, TLR8 ligands, TLR9 ligands, IL-12 and interferons. Thus, suitably the one or more immunostimulants of the composition of the invention are selected from aluminium salts, saponins, immunostimulatory oligonucleotides, oil-in-water emulsions, aminoalkyl glucosaminide 4-phosphates, lipopolysaccharides and derivatives thereof and other TLR4 ligands, TLR7 ligands, TLR8 ligands and TLR9 ligands. Immunostimulants may also include monoclonal antibodies which specifically interact with other immune components, for example monoclonal antibodies that block the interaction of immune checkpoint receptors, including PD-1 and CTLA4.

In the case of recombinant-nucleic acid methods of delivery (e.g., DNA, RNA, viral vectors), the genes encoding protein-based immunostimulants may be readily delivered along with the genes encoding the polypeptides of the invention.

*Sustained release*

The compositions described herein may be administered as part of a sustained-release formulation (i.e., a formulation such as a capsule, sponge, patch or gel (composed of polysaccharides, for example)) that effects a slow/sustained release of compound following administration.

*Storage and packaging*

Compositions of the invention may be presented in unit-dose or multi-dose containers, such as sealed ampoules or vials. Such containers are preferably hermetically sealed to preserve sterility of the formulation until use. In general, formulations may be stored as suspensions, solutions or emulsions in oily or aqueous vehicles. Alternatively, a composition of the invention may be stored in a freeze-dried condition requiring only the addition of a sterile liquid carrier (such as water or saline for injection) immediately prior to use.

*Dosage*

The amount of nucleic acid, polypeptide or vector in each composition of the invention may be prepared is such a way that a suitable dosage for therapeutic or prophylactic use will be obtained. Factors such as solubility, bioavailability, biological half-life, route of administration, product shelf life, as well as other pharmacological considerations will be contemplated by one skilled in the art of preparing such compositions, and as such, a variety of dosages and treatment regimens may be desirable.

Typically, compositions comprising a therapeutically or prophylactically effective amount deliver about 0.1 ug to about 1000 ug of polypeptide of the invention per administration, more typically about 2.5 ug to about 100 ug of polypeptide per administration. If delivered in the form of short, synthetic long peptides, doses could range from 1 to 200ug/peptide/dose. In respect of polynucleotide compositions, these typically deliver about 10 ug to about 20 mg of the nucleic acid of the invention per administration, more typically about 0.1 mg to about 10 mg of the nucleic acid of the invention per administration.

*Diseases to be treated or prevented*

As noted elsewhere, SEQ ID NOs. 1-8 are polypeptide sequences corresponding to CLT antigens which are over-expressed in cutaneous melanoma.

In one embodiment, the invention provides a polypeptide, nucleic acid, vector or composition of the invention for use in medicine.

The present invention also provides a polypeptide, nucleic acid, vector or composition of the invention for use in treating or preventing cancer in a human, wherein the cells of the cancer express a corresponding sequence selected from SEQ ID NO. 2, an immunogenic variant of SEQ ID NO. 2 which is at least 80% identical to SEQ ID NO. 2, and an immunogenic fragment of SEQ ID NO. 2 comprising at least 9 contiguous amino acids of SEQ ID NO. 2.

Transcripts corresponding to SEQ ID NOs. 23, 24, 25, 27 and 30 were also overexpressed in uveal melanoma. Consequently, in an alternative embodiment, the tumor is a uveal melanoma tumor and/or the tumor expresses the sequence of SEQ ID NO. 2.

Thus, the invention provides a method or a polypeptide, nucleic acid, vector or composition for use according to the invention wherein the polypeptide comprises a sequence selected from:

(a) the sequence of SEQ ID NO. 2; and
(b) an variant of the sequence of (a) which is at least 80% identical to SEQ ID NO. 2; and
(c) an immunogenic fragment of the sequence of (a) comprising at least 9 contiguous amino acids of SEQ ID NO. 2.

and for example the polypeptide comprises or consists of a sequence selected from any one of SEQ ID NOs. 11 to 14 and for example the nucleic acid comprises or consists of a sequence selected from SEQ ID NO. 24 or SEQ ID NO. 32; and wherein the cancer is uveal melanoma.

The words "prevention" and "prophylaxis" are used interchangeably herein.

*Treatment and Vaccination Regimes*

A therapeutic regimen may involve either simultaneous (such as co-administration) or sequential (such as a prime-boost) delivery of (i) a polypeptide, nucleic acid or vector of the invention with (ii) one or more further polypeptides, nucleic acids or vectors of the invention and/or (iii) a further component such as a variety of other therapeutically useful compounds or molecules such as antigenic proteins optionally simultaneously administered with adjuvant. Examples of co-administration include homo-lateral co-administration and contra-lateral co-administration. "Simultaneous" administration suitably refers to all components being delivered during the same round of treatment. Suitably all components are administered at the same time (such as simultaneous administration of both DNA and protein), however, one component could be administered within a few minutes (for example, at the same medical appointment or doctor's visit) or within a few hours.

In some embodiments, a "priming" or first administration of a polypeptide, nucleic acid or vector of the invention, is followed by one or more "boosting" or subsequent administrations of a polypeptide, nucleic acid or vector of the invention ("prime and boost" method). In one embodiment the polypeptide, nucleic acid or vector of the invention is used in a prime-boost vaccination regimen. In an embodiment both the prime and boost are of a polypeptide of the invention, the same polypeptide of the invention in each case. In an embodiment both the prime and boost are of a nucleic acid or vector of the invention, the same nucleic acid or vector of the invention in each case. Alternatively, the prime may be performed using a nucleic acid or vector of the invention and the boost performed using a polypeptide of the invention or the prime may be performed using a polypeptide of the invention and the boost performed using a nucleic acid or vector of the invention. Usually the first or "priming" administration and the second or "boosting" administration are given about 1-12 weeks later, or up to 4-6 months later. Subsequent "booster" administrations may be given as frequently as every 1-6 weeks or may be given much later (up to years later).

*Antigen Combinations*

The polypeptides, nucleic acids or vectors of the invention can be used in combination with one or more other polypeptides or nucleic acids or vectors of the invention and/or with other antigenic polypeptides (or polynucleotides or vectors encoding them) which cause an immune response to be raised against melanoma e.g. cutaneous or uveal melanoma. These other antigenic polypeptides could be derived from diverse sources, they could include well-described melanoma-associated antigens, such as GPR143, PRAME, MAGE-A3 or pMel (gp100). Alternatively they could include other types of melanoma antigens, including patient-specific neoantigens (Lauss et al. (2017). Nature Communications, 8(1), 1738. http://doi.org/10.1038/s41467-017-01460-0), retained-intron neoantigens (Smart et al. (2018). Nature Biotechnology. http://doi.org/10.1038/nbt.4239), spliced variant neoantigens (Hoyos et al., Cancer Cell, 34(2), 181-183. http://doi.org/10.1016/j.ccell.2018.07.008; Kahles et al. (2018). Cancer Cell, 34(2), 211-224.e6. http://doi.org/10.1016/j.ccell.2018.07.001), melanoma antigens that fit within the category known as antigens encoding T cell epitopes associated with impaired peptide processing (TIEPPs; Gigoux, M., & Wolchok, J. (2018). JEM, 215, 2233, Marijt et al. (2018). JEM 215, 2325), or to-be discovered neoantigens (including CLT antigens). In addition, the antigenic peptides from these various sources could also be combined with (i) non-specific immunostimulant/adjuvant species and/or (ii) an antigen, e.g. comprising universal CD4 helper epitopes, known to elicit strong CD4 helper T cells (delivered as a polypeptides, or as polynucleotides or vectors encoding these CD4 antigens), to amplify the anti-melanoma-specific responses elicited by co-administered antigens.

Different polypeptides, nucleic acids or vectors may be formulated in the same formulation or in separate formulations. Alternatively, polypeptides may be provided as fusion proteins in which a polypeptide of the invention is fused to a second or further polypeptide (see below).

Nucleic acids may be provided which encode the aforementioned fusion proteins.

More generally, when two or more components are utilised in combination, the components could be presented, for example:

   (1) as two or more individual antigenic polypeptide components;
   (2) as a fusion protein comprising both (or further) polypeptide components;
   (3) as one or more polypeptide and one or more polynucleotide component;
   (4) as two or more individual polynucleotide components;
   (5) as a single polynucleotide encoding two or more individual polypeptide components; or
   (6) as a single polynucleotide encoding a fusion protein comprising both (or further) polypeptide components.

For convenience, it is often desirable that when a number of components are present they are contained within a single fusion protein or a polynucleotide encoding a single fusion protein (see below). In one embodiment of the invention all components are provided as polypeptides (e.g., within a single fusion protein). In an alternative embodiment of the invention all components are provided as polynucleotides (e.g., a single polynucleotide, such as one encoding a single fusion protein).

*Fusion proteins*

As an embodiment of the above discussion of antigen combinations, the invention also provides an isolated polypeptide according to the invention fused to a second or further polypeptide selected from

   (i) one or more other polypeptides selected from

      (x) polypeptides having the sequences of SEQ ID NOs. 1 and 3-8; immunogenic variants of the polypeptides of (x) which are at least 80% identical thereto;
      immunogenic fragments of the polypeptides of (x) which comprise at least 9 contiguous amino acids of the sequence of the said polypeptide; and
(y) polypeptides having the sequences of SEQ ID NOs. 9, 10, 15-22 and 39;

(herein after a "combination polypeptide of the invention"), by creating nucleic acid constructs that fuse together the sequences encoding the individual antigens. Combination polypeptides of the invention are expected to have the utilities described herein for polypeptides of the invention, and may have the advantage of superior immunogenic or vaccine activity or prophylactic or therapeutic effect (including increasing the breadth and depth of responses), and may be especially valuable in an outbred population. Fusions of polypeptides of the invention may also provide the benefit of increasing the efficiency of construction and manufacture of vaccine antigens and/or vectored vaccines (including nucleic acid vaccines).

As described above in the Antigen Combinations section, polypeptides of the invention and combination polypeptides of the invention may also be fused to polypeptide sequences which are not polypeptides of the invention, including one or more of:

(a) other polypeptides which are melanoma associated antigens and thus potentially useful as immunogenic sequences in a vaccine (e.g., GPR143, PRAME, MAGE-A3 and pMel (gp100) referred to *supra*); and
(b) polypeptide sequences which are capable of enhancing an immune response (i.e. immunostimulant sequences).
(c) .

The invention also provides nucleic acids encoding the aforementioned fusion proteins and other aspects of the invention (vectors, compositions, cells etc) *mutatis mutandis* as for the polypeptides of the invention.

*CLT Antigen-binding polypeptides*

Antigen-binding polypeptides which are immunospecific for tumor-expressed antigens (polypeptides of the invention) may be designed to recruit cytolytic cells to antigen-decorated tumor cells, mediating their destruction. One such mechanism of recruitment of cytolytic cells by antigen-binding polypeptides is known as antibody-dependent cell-mediated cytotoxicity (ADCC). Thus the invention provides an antigen-binding polypeptide which is immunospecific for a polypeptide having the sequence of SEQ ID NO. 2 or an immunogenic fragment of SEQ ID NO. 2 comprising at least 9 contiguous amino acids of SEQ ID NO. 2. Antigen-binding polypeptides including antibodies such as monoclonal antibodies and fragments thereof e.g., domain antibodies, Fab fragments, Fv fragments, and VHH fragments which may produced in a non-human animal species (e.g., rodent or camelid) and humanised or may be produced in a non-human species (e.g., rodent genetically modified to have a human immune system).

Antigen-binding polypeptides may be produced by methods well known to a skilled person. For example, monoclonal antibodies can be produced using hybridoma technology, by fusing a specific antibody-producing B cell with a myeloma (B cell cancer) cell that is selected for its ability to grow in tissue culture and for an absence of antibody chain synthesis (Köhler and Milstein, 1975, Nature 256(5517): 495-497 and Nelson et al., 2000 (Jun), Mol Pathol. 53(3):111-7).

A monoclonal antibody directed against a determined antigen can, for example, be obtained by:

a) immortalizing lymphocytes obtained from the peripheral blood of an animal (including a human) previously immunized/exposed with a determined antigen, with an immortal cell and preferably with myeloma cells, in order to form a hybridoma,
b) culturing the immortalized cells (hybridoma) formed and recovering the cells producing the antibodies having the desired specificity.

Monoclonal antibodies can be obtained by a process comprising the steps of:

a) cloning into vectors, especially into phages and more particularly filamentous bacteriophages, DNA or cDNA sequences obtained from lymphocytes especially peripheral blood lymphocytes of an animal (suitably previously immunized with determined antigens),
b) transforming prokaryotic cells with the above vectors in conditions allowing the production of the antibodies,
c) selecting the antibodies by subjecting them to antigen-affinity selection,
d) recovering the antibodies having the desired specificity
e) expressing antibody-encoding nucleic acid molecules obtained from B cells of patients exposed to antigens, or animals experimentally immunized with antigens.

The selected antibodies may then be produced using conventional recombinant protein production technology (e.g., from genetically engineered CHO cells).

The invention provides an isolated antigen-binding polypeptide which is immunospecific for a polypeptide of the invention. Suitably, the antigen-binding polypeptide is a monoclonal antibody or a fragment thereof.

In certain embodiments, the antigen-binding polypeptide is coupled to a cytotoxic moiety. Example cytotoxic moieties include the Fc domain of an antibody, which will recruit Fc receptor-bearing cells facilitating ADCC. Alternatively, the antigen-binding polypeptide may be linked to a biological toxin, or a cytotoxic chemical.

Another important class of antigen-binding polypeptides include T-cell receptor (TCR)-derived molecules that bind to HLA-displayed fragments of the antigens of this invention. In this embodiment, TCR-based biologicals (including TCRs derived directly from patients, or specifically manipulated, high-affinity TCRs) that recognize CLT antigens (or derivatives thereof) on the surface of tumor cells may also include a targeting moiety which recognizes a component on a T cell (or another class of immune cell) that attract these immune cells to tumors, providing therapeutic benefit. In some embodiments, the targeting moiety may also stimulate beneficial activities (including cytolytic activities) of the redirected immune cells.

Thus, in an embodiment, the antigen-binding polypeptide is immunospecific for an HLA-bound polypeptide that is or is part of a polypeptide of the invention. For example, the antigen-binding polypeptide is a T-cell receptor.

In an embodiment, an antigen-binding polypeptide of the invention may be coupled to another polypeptide that is capable of binding to cytotoxic cells or other immune components in a subject.

In an embodiment, the antigen-binding polypeptide is for use in medicine.

In an embodiment, there is provided a pharmaceutical composition comprising an antigen-binding polypeptide of the invention together with a pharmaceutically acceptable carrier. Such a composition may be a sterile composition suitable for parenteral administration. See e.g., disclosure of pharmaceutical compositions *supra.*

In an embodiment, there is provided an antigen-binding polypeptide of the invention, which may be coupled to a cytotoxic moiety, or composition comprising said antigen-binding polypeptide of the invention for use in treating or preventing cancer in a human, wherein the cells of the cancer express a corresponding sequence selected from SEQ ID NO. 2, an immunogenic fragment of SEQ ID NO. 2 comprising at least 9 contiguous amino acids of SEQ ID NO. 2, and an immunogenic variant of SEQ ID NO. 2 which is at least 80% identical to SEQ ID NO. 2.

Suitably in any of the above embodiments, the cancer is melanoma particularly cutaneous melanoma.

Antigen-binding polypeptides (such as antibodies or fragments thereof may be administered at a dose of e.g. 5-1000 mg e.g. 25-500 mg e.g. 100-300 mg e.g. *ca.* 200 mg.

*Engineered immune cell therapies*

Derivatives of all types of CLT antigen-binding polypeptides described above, including TCRs or TCR mimetics (see Dubrovsky *et al.,* 2016, Oncoimmunology) that recognize CLT antigen-derived peptides complexed to human HLA molecules, may be engineered to be expressed on the surface of T cells (autologous or non-autologous), which can then be administered as adoptive T cell therapies to treat cancer.

These derivatives fit within the category of "chimeric antigen receptors (CARs)," which, as used herein, may refer to artificial T-cell receptors, chimeric T-cell receptors, or chimeric immunoreceptors, for example, and encompass engineered receptors that graft an artificial specificity onto a particular immune effector cell. CARs may be employed to impart the specificity of a monoclonal antibody onto a T cell, thereby allowing a large number of specific T cells to be generated, for example, for use in adoptive cell therapy. CARs may direct specificity of the cell to a tumor associated antigen, a polypeptide of the invention, wherein the polypeptide is HLA-bound.

Another approach to treating cancer in a patient is to genetically modify T-cells to target antigens expressed on tumor cells, via the expression of chimeric antigen receptors (CARs). This technology is reviewed in Wendell & June, 2017, Cell, 168: 724-740.

Such CAR T-cells may be produced by the method of obtaining a sample of cells from the subject, e.g., from peripheral blood, umbilical cord blood and/or by apheresis, wherein said sample comprises T-cells or T-cell progenitors, and transfecting said cells with a nucleic acid encoding a chimeric T-cell receptor (CAR) which is immunospecific for the polypeptide of the invention, wherein the polypeptide is HLA-bound. Such nucleic acid will be capable of integration into the genome of the cells, and the cells may be administered in an effective amount the subject to provide a T-cell response against cells expressing a polypeptide of the invention. For example, the sample of cells from the subject may be collected.

It is understood that cells used to produce said CAR-expressing T-cells may be autologous or non-autologous.

Transgenic CAR-expressing T cells may have expression of an endogenous T-cell receptor and/or endogenous HLA inactivated. For example, the cells may be engineered to eliminate expression of endogenous alpha/beta T-cell receptor (TCR).

Methods of transfecting of cells are well known in the art, but highly efficient transfection methods such as electroporation may be employed. For example, nucleic acids or vectors of the invention expressing the CAR constructs may be introduced into cells using a nucleofection apparatus.

The cell population for CAR-expressing T-cells may be enriched after transfection of the cells. For example, the cells expressing the CAR may be sorted from those which do not (e.g., via FACS) by use of an antigen bound by the CAR or a CAR-binding antibody. Alternatively, the enrichment step comprises depletion of the non-T-cells or depletion of cells that lack CAR expression. For example, CD56+ cells can be depleted from a culture population.

The population of transgenic CAR-expressing cells may be cultured *ex vivo* in a medium that selectively enhances proliferation of CAR-expressing T-cells. Therefore, the CAR- expressing T cell may be expanded *ex vivo*.

A sample of CAR cells may be preserved (or maintained in culture). For example, a sample may be cryopreserved for later expansion or analysis.

CAR-expressing T cells may be employed in combination with other therapeutics, for example checkpoint inhibitors including PD-L1 antagonists.

In an embodiment, there is provided a cytotoxic cell that has been engineered to express any of the above antigen-binding polypeptides on its surface. Suitably, the cytotoxic cell is a T-cell.

In an embodiment, there is provided a cytotoxic cell, which is suitably a T-cell, engineered to express any of the above antigen-binding polypeptides on its surface, for use in medicine

The invention provides a pharmaceutical composition comprising a cytotoxic cell of the invention, which is suitably a T-cell.

In an embodiment the cytotoxic cell of the invention, which is suitably a T-cell, is for use in treating or preventing cancer in a human, wherein the cells of the cancer express a sequence selected from SEQ ID NO. 2 and an immunogenic fragment of SEQ ID NO. 2 comprising at least 9 contiguous amino acids of SEQ ID NO. 2.

*Combination Therapies*

The polypeptides, nucleic acids, vectors, antigen-binding polypeptide and adoptive cell therapies (APC and T cell-based) can be used in combination with other components designed to enhance their immunogenicity, for example, to improve the magnitude and/or breadth of the elicited immune response, or provide other activities (e.g., activation of other aspects of the innate or adaptive immune response, or destruction of tumor cells).

Accordingly, the invention provides a composition of the invention (i.e. an immunogenic, vaccine or pharmaceutical composition) or a kit of several such compositions comprising a polypeptide, nucleic acid or vector of the invention together with a pharmaceutically acceptable carrier; and (i) one or more further immunogenic or immunostimulant polypeptides (e.g., interferons, IL-12, checkpoint blockade molecules or nucleic acids encoding such, or vectors comprising such nucleic acids), (ii) small molecules (e.g., HDAC inhibitors or other drugs that modify the epigenetic profile of cancer cells) or biologicals (delivered as polypeptides or nucleic acids encoding such, or vectors comprising such nucleic acids) that will enhance the translation and/or presentation of the polypeptide products that are the subject of this invention.

Checkpoint inhibitors, which block normal proteins on cancer cells, or the proteins on the T cells that respond to them, may be a particularly important class of drugs to combine with CLT-antigen based therapies, since these inhibitors seek to overcome one of cancer's main defences against an immune system attack.

Thus, an aspect of the invention includes a polypeptide, nucleic acid, vector, antigen-binding polypeptide, composition, T-cell, T-cell population, or antigen presenting cell of the present invention for use in combination with a checkpoint inhibitor. Example check point inhibitors are selected from PD-1 inhibitors, such as pembrolizumab, (Keytruda) and nivolumab (Opdivo), PD-L1 inhibitors, such as atezolizumab (Tecentriq), avelumab (Bavencio) and durvalumab (Imfinzi) and CTLA-4 inhibitors such as ipilimumab (Yervoy).

Interferons (e.g., alpha, beta and gamma) are a family of proteins the body makes in very small amounts. Interferons may slow down or stop the cancer cells dividing, reduce the ability of the cancer cells to protect themselves from the immune system and/or enhance multiple aspects of the adaptive immune system. Interferons are typically administered as a subcutaneous injection in, for example the thigh or abdomen.

Thus, an aspect of the invention includes a polypeptide, nucleic acid, vector, antigen-binding polypeptide or composition of the present invention for use in combination with interferon e.g., interferon alpha.

Different modes of the invention may also be combined, for example polypeptides, nucleic acids and vectors of the invention may be combined with an APC, a T-cell or a T-cell population of the invention (discussed *infra*).

One or more modes of the invention may also be combined with conventional anti-cancer chemotherapy and/or radiation.

Kits for detecting the presence of nucleic acids are well known. For example, kits comprising at least two oligonucleotides which hybridise to a polynucleotide may be used within a real-time PCR (RT-PCR) reaction to allow the detection and semi-quantification of specific nucleic acids. Such kits may allow the detection of PCR products by the generation of a fluorescent signal as a result of Forster Resonance Energy Transfer (FRET) (for example TaqMan® kits), or upon binding of double stranded DNA (for example, SYBR® Green kits). Some kits (for example, those containing TaqMan® probes whch span the exons of the target DNA) allow the detection and quanitfication of mRNA, for example transcripts encoding nucleic acids of the invention. Assays using certain kits may be set up in a multiplex format to detect multiple nucleic acids simultaneously within a reaction. Kits for the detection of active DNA (namely DNA that carries specific epigenetic signatures indicative of expression) may also be used. Additional components that may be present within such kits include a diagnostic reagent or reporter to facilitate the detection of a nucleic acid of the invention.

*Specific embodiments*

In an embodiment of the disclosure, the CLT antigen polypeptide comprises or consists of SEQ ID NO. 1. Exemplary fragments comprise or consist of any one of SEQ ID NOs. 9 and 10. Exemplary nucleic acids encoding said polypeptide sequence comprise or consists of SEQ ID NO 23 or SEQ ID NO. 31. Corresponding nucleic acids (e.g., DNA or RNA), T-cells, T-cell populations, cytocotic cells, antigen-binding polypeptides, antigen presenting cells and exosomes as described *supra* are provided. Said nucleic acids (e.g., DNA or RNA), T-cells, T-cell populations, cytotoxic cells, antigen-binding polypeptides, antigen presenting cells and exosomes may be used in the treatment of cancer especially melanoma e.g. cutaneous melanoma or uveal melanoma. Related methods of diagnosis are also provided.

In an embodiment of the disclosure, the CLT antigen polypeptide comprises or consists of SEQ ID NO. 2. Exemplary fragments comprise or consist of any one of SEQ ID NOs. 11-14. Exemplary nucleic acids encoding said polypeptide sequence comprise or consists of SEQ ID NO. 24 or SEQ ID NO. 32. Corresponding nucleic acids (e.g., DNA or RNA), T-cells, T-cell populations, cytocotic cells, antigen-binding polypeptides, antigen presenting cells and exosomes as described *supra* are provided. Said nucleic acids (e.g., DNA or RNA), T-cells, T-cell populations, cytotoxic cells, antigen-binding polypeptides, antigen presenting cells and exosomes may be used in the treatment of cancer especially melanoma e.g. cutaneous or uveal melanoma. Related methods of diagnosis are also provided.

In an embodiment of the disclosure, the CLT antigen polypeptide comprises or consists of SEQ ID NO. 3. Exemplary fragments comprise or consist of SEQ ID NO. 15. Exemplary nucleic acids encoding said polypeptide sequence comprise or consists of SEQ ID NO. 25 or SEQ ID NO. 33. Corresponding nucleic acids (e.g., DNA or RNA), T-cells, T-cell populations, cytocotic cells, antigen-binding polypeptides, antigen presenting cells and exosomes as described *supra* are provided. Said nucleic acids (e.g., DNA or RNA), T-cells, T-cell populations, cytotoxic cells, antigen-binding polypeptides, antigen presenting cells and exosomes may be used in the treatment of cancer especially melanoma e.g. cutaneous melanoma or uveal melanoma. Related methods of diagnosis are also provided.

In an embodiment of the disclosure, the CLT antigen polypeptide comprises or consists of SEQ ID NO. 4. Exemplary fragments comprise or consist of any one of SEQ ID NOs. 16 and 17. Exemplary nucleic acids encoding said polypeptide sequence comprise or consists of SEQ ID NO. 26 or SEQ ID NO. 34. Corresponding nucleic acids (e.g., DNA or RNA), T-cells, T-cell populations, cytocotic cells, antigen-binding polypeptides, antigen presenting cells and exosomes as described *supra* are provided. Said nucleic acids (e.g., DNA or RNA), T-cells, T-cell populations, cytotoxic cells, antigen-binding polypeptides, antigen presenting cells and exosomes may be used in the treatment of cancer especially melanoma e.g. cutaneous melanoma. Related methods of diagnosis are also provided.

In an embodiment of the disclosure, the CLT antigen polypeptide comprises or consists of SEQ ID NO. 5. Exemplary fragments comprise or consist of SEQ ID NO. 18. Exemplary nucleic acids encoding said polypeptide sequence comprise or consists of SEQ ID NO. 27 or SEQ ID NO. 35. Corresponding nucleic acids (e.g., DNA or RNA), T-cells, T-cell populations, cytocotic cells, antigen-binding polypeptides, antigen presenting cells and exosomes as described *supra* are provided. Said nucleic acids (e.g., DNA or RNA), T-cells, T-cell populations, cytotoxic cells, antigen-binding polypeptides, antigen presenting cells and exosomes may be used in the treatment of cancer especially melanoma e.g. cutaneous melanoma or uveal melanoma. Related methods of diagnosis are also provided.

In an embodiment of the disclosure, the CLT antigen polypeptide comprises or consists of SEQ ID NO. 6. Exemplary fragments comprise or consist of any one of SEQ ID NOs. 19, 20. A further exemplary fragment comprises or consists of SEQ ID NO. 39. Exemplary nucleic acids encoding said polypeptide sequence comprise or consists of SEQ ID NO. 28 or SEQ ID NO. 36. Corresponding nucleic acids (e.g., DNA or RNA), T-cells, T-cell populations, cytocotic cells, antigen-binding polypeptides, antigen presenting cells and exosomes as described *supra* are provided. Said nucleic acids (e.g., DNA or RNA), T-cells, T-cell populations, cytotoxic cells, antigen-binding polypeptides, antigen presenting cells and exosomes may be used in the treatment of cancer especially melanoma e.g. cutaneous melanoma. Related methods of diagnosis are also provided.

In an embodiment of the disclosure, the CLT antigen polypeptide comprises or consists of SEQ ID NO. 7. Exemplary fragments comprise or consist of SEQ ID NO. 21. Exemplary nucleic acids encoding said polypeptide sequence comprise or consists of SEQ ID NO. 29 or SEQ ID NO. 37. Corresponding nucleic acids (e.g., DNA or RNA), T-cells, T-cell populations, cytocotic cells, antigen-binding polypeptides, antigen presenting cells and exosomes as described *supra* are provided. Said nucleic acids (e.g., DNA or RNA), T-cells, T-cell populations, cytotoxic cells, antigen-binding polypeptides, antigen presenting cells and exosomes may be used in the treatment of cancer especially melanoma e.g. cutaneous melanoma. Related methods of diagnosis are also provided.

In an embodimen of the disclosure t, the CLT antigen polypeptide comprises or consists of SEQ ID NO. 8. Exemplary fragments comprise or consist of SEQ ID NO. 22. Exemplary nucleic acids encoding said polypeptide sequence comprise or consists of SEQ ID NO. 30 or SEQ ID NO. 38. Corresponding nucleic acids (e.g., DNA or RNA), T-cells, T-cell populations, cytotoxic cells, antigen-binding polypeptides, antigen presenting cells and exosomes as described *supra* are provided. Said nucleic acids (e.g., DNA or RNA), T-cells, T-cell populations, cytotoxic cells, antigen-binding polypeptides, antigen presenting cells and exosomes may be used in the treatment of cancer especially melanoma e.g. cutaneous melanoma or uveal melanoma. Related methods of diagnosis are also provided.

**Examples**

Example 1 - CLT identification

The objective was to identify cancer-specific transcripts that entirely or partially consist of LTR elements.

As a first step, we *de novo* assembled a comprehensive pan-cancer transcriptome. To achieve this, RNA-sequencing reads from 768 patient samples, obtained from The Cancer Genome Atlas (TCGA) consortium to represent a wide variety of cancer types (24 gender-balanced samples from each of 32 cancer types (31 primary and 1 metastatic melanoma); Table S1), were used for genome-guided assembly. The gender-balanced samples (excluding gender-specific tissues) were adapter and quality (Q20) trimmed and length filtered (both reads of the pair ≥35 nucleotides) using cutadapt (v1.13) (Marcel M., 2011, EMBnet J., 17:3) and kmer-normalized (k=20) using khmer (v2.0) (Crusoe et al., 2015, F1000Res., 4:900) for maximum and minimum depths of 200 and 3, respectively. Reads were mapped to GRCh38 using STAR (2.5.2b) with settings identical to those used across TCGA and passed to Trinity (v2.2.0) (Trinity, Grabherr, M.G., et al., 2011, Nat. Biotechnol., 29:644-52) for a genome-guided assembly with inbuilt in silico depth normalization disabled. The majority of assembly processes were completed within 256GB RAM on 32-core HPC nodes, with failed processes re-run using 1.5TB RAM nodes. Resulting contigs were poly(A)-trimmed (trimpoly within SeqClean v110222) and entropy-filtered (≥0.7) to remove low-quality and artefactual contigs (bbduk within BBMap v36.2). Per cancer type, the original 24 samples were quasi-mapped to the cleaned assembly using Salmon (v0.8.2 or v0.9.2) (Patro, R., et al., 2017, Nat. Methods, 14:417-419), with contigs found expressed at <0.1 transcripts per million (TPM) being removed. Those remaining were mapped to GRCh38 using GMAP (v161107) (Wu et al., 2005, Bioinf., 21:1859-1875), and contigs not aligning with ≥85% identity over ≥85% of their length were removed from the assembly. Finally, assemblies for all cancer types together were flattened and merged into the longest continuous transcripts using gffread (Cufflinks v2.2.1) (Trapnell et al., 2010, Nat. Biotech., 28:511-515). As this assembly process was specifically designed to enable assessment of repetitive elements, monoexonic transcripts were retained, but flagged. Transcript assembly completeness and quality was assessed by comparison with GENCODE v24basic and MiTranscriptome1 (Iyer et al. 2015, Nat. Genet., 47: 199-208). We compiled the list of unique splice sites represented within GENCODE and tested if the splice site was present within the transcriptome assembly within a 2-nucleotide grace window. This process resulted in the identification of 1,001,931 transcripts, 771,006 of which were spliced and 230,925 monoexonic.

Separately, the assembled contigs were overlaid with a genomic repeat sequence annotation to identify transcripts that contain an LTR element. LTR and non-LTR elements were annotated as previously described (Attig et al., 2017, Front. In Microbiol., 8:2489). Briefly, hidden Markov models (HMMs) representing known Human repeat families (Dfam 2.0 library v150923) were used to annotate GRCh38 using RepeatMasker Open-3.0 (Smit, A., R. Hubley, and P. Green, http://www.repeatmasker.org, 1996-2010), configured with nhmmer (Wheeler et al., 2013, Bioinform., 29:2487-2489). HMM-based scanning increases the accuracy of annotation in comparison with BLAST-based methods (Hubley et al., 2016, Nuc. Acid. Res., 44:81-89). RepeatMasker annotates LTR and internal regions separately, thus tabular outputs were parsed to merge adjacent annotations for the same element. This process yielded 181,967 transcripts that contained one or more, complete or partial LTR element.

Transcripts per million (TPM) were estimated for all transcripts using Salmon and expression within each cancer type was compared with expression across 811 healthy tissue samples (healthy tissue-matched controls for all cancer types, where available, from TCGA and, separately from, GTEx (The Genotype-Tissue Expression Consortium, 2015, Science, 348:648-60). Transcripts were considered expressed in cancer if detected at more than 1 TPM in any sample and as cancer-specific if the following criteria were fulfilled: i, expressed in ≥6 of the 24 samples of each cancer type; ii, expressed at <10 TPM in ≥90% of all healthy tissue samples; iii, expressed in the cancer type of interest ≥3× the median expression in any control tissue type; and iv, expressed in the cancer type of interest ≥3× the 90th percentile of the respective healthy tissue, where available.

The list of cancer-specific transcripts was then intersected with the list of transcripts containing complete or partial LTR elements to produce a list of 5,923 transcripts that fulfilled all criteria (referred to as CLTs for Cancer-specific LTR element-spanning Transcripts).

Further curation was carried out on 403 CLTs specifically expressed in melanoma to exclude potentially misassembled contigs and those corresponding to the assembly of cellular genes. Additional manual assessment was conducted to ensure that splicing patterns were supported by the original RNA-sequencing reads from the cancer(s) in which they were determined to be specifically expressed. CLTs were additionally triaged such that those where the median expression in any GTEx normal tissue exceeded 1 TPM were discarded.

Within the 403 CLTs for cutaneous melanoma, 97 CLTs passed these filters.

Example 2 - Immunopeptidomic analysis

Mass spectrometry (MS)-based immunopeptidomics analysis is a powerful technology that allows for the direct detection of specific peptides associated with HLA molecules (HLAp) and presented on the cell surface. The technique consists of affinity purification of the HLAp from biological samples such as cells or tissues by anti-HLA antibody capture. The isolated HLA molecules and bound peptides are then separated from each other and the eluted peptides are analyzed by nano-ultra performance liquid chromatography coupled to mass spectrometry (nUPLC-MS) (Freudenmann et al., 2018, Immunology 154(3):331-345). In the mass spectrometer, specific peptides of defined charge-to-mass ratio (m/z) are selected, isolated, fragmented, and then subjected to a second round of mass spectrometry (MS/MS) to reveal the m/z of the resulting fragment ions. The fragmentation spectra (MS/MS) can then be interrogated to precisely identify the amino acid sequence of the selected peptide that gave rise to the detected fragment ions.

MS/MS spectral interpretation and subsequent peptide sequence identification relies on the match between experimental data and theoretical spectra created from peptide sequences found in a reference database. Although it is possible to search MS data by using pre-defined lists corresponding to all open reading frames (ORFs) derived from the known transcriptome or even the entire genome (Nesvizhskii et al., 2014, Nat. Methods 11: 1114-1125), interrogating these very large sequence databases leads to very high false discovery rates (FDR) that limit the identification of presented peptides. Further technical issues (e.g., mass of leucine = mass of isoleucine), and theoretical issues (e.g., peptide splicing (Liepe, et al., 2016, Science 354(6310): 354-358)) increase the limitations associated with use of very large databases, such as those produced from the known transcriptome or entire genome. Thus, in practice, it is exceptionally difficult to perform accurate immunopeptidomics analyses to identify novel antigens without reference to a well-defined set of potential polypeptide sequences (Li, et al., 2016, BMC Genomics 17 (Suppl 13):1031).

We thus constructed a database of all predicted polypeptide sequences (ORFs) of ≥10 residues from the 97 cutaneous melanoma CLTs of Example 1. This yielded 2,269 ORFs ranging in length from 10 to 207 amino acids.

Bassani-Sternberg et al. (Bassani-Sternberg et al., 2016, Nature Commun., 7: 13404; database link: https://www.ebi.ac.uk/pride/archive/projects/PXD004894) interrogated MS/MS data collected from HLA-bound peptide samples derived from 25 cutaneous melanoma patients against the polypeptide sequences reported for the entire human proteome. These analyses revealed tens of thousands of peptides that matched to known human proteins. As expected, these peptides included peptides found within multiple tumor-associated antigens (TAA), including PRAME, MAGEA3, and TRPM1 (melastatin).

The inventors procured frozen tumor tissue from 2 patients diagnosed with melanoma. Samples between 0.6-1 g were homogenized, the lysate was centrifugate at high speed and the cleared lysate was mixed with protein A (ProA) beads covalently linked to an anti-human HLA class I monoclonal antibody (W6/32). The mixture was incubated overnight at 4°C to improve HLA Class I molecule binding to antibody (Ternette et al., 2018 Proteomics 18, 1700465). The HLA Class I-bound peptides were eluted from the antibody by using 10% acetic acid, and the peptides were then separated from other high molecular mass components using reversed-phase column chromatography (Ternette et al., 2018). The purified, eluted peptides were subjected to nUPLC-MS, and specific peptides of defined charge-to-mass ratio (m/z) were selected within the mass spectrometer, isolated, fragmented, and subjected to a second round of mass spectrometry (MS/MS) to reveal the m/z of the resulting fragment ions (Ternette et al., 2018), producing an MS/MS dataset corresponding to the immunopeptidome for each of these tumor samples.

By applying detailed knowledge of immunopeptidomics evaluation, the inventors interrogated the spectra from the PXD004894 HLA Class I dataset for 25 melanoma patients (Bassani-Sternberg et al., 2016) and the spectra of the HLA-Class I dataset for the 10 melanoma tumors prepared by the inventors with the CLT-derived ORFs, of greater than 9AA alongside all polypeptide sequences found in the human proteome (UniProt) using PEAKS™ software (v8.5 and vX, Bioinformatics Solutions Inc). Since the majority of Class I HLA-bound peptides found in cells are derived from constitutively expressed proteins, the simultaneous interrogation of these databases with the UniProt proteome helps to ensure that assignments of our CLT ORF sequences to MS/MS spectra are correct. The PEAKS software, like other MS/MS interrogation software, assigns a probability value (-10IgP; see Table 1) to each assignment of spectra to quantify the assignment.

The results of these studies identified >50 individual peptides that were associated with the HLA Class I molecules immunoprecipitated from tumor samples from the 25 patients examined by Bassani-Sternberg et al. and the 2 melanoma patient samples in the inventors' dataset, that correspond to the amino acid sequence of CLT-derived ORFs, and did NOT correspond to polypeptide sequences present within the known human proteome (UniProt).

Further manual review of the peptide spectra assigned by the PEAKS software was used to confirm assignment of spectra to peptides that were mapped to 8 CLT-derived ORFs, and thus defined as CLT antigens (Table 1; SEQ ID NOs. 1-8).

The detection of these peptides associated with the HLA Class I molecules confirms, that the 8 ORFs from which they were derived, were first translated in melanoma tissues, processed through the HLA Class I pathway and finally presented to the immune system in a complex with HLA Class I molecules. Table 1 shows the properties of the peptides found in the CLT antigens. Figures 1-14 and 29-31 show representative MS/MS spectra from each of the peptides shown in Table 1. The top panel of each of these figures shows the MS/MS peptide fragment profile, with standard MS/MS annotations (b: N-terminal fragment ion; y: C-terminal fragment ion; $-H_2O$: water loss; $-NH_3$: loss of ammonia; [2+]: doubly charged peptide ion; pre: unfragmented precursor peptide ion; $a_n$-n: internal fragment ion) shown above the most abundant fragment ion peaks (images extracted by PEAKS™ software from the inventors' internal dataset or from Bassani-Sternberg et al. dataset stored in PRIDEdatabase link: https://www.ebi.ac.uk/pride/archive/projects/PXD004894). The lower panel of each figure shows a rendering of the spectrum indicating the positions of the linear peptide sequences that have been mapped to the fragment ions. Consistent with the high -10lgP scores assigned to the peptides in Table 1, these spectra contain numerous fragments that precisely match the sequences of the peptides (SEQ ID Nos. 9-22) that we discovered in these analyses.

All of the peptides detected in association with HLA Class I molecules from Table 1 that were over 9AA in length were assessed to determine their predicted strength of binding to HLA Class I type A and B supertypes by using the NetMHCpan 4.0 prediction software (http://www.cbs.dtu.dk/services/NetMHCpan). The results of these prediction studies showed that all 14 peptides (or 9-mers contained within each full sequence) were predicted to bind to at least one of the supertypes tested (see Table 2). Amongst these, many of sequences were predicted to bind with high confidence (low % rank scores) to specific types within the HLA Class I supertypes examined. The fact that all of the detected peptides were expected to bind to HLA types that were expected to be in the patient population is consistent with their detection. Moreover, although the HLA types were only reported by Bassani-Sternberg et al. for a fraction (20%) of the tumor samples interrogated, every peptide identified in a tumor sample of a typed patient or discovered in a tumor sample from the inventors' dataset was predicted by NetMHCpan 4.0 to bind to one of the HLA types reported for that patient.

To provide further certainty of the assignment of tumor tissue-derived MS spectra to the peptide sequences that we discovered, peptides with these discovered sequences were synthesized and subjected to nUPLC-MS$^2$ using the same conditions applied to the tumor samples in the original study (Bassani-Sternberg et al., 2016, Nature Commun., 7: 13404; Inventors' database). Comparison of the spectra for selected peptides are shown in Figures 15-28. In each figure the upper spectrum corresponds to the tumor sample (from the PRIDE database (Bassani-Sternberg et al., 2016, Nature Commun., 7: 13404; database link: https://www.ebi.ac.uk/pride/archive/projects/PXD004894 or in the inventors' database) and the lower spectrum corresponds to the synthetically produced peptide of the same sequence. Selected m/z values of detected ion fragments are shown above/below each fragment peak in these MS/MS spectra. These figures reveal a precise alignment of fragments (tiny differences in the experimentally determined m/z values between tumor- and synthetic peptide-derived fragment ions being well within the m/z tolerances of <0.05 Daltons), confirming the veracity of the assignment of each of the tumor tissue-derived spectra to the CLT-encoded peptides.

Taken together, the data shown in Tables 1 & 2, Figures 1-14 and 29-31, and Figures 15-28 supply exceptionally strong support for the translation, processing, and presentation of the corresponding CLT antigens in melanoma patients.

To further confirm the cancer-specificity of these CLTs, the inventors processed 37 normal tissue samples (10 normal skin, 9 normal lung and 18 normal breast tissue) and prepared for immunopeptidomic analysis. The inventors interrogated the spectra of the HLA-Class I dataset from these normal tissue samples, searching for all possible peptide sequences derived from the polypeptide sequences of CLT antigens 1 to 8. No peptides derived from these CLT Antigens were detected in the set of normal tissue samples (Table 3) providing additional confirmation that the CLTs have cancer-specific expression.

In summary: the identification of immunopeptidomic peptides derived from the predicted ORFs, demonstrates that these CLTs are translated into polypeptides (SEQ ID NOs. 1-8; referred to as CLT antigens) in tumor tissue. These are then processed by the immune surveillance apparatus of the cells, and component peptides are loaded onto HLA Class I molecules, enabling the cell to be targeted for cytolysis by T cells that recognize the resulting peptide/HLA Class I complexes. Thus, these CLT antigens and fragments of them are expected to be useful in a variety of therapeutic modalities for the treatment of melanoma in patients whose tumors express these antigens.

Table 1: List of peptides identified by immunopeptidomic analyses of melanoma tumor samples, along with CLT antigen name and cross reference to SEQ ID NOs.

| Peptide sequence[1] | Peptide SEQ ID NO. | CLT Ant. NO. | CLT Ant. SEQ ID NO. | Patient #[2] | Peptide mass[3] | Peptide length | -10lgP[4] | # of spectra[5] | Ppm[6] |
|---|---|---|---|---|---|---|---|---|---|
| GLNSIIWRL | SEQ ID NO. 9 | 1 | SEQ ID NO. 1 | Mel27 | 1070.6 237 | 9 | 23.25 | 1 | 9.1 |
| ILIQTTGIFK | SEQ ID NO. 10 | 1 | SEQ ID NO. 1 | Mel20 | 1132.6 855 | 10 | 22.89 | 1 | 2.8 |
| ILIQTTGIFK | SEQ ID NO. 10 | 1 | SEQ ID NO. 1 | Mel21 | 1132.6 855 | 10 | 27.33 | 4 | 7 |
| ILIQTTGIFK | SEQ ID NO. 10 | 1 | SEQ ID NO. 1 | Mel36 | 1132.6 855 | 10 | 20.36 | 2 | 2.3 |
| ILIQTTGIFK | SEQ ID NO. 10 | 1 | SEQ ID NO. 1 | Mel39 | 1132.6 855 | 10 | 20.36 | 2 | 2.9 |
| ILIQTTGIFK | SEQ ID NO. 10 | 1 | SEQ ID NO. 1 | Mel8 | 1132.6 855 | 10 | 17.68 | 2 | 6.4 |
| ATIFPDPWLLK | SEQ ID NO. 11 | 2 | SEQ ID NO. 2 | Mel41 | 1299.7 227 | 11 | 36.89 | 3 | 8.5 |
| FPFYKDTVL | SEQ ID NO. 12 | 2 | SEQ ID NO. 2 | Mel41 | 1128.5 854 | 9 | 31.32 | 5 | 8.3 |
| FPFYKDTVLL | SEQ ID NO. 13 | 2 | SEQ ID NO. 2 | Mel41 | 1241.6 696 | 10 | 29.36 | 1 | 9.2 |
| TIFPDPWLLK | SEQ ID NO. 14 | 2 | SEQ ID NO. 2 | Mel41 | 1228.6 855 | 10 | 31.9 | 3 | 8.9 |
| IVLDAPVTK | SEQ ID NO. 15 | 3 | SEQ ID NO. 3 | Mel21 | 954.57 5 | 9 | 19.95 | 1 | 8.7 |
| IVLDAPVTK | SEQ ID NO. 15 | 3 | SEQ ID NO. 3 | Mel36 | 954.57 5 | 9 | 22.36 | 2 | 1.6 |
| IVLDAPVTK | SEQ ID NO. 15 | 3 | SEQ ID NO. 3 | Mel39 | 954.57 5 | 9 | 20.11 | 2 | 1.3 |
| IVLDAPVTK | SEQ ID NO. 15 | 3 | SEQ ID NO. 3 | 2MT3 | 954.57 5 | 9 | 24.77 | 7 | 0.3 |
| RITSGVKVK | SEQ ID NO. 16 | 4 | SEQ ID NO. 4 | Mel21 | 986.62 37 | 9 | 28.05 | 3 | 7.3 |

(continued)

| Peptide sequence[1] | Peptide SEQ ID NO. | CLT Ant. NO. | CLT Ant. SEQ ID NO. | Patient #[2] | Peptide mass[3] | Peptide length | -10lgP[4] | # of spectra[5] | Ppm[6] |
|---|---|---|---|---|---|---|---|---|---|
| RITSGVKVKK | SEQ ID NO. 17 | 4 | SEQ ID NO. 4 | Mel21 | 1114.7 186 | 10 | 20.66 | 1 | 8.2 |
| ALRAVTLTAK | SEQ ID NO. 18 | 5 | SEQ ID NO. 5 | Mel15 | 1042.6 499 | 10 | 30.04 | 11 | 5.6 |
| ALRAVTLTAK | SEQ ID NO. 18 | 5 | SEQ ID NO. 5 | Mel39 | 1042.6 499 | 10 | 23.93 | 1 | 2.7 |
| GHIHNEAV | SEQ ID NO. 19 | 6 | SEQ ID NO. 6 | Mel27 | 875.42 49 | 8 | 21.32 | 2 | 7.9 |
| SLYGHIHNEAV | SEQ ID NO. 20 | 6 | SEQ ID NO. 6 | Mel27 | 1238.6 044 | 11 | 35.11 | 1 | 8.5 |
| SLYGHIHNEAV | SEQ ID NO. 20 | 6 | SEQ ID NO. 6 | 2MT4 | 1238.6 04 | 11 | 56.34 | 2 | 4.7 |
| APSKDHPLYTA | SEQ ID NO. 21 | 7 | SEQ ID NO. 7 | Mel25 | 1198.5 981 | 11 | 21.03 | 1 | 3 |
| APSKDHPLYTA | SEQ ID NO. 21 | 7 | SEQ ID NO. 7 | 2MT3 | 1198.5 981 | 11 | 28.79 | 5 | 0.4 |
| RLFSEDIHV | SEQ ID NO. 22 | 8 | SEQ ID NO. 8 | Mel25 | 1114.5 77 | 9 | 24.34 | 2 | 2 |

[1] HLA Class I peptides identified by mass spectrometry.
[2] Bassani-Sternberg *et al*, 2016, Nature Comm., 7: 13404. Inventors' dataset (2MT3, 2MT4)
[3] Calculated peptide mass.
[4] PEAKS™ program -10lgP values are shown for peptides for highest match for peptide/patients for which more than one spectral detection was obtained.
[5] Number of spectra in which peptide was detected.
[6] Deviation between observed mass and calculated mass; selected ppm values are shown for peptides for which more than one spectrum was obtained.

Table 2: Predicted NetMHCpan4.0 binding of Mass Spectrometry-identified peptides (length ≥ 9 residues) to 18 HLA Class I Supertype Alleles (HLA-A0101, HLA-A0201, HLA-A0301, HLA-A1101, HLA-A2402, HLA-A2501, HLA-A2601, HLA-A6801, HLA-B0702, HLA-B0801, HLA-B1501, HLA-B1801, HLA-B2705, HLA-B3501, HLA-B3503, HLA-B4001, HLA-B4002, HLA-B5101), along with CLT antigen name and cross reference to SEQ ID NOs.

| | Peptide sequence [1] | Number of alleles with a rank score of ≤2%[2] | Number of alleles with a rank score of ≤0.5%[3] | Peptide SEQ ID NO. | CLT Antigen NO. | Patient #[4] |
|---|---|---|---|---|---|---|
| YES | GLNSIIWRL | 1 | 1 | SEQ ID NO. 9 | 1 | Mel27 |

(continued)

| | Peptide sequence [1] | Number of alleles with a rank score of ≤2%[2] | Number of alleles with a rank score of ≤0.5%[3] | Peptide SEQ ID NO. | CLT Antigen NO. | Patient #[4] |
|---|---|---|---|---|---|---|
| YES | ILIQTTGIFK | 4 | 2 | SEQ ID NO. 10 | 1 | Mel8, Mel20, Mel21, Mel36, Mel39 |
| YES | ATIFPDPWLLK | 7 | 1 | SEQ ID NO. 11 | 2 | Mel41 |
| YES | FPFYKDTVL | 3 | 3 | SEQ ID NO. 12 | 2 | Mel41 |
| YES | FPFYKDTVLL | 4 | 4 | SEQ ID NO. 13 | 2 | Mel41 |
| YES | TIFPDPWLLK | 6 | 1 | SEQ ID NO. 14 | 2 | Mel41 |
| YES | IVLDAPVTK | 1 | 1 | SEQ ID NO. 15 | 3 | Mel21, Mel34, Mel39 |
| YES | RITSGVKVK | 1 | 1 | SEQ ID NO. 16 | 4 | Mel21 |
| YES | RITSGVKVKK | 2 | 2 | SEQ ID NO. 17 | 4 | Mel21 |
| YES | ALRAVTLTAK | 2 | 0 | SEQ ID NO. 18 | 5 | Mel15, Mel39 |
| YES | SLYGHIHNEAV | 3 | 0 | SEQ ID NO. 20 | 6 | Mel27 |
| YES | APSKDHPLYTA | 3 | 0 | SEQ ID NO. 21 | 7 | Mel25 |
| YES | RLFSEDIHV | 2 | 1 | SEQ ID NO. 22 | 8 | Mel25 |

[1] Predicted binding to interrogated HLA Class I supertypes at a Rank score of ≤ 2.0%score.
[2] Number of the 18 HLA Class I supertypes that were predicted to bind with a rank score of ≤2.0% (all binding).
[3] Number of the 18 HLA Class I supertypes that were predicted to bind with a rank score of ≤0.5% (strong binding).
[4] Bassani-Sternberg et al, 2016, Nature Comm., 7: 13404

Table 3 Number of peptides-derived from CLT Antigens 1 to 8 in a set of normal tissue samples.

| Antigen | Skin | Lung | Breast |
|---|---|---|---|
| CLT Antigen 1 | 0/10 | 0/9 | 0/18 |
| CLT Antigen 2 | 0/10 | 0/9 | 0/18 |
| CLT Antigen 3 | 0/10 | 0/9 | 0/18 |
| CLT Antigen 4 | 0/10 | 0/9 | 0/18 |
| CLT Antigen 5 | 0/10 | 0/9 | 0/18 |
| CLT Antigen 6 | 0/10 | 0/9 | 0/18 |
| CLT Antigen 7 | 0/10 | 0/9 | 0/18 |
| CLT Antigen 8 | 0/10 | 0/9 | 0/18 |

The results presented here in Examples 1 and 2 are in whole or part based upon data generated by the The Cancer Genome Atlas (TCGA) Research Network (http://cancergenome.nih.gov/); and the Genotype-Tissue Expression (GTEx) Project (supported by the Common Fund of the Office of the Director of the National Institutes of Health, and by NCI, NHGRI, NHLBI, NIDA, NIMH, and NINDS).

Example 3 - Assays to demonstrate T cell specificity for CLT antigens in melanoma patients

(a) Staining reactive T cells with CLT antigen peptide pentamers

The presence and activity of circulating CD8 T cells specific for CLT antigens in melanoma patients can be measured by using HLA Class I/peptide-pentamer ("pentamer") staining and/or *in vitro* killing assays. Thus, application of these methodologies to CLT antigens discovered using the methods elucidated in Example 1 & 2 (Table 1-3, Figures 1-31) can be used to demonstrate the existence of therapeutically relevant T cell responses to the CLT antigens in cancer patients.

For these studies, CD8 T cells isolated from patient blood are expanded using various cultivation methods, for example anti-CD3 and anti-CD28 coated microscopic beads plus Interleukin-2. Expanded cells can then be stained for specific CLT antigen-reactivity of their T cell receptors using CLT peptide pentamers, which consist of pentamers of HLA Class I molecules bound to the relevant CLT Antigen peptide in the peptide-binding groove of the HLA molecule. Binding is measured by detection with phycoerythrin or allophycocyanin-conjugated antibody fragments specific for the coiled-coil multimerisation domain of the pentamer structure. In addition to the pentamer stain, further surface markers can be interrogated such as the memory marker CD45RO and the lysosomal release marker CD107a. Association of pentamer positivity with specific surface markers can be used to infer both the number and state (memory versus naive/stem) of the pentamer-reactive T cell populations

Pentamer stained cells may also be sorted and purified using a fluorescence activated cell sorter (FACS). Sorted cells may then be further tested for their ability to kill target cells in *in vitro* killing assays. These assays comprise a CD8 T cell population, and a fluorescently labelled target cell population. In this case, the CD8 population is either CLT antigen-specific or CD8 T cells pentamer-sorted and specific for a positive-control antigen known to induce a strong killing response such as Mart-1. The target cells for these studies may include peptide-pulsed T2 cells which express HLA-A*02, peptide-pulsed C1R cells transfected with HLA-A*02,03 or B*07, melanoma cells lines previously shown to express the CLTs/CLT antigens, or patient tumor cells or cell lines such as CaSki transfected with the CLT open reading frames. Target cell death is indicated by take up of 7AAD. In this way, as target cells are killed, by apoptosis mediated by CD8 T-cells, they gain red fluorescence. Thus, application of such killing assays to pentamer-sorted, CLT antigen-specific CD8 T-cells can be used to enumerate the cytotoxic activity of CLT-antigen-specific T-cells in *ex vivo* cultures of melanoma patient or healthy donor T-cells.

Figure 32 shows HLA pentamer staining of healthy donor CD8 T-cells with peptides-derived from CLT Antigen 6, peptide SLYGHIHNEA (SEQ ID NO. 39) following fluorescence activated cell sorting of pentamer positive cells and 14 days of expansion using anti-CD3 and anti-CD28 coated beads plus IL-2. The right-hand side panel shows very weak antigen-specific killing of peptide-pulsed A2 target cells by these CD8+ T cells but effective antigen specific killing of CaSki cells transfected with the open reading frame of CLT Antigen 6. The negative controls for this in vitro killing assay include an irrelevant T2 cells with no peptide and untransfected CaSki cells.

(b) HERVfest analyses of T cell specificity in melanoma patients

Functional expansion of specific T cells (fest) technology has been used identify specific tumor-derived epitopes present in the "mutation-associated neoantigen" (MANA) repertoire found in tumor cells of patients who have responded to checkpoint-blockade therapies (Anagnostou et al., Cancer Discovery 2017; Le et al., Science 2017). Application of this technology to CLT antigens discovered using the methods elucidated in Examples 1 & 2 (Tables 1 to 3, Figures 1-31) can confirm the existence of therapeutically relevant T cell responses to the CLT antigens in cancer patients.

Like other assays (e.g., ELISPOT) to identify epitope-specific T cells in a subject who has undergone immune exposure, "fest" technologies derive their specificity by expanding the cognate T cells in *ex vivo* cultures that include antigen-presenting cells and suitable antigenic peptides. The technique differs from other immunological assays in that it utilizes next-generation sequencing of the T cell receptor (TCR) mRNA present in these amplified cultures (specifically: TCRseq targeting the TCR-Vβ CDR3 region) to detect the specific TCRs that are expanded in the cells cultured with the target peptides (preselected to match the HLA type of the patient, using standard HLA-binding algorithms). Application of TCRseq to tumor tissues in the same patient, harvested after successful checkpoint-blockade therapy, can then be used to determine which TCRs/T cells detected in the *ex vivo,* peptide-stimulated cultures, are also present at the site of immune-suppression of the cancer. In the case of MANAfest, the method is used to identify specific TCRs that recognize MHC-presented neoantigen peptides that evolve in each patient's tumor and are also detected in the T cells in the patients' tumors, permitting the identification of the functionally relevant neoantigens peptides among the thousands of possible mutant peptides found by full-exome sequencing of normal and tumor tissues from each patient (Le et al., Science 2017).

Application of MANAfest (Anagnostou et al., 2017 Cancer Discovery) technology to CLT antigens is done as follows. Step 1: Peptides predicted to contain epitopes that efficiently bind selected HLA supertypes are identified in CLT antigens. Step 2: PBMCs from appropriate patients are selected, and matched by HLA type to the peptide library selected in step 1. Step 4: PBMCs from these patients are separated into T cell and non-T cell fractions. Non-T cells are irradiated (to prevent proliferation), added back to the patient's T cells, and then divided into 20-50 samples, and cultivated in T cell growth factors and individual CLT-specific synthetic peptides (selected in step 1) for 10 to 14 days. Step 4: TCRseq (sequencing of the epitope-specific TCR-Vβ CDR3 sequences) is performed on all wells to identify the cognate T cells/TCRs that have been amplified in the presence of the test peptides, specificity of these TCRs is determined by comparison to TCRs detected in unamplified/propagated T cells using TCRseq. Data obtained from this step can confirm which peptides elicited an immune response in the patient. Step 5: TCRseq is performed on tumor samples to determine which of the specifically amplified TCRs homed to the tumor of patients who have responded to checkpoint-blockade therapy, providing evidence that T cells bearing this TCRs may contribute to the effectiveness of the checkpoint blockade therapy.

Example 4 - Assays to demonstrate high-affinity T cells specific for CLT antigens have not been deleted from normal subjects' T cell repertoire

An ELISPOT assay may be used to show that CLT antigen-specific CD8 T cells are present in the normal T cell repertoire of healthy individuals, and thus have not been deleted by central tolerance due to the expression of cancer-specific CLT antigens in naïve and thymic tissues in these patients. This type of ELISPOT assay comprises multiple steps. Step 1: CD8 T cells and CD14 monocytes can be isolated from the peripheral blood of normal blood donors, these cells are HLA typed to match the specific CLT antigens being tested. CD8 T cells can be further sub-divided into naïve and memory sub-types using magnetically labelled antibodies to the memory marker CD45RO. Step 2: CD14 monocytes are pulsed with individual or pooled CLT antigen peptides for three hours prior to being co-cultured with CD8 T cells for 14 days. Step 3: Expanded CD8 T cells are isolated from these cultures and re-stimulated overnight with fresh monocytes pulsed with peptides. These peptides may include; individual CLT antigen peptides, irrelevant control peptides or peptides known to elicit a robust response to infectious (e.g., CMV, EBV, Flu, HCV) or self (e.g. MART-1) antigens. Re-stimulation is performed on anti-Interferon gamma (IFNγ) antibody-coated plates. The antibody captures any IFNγ secreted by the peptide-stimulated T cells. Following overnight activation, the cells are washed from the plate and IFNγ captured on the plate is detected with further anti- IFNγ antibodies and standard colorimetric dyes. Where IFNγ -producing cells were originally on the plate, dark spots are left behind. Data derived from such assays includes spot count, median spot size and median spot intensity. These are measures of frequency of T cells producing IFNγ and amount of IFNγ per cell. Additionally, a measure of the magnitude of the response to the CLT antigen can be derived from the stimulation index (SI) which is the specific response, measured in spot count or median spot size, divided by the background response to monocytes with no specific peptide. A metric of stimulation strength is derived by multiplying the stimulation index for spot number by the stimulation index for spot intensity. In this way, comparisons of the responses to CLT antigens and control antigens can be used to demonstrate that naïve subjects contain a robust repertoire of CLT antigen-reactive T-cells that can be expanded by vaccination with CLT antigen-based immunogenic formulations. Table 4 provides a list of CLT Antigen-derived peptides that induced significant CD8 T-cell responses from HLA-matched normal blood donors. The results are shown in Figures 33-38. Horizontal bars represent the mean of the data. M+t indicates the no peptide, negative control (monocytes and T cells). CEF indicates the positive control (a mixture of 23 CMV, EBV and influenza peptides). Statistical significance was measured with Kruskall Wallis test One-way Anova with correction for repeated measures with Dunns correction. Figure 33 shows significant CD8 T-cell responses from a normal blood donor to an HLA-A *02:01-restricted peptide from CLT Antigen 1 (CLT001 in the Figure). Figure 34 shows significant CD8 T-cell responses from a normal blood donor to an HLA-A*0301-restricted peptide from CLT Antigen 1 (CLT001 in the Figure). Figure 35 shows significant CD8 T-cell responses from a normal blood donor to an HLA-B*0702-restricted peptide from CLT Antigen 2 (CLT002 in the Figure). Figure 36 shows significant CD8 T-cell responses from a normal blood donor to an HLA-A*0301-restricted peptide from CLT Antigen 3 (CLT003 in the Figure). Figure 37 shows significant CD8 T-cell responses from a normal blood donor to an HLA-A*0301-restricted peptide from CLT Antigen 5 (CLT005 in the Figure). Figure 38 shows significant CD8 T-cell responses from a normal blood donor to an HLA-A*0201-restricted peptide from CLT Antigen 6 (CLT006 in the Figure).

Table 4: CLT Antigen-derived peptides that induced significant CD8 T-cell responses from HLA-matched normal blood donors

| Reference CLT Antigen | SEQ ID NO | Peptide sequence | HLA-type for peptide (predicted by NetMHC) |
|---|---|---|---|
| 1 | SEQ ID. NO. 9 | GLNSIIWRL | HLA-A*02:01 |
| 1 | SEQ ID. NO. 10 | ILIQTTGIFK | HLA-A*03:01 |

(continued)

| Reference CLT Antigen | SEQ ID NO | Peptide sequence | HLA-type for peptide (predicted by NetMHC) |
|---|---|---|---|
| 2 | SEQ ID. NO. 13 | FPFYKDTVLL | HLA-B*07:02 |
| 3 | SEQ ID. NO. 15 | IVLDAPVTK | HLA-A*03:01 |
| 5 | SEQ ID. NO. 18 | ALRAVTLTAK | HLA-A*03:01 |
| 6 | SEQ ID. NO. 39 | SLYGHIHNEA | HLA-A*02:01 |

Example 5 - Assays to validate CLT expression in melanoma cells

a) qRT-PCR validation of CLT expression in melanoma cell lines

Quantiative real-time polymerase chain reaction (qRT-PCR) is a widespread technique to determine the amount of a particular transcript present in RNA extracted from a given biological sample. Specific nucleic acid primer sequences are designed against the transcript of interest, and the region between the primers is subeqeuntly amplified through a series of thermocyle reactions and fluorescently quantified through the use of intercalating dyes (SYBR Green). Primer pairs were designed against the CLTs and assayed against RNA extracted from melanoma cell lines or primary patient tissue. Non-melanoma cell lines were utilised as negative controls. Melanoma cell lines used included COLO 829 (ATCC reference CRL-1974), MeWo (ATCC reference HTB-65), SH-4 (ATCC reference CRL-7724) and control cell lines HepG2 (hepatocellular carcinoma, ATCC reference HB-8065), Jurkat (T-cell leukemia, ATCC reference TIB152) and MCF7 (adenocarcinoma, ATCC reference HTB-22). Patient-derived melanoma tissue was obtained from 6 primary lesions and 6 metastases, all from patients with at least stage IIC disease. RNA was extracted from each sample and reverse transcribed into cDNA following standard procedures. qRT-PCR analysis WITH SYBR Green detection following standard techniques was performed with primers designed against two regions of each CLT, and reference genes. Relative quantification (RQ) was calculated as:

$$RQ = 2[Ct(REFERENCE)-Ct(TARGET)].$$

The results of these experiments are presented in Figure 39. Panel A shows results from qRT-PCR assay with two primer sets (76+77 AND 78+79) targeting different regions of the CLT encoding CLT Antigen 2 (SEQ ID NO. 24) on RNA extracted from 12 melanoma tissue samples and one non-melanoma cell line. Panel B shows results from qRT-PCR assay with two primer sets (44+45 AND 46+47) targeting different regions of the CLT encoding CLT Antigen 3 (SEQ ID NO. 25) on RNA extracted from 12 melanoma tissue samples and one non-melanoma cell line. Panel C shows results from qRT-PCR assay with two primer sets (80-81 AND 82-83) targeting different regions of the CLT encoding CLT Antigen 6 (SEQ ID NO. 28) on RNA extracted from 12 melanoma tissue samples and one non-melanoma cell line. These results confirmed the specific expression of CLTs in RNA extracted from melanoma tissue samples, compared to non-melanoma cell lines. Each CLT was detected in two or more tissue samples analysed, with little to no expression detected in non-melanoma control cell lines.

b) RNAScope validation of CLT expression in melanoma cells in situ

In situ hybridisation (ISH) methods of transcript expression analysis allow the presence and expression levels of a given transcript to be visualised within the histopathological context of a specimen. Traditional RNA ISH assays involve the recognition of native RNA molecules in situ with oligonucleotide probes specific to a short stretch of the desired RNA sequence, which are visualised through a signal produced by a combination of antibody or enzymatic-based colorimetric reactions. RNAScope is a recently developed in situ hybridization-based technique with more advanced probe chemistry ensuring specificity of the signal produced and allowing sensitive, single-molecule visualization of target transcripts (Wang et al 2012 J Mol Diagn. 14(1): 22-29). Positive staining for a transcript molecule appears as a small red dot in a given cell, with multiple dots indicative of multiple transcripts present.

RNAScope probes were designed against the CLTs and assayed on sections of 12 formalin-fixed, paraffin-embedded cutaneous melanoma tumour cores. Scoring of the expression signal was performed on representative images from each core as follows:

- Estimated % cells with positive staining for the CLT probe, rounded up to the nearest 10
- Estimated level of per cell expression across the given section as:

- 0 = no staining
- 1 = 1-2 dots per cell
- 2 = 2-6 dots per cell
- 3 = 6-10 dots per cell
- 4 = > 10 dots per cell

Expression of each of each CLT was detected across a number of different patient tumour cores, independently validating the discovery of CLTs from tumour-derived RNAseq data and confirming homogeneity of expression within tumour tissue across certain samples and also highlighting the presence of at least one CLT in each patient core analysed (Table 5).

Table 5 - Scoring of RNAScope in melanoma patient tissue cores

| Tissue | Core | CLT Antigen 2 (SEQ ID NO. 24) | | CLT Antigen 3 (SEQ ID NO. 25) | | CLT Antigen 6 (SEQ ID NO. 28) | |
|---|---|---|---|---|---|---|---|
| | | % of (+)ve cells | Score | % of (+)ve cells | Score | % of (+)ve cells | Score |
| Melanoma | 61 | 0 | 0 | 0 | 0 | 10 | 1 |
| Melanoma | 62 | 90 | 3 | 20 | 1 | 10 | 1 |
| Melanoma | 63 | 100 | 4 | 0 | 0 | 80 | 2 |
| Melanoma | 64 | 50 | 1 | 0 | 0 | 40 | 2 |
| Melanoma | 65 | 50 | 2 | 30 | 2 | 50 | 2 |
| Melanoma | 66 | 100 | 4 | 50 | 1 | 50 | n/a |
| Melanoma | 67 | n/a | n/a | n/a | n/a | n/a | n/a |
| Melanoma | 68 | 40 | 1 | 0 | 0 | 50 | 1 |
| Melanoma | 69 | 80 | 3 | 80 | 1 | 50 | 1 |
| Melanoma | 70 | 10 | 1 | 20 | 1 | 10 | 1 |
| Melanoma | 71 | 0 | 0 | 0 | 0 | 10 | 1 |
| Melanoma | 72 | 100 | 3 | 0 | 0 | 10 | 1 |

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

SEQUENCE LISTING

SEQ ID NO. 1 (Polypeptide sequence of CLT Antigen 1)

MKQTEILIQTTGIFKCIAMIMTTGKEREEGNKEKPRKLFGVRDYGNIKGYNVVKSWG
LNSIIWRLLNVSETACSSSPHKLGAT

SEQ ID NO. 2 (Polypeptide sequence of CLT Antigen 2)

MWNSLEARSPKSRCCSTGPWEAVQENLLWASLLAPGGATIFPDPWLLKASPSSLP
HLHRTSPCACLCPNFPFYKDTVLLHQGPR

SEQ ID NO. 3 (Polypeptide sequence of CLT Antigen 3)

MAMGQRTPSLAELRKSSATFLTCNLGAQAEKRSRAPGKLTYVSTIVLDAPVTKLEQ

GLVMKRYKIVTQGFDYTSVES

SEQ ID NO. 4 (Polypeptide sequence of CLT Antigen 4)
MIVRPPQPCGTKCLRITSGVKVKKKTVNCVLTALKNEKSCQAK

SEQ ID NO. 5 (Polypeptide sequence of CLT Antigen 5)

MSPPGGRNNSRHAALRAVTLTAKVCSFTPEPVRPRTRHKEETLNTSSEHQKEQTP

DAPP

SEQ ID NO. 6 (Polypeptide sequence of CLT Antigen 6)
MCALQGRGASPAGAGLFHWTMSPFLLGSLYGHIHNEAV

SEQ ID NO. 7 (Polypeptide sequence of CLT Antigen 7)
MRFGDLSSLLKALTLKDFMKLKFKSRCAPSKDHPLYTATCCFPWIKKHKEPSSTT

SEQ ID NO. 8 (Polypeptide sequence of CLT Antigen 8)

MNSPVKKWAKNMNRLFSEDIHVANKHMKNYSKSQVRKEMRMKAIMKYHHTPVRM

ATIKK

SEQ ID NO. 9 (peptide sequence derived from CLT Antigen 1)
GLNSIIWRL

SEQ ID NO. 10 (peptide sequence derived from CLT Antigen 1)
ILIQTTGIFK

SEQ ID NO. 11 (peptide sequence derived from CLT Antigen 2)
ATIFPDPWLLK

SEQ ID NO. 12 (peptide sequence derived from CLT Antigen 2)
FPFYKDTVL

SEQ ID NO. 13 (peptide sequence derived from CLT Antigen 2)
FPFYKDTVLL

SEQ ID NO. 14 (peptide sequence derived from CLT Antigen 2)
TIFPDPWLLK

SEQ ID NO. 15 (peptide sequence derived from CLT Antigen 3)
IVLDAPVTK

SEQ ID NO. 16 (peptide sequence derived from CLT Antigen 4)
RITSGVKVK

SEQ ID NO. 17 (peptide sequence derived from CLT Antigen 4)
RITSGVKVKK

SEQ ID NO. 18 (peptide sequence derived from CLT Antigen 5)
ALRAVTLTAK

SEQ ID NO. 19 (peptide sequence derived from CLT Antigen 6)
GHIHNEAV

SEQ ID NO. 20 (peptide sequence derived from CLT Antigen 6)
SLYGHIHNEAV

SEQ ID NO. 21 (peptide sequence derived from CLT Antigen 7)
APSKDHPLYTA

SEQ ID NO. 22 (peptide sequence derived from CLT Antigen 8)
RLFSEDIHV

SEQ ID NO. 23 (cDNA sequence of CLT encoding CLT Antigen 1)

GTTTTTGCCATCTGTAGTGTTGCCCTTAGCAACAGAAAAGCATGTGACAGTGCT
TATGTGACTCTGCTGGCCTGCAACAGTGTGTAGAAGGATTTAAGCAAAATGAAA
CAGACAGAAATATTGATCCAGACAACTGGAATTTTCAAGTGTATTGCAATGATAA
TGACGACTGGAAAGGAAAGAGAGGAAGGTAACAAAGAAAGCCAAGAAACTA

TTTGGAGTGAGAGACTATGGTAATATAAAAGGCTATAATGTGGTAAAATCGTGG

GGACTTAATTCCATCATATGGAGGTTACTGAATGTATCAGAGACAGCCTGCAGT

TCATCACCACACAAATTAGGAGCAACGTAAGAAGAGAAAAGAAAAGGAATTA

CAGATTAAAAAATGCAAGAGAATATAAACAAATAAAAATATGTTCAACTGGAATT

TGTATTGGGATTGTATTAAATGGATGAACTAATTTGTGAAGAAATGATATCATTC

CATCCAAGAGCATGGAACTCCTATTTTTCCTATTGTCTTCTATGTTTCTTTTTAAA

AAGTCTTAAGTTTTCTCTGTAGAGTTTTTTGCATTCTGGATGATTTTAATTGAAAC

ATACTGTATAGATATTGTTACTATGATAAGTGGTGCCTCTTATTAGATTTTTTAAT

TCATTATGATTGGTAAAGCAAATAGGAAAGATTTTGAGCATAGAAAACTGCCTCA

ATTACCTCATTACTTCAAAAAGTATTGCCATTGGTTCTATTGCTTTTTAAAAAAAT

ACATGATGATATCTTCTGCACATTATGAAATTTTATCTCAACACTTTCAATCACCG

CATCTCTGTTTGACACTGATGAACATTTCCAAAGTGAAATATTTTGCTCTCTTTAT

TTTTCTCCTTTTGCTCTTTGGGGCCTCTTTACTTTCTAAACTTGTCACTTCTTGGT

TTATCTTCCAAACCTCTTGGCTTTTCTTTTGTGTAGTATACTTTAGCTTCATTCTG

GGAGAGTTTTTCATTCTGAACTTTTCGATTCACTAGTTTATTCTCCATCAGTATGT

ATGCTAATTTTTCTTCCATTGACTTTTTTTCCCAAACACTTATTTTTCATACTTTAT

ATTTTCACTTGGAATTTTGGGGGAATATTTTGTTCTAGTTTCAAATTGATCCTATA

TTTTTTGTCTCCTTAAGTATATTTATCAGGCTTATTTTAAATTCTTGATTTATATGT

TATAATAATGCTACCTTAGATGGCATATGCTGCTCAATTTTTCTTTCCTTTTTCCA

CTCTAGTAGTGGTAGTCCTCTGGCTTCTGGTTATTTTTGTCTCTAGAGTTATGTT

ATGTTATTATAACAGATGTGAGAAAGGGCCCAAAACCAGGTTTTAGCCTGTGTC

TGCTCTAATGAGTTAATGTGATTACTCTGGATGATCTTCAGAGTAGAATACAATA

AGCAATTGTTGTTTATTACAAGTTTCGAATTTGTAACTGTGAAATCTACTGTGCTT

CCTAATTTAGCACCTTTAACAATCTATGATCTAGGCTATTGCTTCCAACTTTTAAA

GTTTAAGGGAAACATTGATTGTCCCAAGGAAATGTGGTCAGAGGAAATAACAG

AGGTAATACTCAAAATAAGAAATACTCAAAATAAGTTAATGTGAAAAATAATAGTA

TAGAAGCAGCTCAAGAAGGAAATGATGAGTTGCAAGGTCAGATTGAAGAACTGT

TCCAGAAGGCAGCAAGAAACAATAATGTAAAATTTTTAAAATTAATAATAATAACA

AAAAACTAAAGACATGGAGGATAGAAATAGATATATATGGTGTGTTGTATGAGA

TGAGTCAGTTAACTTATGAGCAAGCAACCCAACCATATCTAAACATATACCTGAG

TATGCTTTAACTCATGTACTTACAAAATTGCATTTCTTAGTGAAAAAAAAATCAAG

AAAGAAACAATTCCTAAGGTTAGTGATGAAAGTATAAAGTCAAGAGATAAACTA

GTGATTTTCAAAAAAAAAAAAAGTAACTTTAGAGTGGCAAATCAAATGTCACTC

TGTTCATACAAGATTCATGAAACTGTTTCAGAGAGGGTTTTGTTCCTTGCAAAAG

TTGTTCACCCACAAGAGACAGATTTTGTAAAATTTAAAATGTAGCTAATTTTCCAT
GTGATTAGGCAGTGTAATTTAAAGAAACTCAAGATTTTCTACAAATAATTTTTTGA
GGCAAGAAGATGCTAAAGTCACTTGTACTGCCACTAAACTTTACTAAAAGTTGG
TTTGAACACTTTAATTGCTTTTGAATAGATGATTGAAGCTGTGAAGAGGTGGCAG
CAGCTGACATGTTGCCATGGTAGCATTCCTCCCCAGCTGAGGAGGCTATACA
GAAGAAAGGATATAGGTCAGAAAGTGTTTATACTGACAAAACAACCACATATTAA
AATCAAATGCTTTTGTAAACATCATAAGCCAAATGAGAGTTGTGAAATCTAGTTT
CAGGATATTAAAGACAGACTGATTTATCTTTAGGATGATGTATCAAGCTGTACTG
TGAGAATGGAGCATTCAAGGTCAAGAATACTCTCCTTCCTGCATATTGGCAATT
CAATTTTAAAAGATTGAAACACGATGAAAGTGTTTCTATCAGTTTCTTCTGTGTTC
CTTGCAATAAGGATATATTTAATTGAAAAATTCTTCTTGAAGGTAGTTCATCTCTG
AATAAACTACTCCAGGGTAAGGAAGATCAACTCTATGATCAATA

SEQ ID NO. 24 (cDNA sequence of CLT encoding CLT Antigen 2)

GAGGCCTCACAGCCATGTGGAACTCTCTGGAGGCCAGAAGTCCAAAATCAAGA
TGTTGCAGCACTGGTCCCTGGGAGGCTGTGCAGGAGAATCTGCTCTGGGCGTC
TCTCCTGGCTCCTGGTGGTGCCACAATCTTCCCTGATCCTTGGCTTTTGAAGGC
ATCCCCCAGCTCTCTGCCTCATCTTCACAGGACTTCTCCCTGTGCCTGTCTGTG
CCCAAATTTCCCCTTCTATAAGGACACAGTCCTACTGCATCAGGGCCCACGCTA
ATCACTTCTTCTTCACTAATTACATCGGCAAACCCCCTATTTCCAAGTTAAGTCA
TGTTCTGAGGTTAGGATTTCAATACCTGAATTTTAGGGGACATAGTTCAGCCCA
AAGGACCTGGGGGGACAGAAACACCCAGGAAAGTCCACAGGTCAGCGCCTGG
ACCCGCCTGGCCGTGGGACATTGCTCGGCCGAGTTCATCCTCATAAACGCTGA
CGTGTGCTAGAAGATACAATATTAGTTTCATAAACAACAGTTTCTTTTTTTGCAAA
CCATACCCACAGGTCCTATTGCCACTTTTCAGTGCTGCAGCAGCTAGTCAGATG
ACAACTGGGTGAGAGCTTGGTCTCGAGTGTTTCCTGTGAGGCCAGAATATTGCT
CTGATTCCTCAGGGGTGACTCAGCCCTCCGGTGGGAAATATGTGACCTCATTT
GCATTTCCTAATTGAATTGCTATCAGCTTTCCTGCAAGACTGCACCAAAAAACAG
TTCTTCCAGATTCCAGCACTGCCTGCATTGCCCACAGAAGGCTGCTTTTAAAT
AGTTGCTAAGAGAGGGCGACAAAGAAGGAGAAAGTTGCCAAATTTTTATGGT
TCATCTAACTGTAAAGCTGTCAGTTTTACCCAGCTCCCCAGAGTCGCTAAAATAA
TGGACAGAGGAGGCGGCACTTGAAGAAACCTCGACATAAGAGAAATGAAATTT
ACAGAACCAGGAAATTAGGGGTCCCCATGCACCTGCCCTGGCAGAATCTGCTT
CCAGGCTCCTCTCAATAAACACTGCCCTGGGACTCCCAGCGCTCTGTAAACTG

ATGCTTTGAAGGAGGCCATTTGTTTCTCCAGAACCTTCTGCCAGCAGAACTCTT

GCTTACCACGTGCCCCCATCAGAACTCCCACCTTCTCTAAACACAGTTAGGTAG

CCCTCTGCATGCATTTGTTTAAAAAAATTAAATTGTGAGTAATAGACTATAAGTA

CAACAGCGTACAGAACACCAGCTAAAAAAATGATGGCAGAATGAGCACCTGTCT

TGCCACAAGACTCCCTCCCACCCTCTGGTGTCCCCAGCATGGCTTCCTGCTG

CCCCCTGAAAGGAACCCTCAACCGACTGTAACTCTGCACCTCAGTGCCACCCG

AATTTGGACTTCATGGGAGTGCGGTCACTCGGCTGACACGTGTTCCTGCTGCC

AGGCACCTGCCTGCTCTCCTTCTTTGGTCTGTTTGTGAGATTCGCTGGCTTTGT

TGCTTCTAGCTGCAGTTTGTTCACTTTTACTGCCGTATAATATTCCACTTAGGA

GCCCACTGCAATTTATTGACTCATTGCAATGGTGAGGGCATGTGTGTTGTTTCC

AGCTTTTGGTGATTATGCGTAAGATTTCTCTTAACGTCCCGTACAGAGTTCTTGG

TATTACCTGCCTATACTGCTATGGGCATACACCAAAAACGAGGCATATGTATATT

TGACTTTAATAGTAATTCTGAATTGCTGTCTAAAGTGTTTGTACCATTGTACACTC

CCATCAAGAGTATACGAGTGCTGCAAATGTCCTTTCCCAGACTCTGCCTTATTG

TCTCATTTTTAAGATATATGTTTTGATGATCAGAAGTTCTTTTTTTTTTTTTTTTTT

TTGACAGGCTTTTGCTCTGTTGCCCAGGCTGGAGTGCAGTGGCATGATCTCAG

CTCACTGCAACCTCTACCTCTCGGGCTCAAGTGATTCTCCTGTTTGAGACTCCC

TAGTAGCTGGGATTACAGGTGTGCACCACCATGCCTAGCTAATTTTTGTATTTTT

AGTGGAGACAGGGTTTCACCATGTTAGCCAGGCTGGTCTCGAACTCCTGACCT

TGGGTGATCTGCCCACCTTGGCCTCCCAAAGTGCTGGGATTACAGGCGTGAGC

CACCGCACCTGGCAGAAGTTCTTATTTTTAATAAAGTCCAGTGATCATTCTTTCC

CGTGTTTCACGCTTCACGTGCCATGTGCGAGGAAGCTTTCCACACCCGGGGGT

CAGTTCACTCCCAGCTGCATTGCTTTGCCTTTCCTGTTTAGGTTTTCAGTCCACC

TCAAATGGATTTTGGTTGTGGTGTGAGGTAGGGGTCATTTTACTTTTTTCTTAAA

GATGCCCCATTGGCCCAGCATCATTTATTGAAAGACAGTCTTTCCTCTGTTGTT

TCTGTAGAAATGGGCAAACTTTCCCCAAATGGCCAGATGGTAAATATTTTAGG

CATCTCAGGTTAAGGGGCAAAATTGAGGACATCGTATAGGTACTTACATAACCC

TTTAAAATGGAACCATTTAAAAATGTAAAACCAGTCTTAGCTTGCTGGCCATAA

AAGGCCTGTGGGCTACAGCATGCTGGCACCTGCTCTACAGCACTGCTCTTATA

ATAAAAAATCAAGCGTCTGCACCCTGGGCATTTGACAAGGCTCATCAGATTGTA

AAGATGAACTGGGTGGATTTTGTAGTATGAGAGTTATACGTCCATGAAGCTGAT

TTTTAAACACATTACATGTCTTTTTCTGGGCTCATTATTTTTGTTCCAAGTGTCTA

TCTAATCATCTTTGCTTCAAAATGACACCGTTTTAATGGCTGTAGTTCAGTGGTT

CTCAAAAGGGGGCAGTTTTGCCCCCCAGGGAACATTTGGCAGTGGCTGGAAAG

AGCTTTGGTTGCATATGGAGGTTATGGGGCTTTACCTGGCGTTAGTGGGTAGA
GGCCAGGAGTCTGGCTTACTATCCTGTAGCACACAGGACAGCCCCAACAACTT
GTCTGGAAGGATGTGTCCTCCCATCTTTTTCTTGTTCTAGGCTACCTTCGCCCTT
TCCATTTCCATTAAAAAAT

SEQ ID NO. 25 (cDNA sequence of CLT encoding CLT Antigen 3)

GTGTGCCCTTTGCAGCAAAGAGCGTGGTTCCTCCTCTTCCTGGGTGTAAACTG
CGATTCAAAAAGCCAGGTGGGAAGCCCTGTAGTAGGGACTCTGGCTTTGTCCC
TGTTTCCCCCTTTTCTTCCTCTTCACCCACTAAAACCCTGTTTTACTCACGGTTC
AAATTGTTTGGCAGCCTGAATTTTCATGGCCATGGGACAAAGAACCCCGTCTTT
AGCTGAATTAAGGAAAAGTTCTGCAACATTTTTGACGTGCAACTTGGGGGCTCA
AGCGGAGAAGCGAAGCAGAGCCCCTGGAAAACTCACATATGTGTCAACAATAG
TCCTTGATGCCCCTGTAACAAACTTGAGCAAGGCCTGGTGATGAAGAGATACA
AGATTGTCACCCAAGGATTTGATTATCCTCTGTTGAAAGTTAATGCACACTCAA
CCGAATATGGCAATTGGTACTCTCAATTCTATATTCTCAACAATTACAGTGAAGA
TACAAGTAGAATACAATCACTGCCACATTTTTTGAAATTCTGTGTAAACTGTAATA
TTCTGTCAACATTAATGACATTTGAAGTGTCCTGTCAAACAATTGCAGCTACTT
TATGTATAAACATATTAAATAGGCTCATCCAACTTGCATTCTTTATTAAGCTTAT
TTTCATATTTTTTCCTATGGATGAACTTAAAAATAATTTTGTTTCTTAATTAAGATT
CTATGCATGAAGATGCTGAATAATTTAAGACAATTGTTCATTCAAATAGTTGCTA
ATTACACCCTCCTGGCATAGTTATTGTATTATTACTACATTTAGGAATAATATGCT
GTACTACTTGGACTTGAAAATGTTTCTGACATTTTAATGAACACACTACTTAGTTA
TATTTTACAAGGGTTTTCAGTGAACCACAGAGGATTAAAAAATGTCATTCAAGGG
TTGTAGATAATTAAACTGACTGAATATAAGAAGCCTCATATGGAAGTGAAAATTA
TGTATGAATTTTGTTGAGCTGGAAATGTGTTTTACTAAATGACTTCAGATTTGTTA
CTTTTAAATACAAGATGAAGGGAATCAAGGGGATACTTTCTTCTCACCTCAATTT
GTTCCATTTGCATAAGCTATTTTATCTTTCAATATCCCTTTTGATATTTCATTTTGG
CCCTTGAATACACTAAAGATTATTTAAAATAAATAAATGTTGACTTTGAAATACTG
CTATATATATTCATTGCAATACATCAGGTGGGAAATTGGTGCAATTCAGTCACAC
ACACAACCTCATAGATCAGATTTCCAGTTAAGTTTTAATAGAAAGAGATTTTAGA
TAGACATGACTTTCAATAAATGGAAGAATTTTTATTAGTTTCAAAATAATGATTT
TACTTGCTTATCAACACAGTTGACCTTTCCCCACAGTACTATGATCTTCTTAGTA
CCTCTGCTATAATTATATTAATGTCTTCCTATATCTAGAAATTTGATTATTATTCTA
TTTTTATGGATCATTAAATTTTTCTATTCAAGAATGATCCCTATAGTCAACTTCCT

GAGGGATTTCTGCTTGCTATTTTCTGTTTCAATTGTGTGCATTTCATATTTACTAT
TAAAGATTTTAAATAATGACACATTGTTTAAGCGGTTGTAAGTCTGATTGTTATAA
AGCTCTCTGGAGAATTCTGTAAGCTTTCACATCTGATGGAGAACTTCAATGAGA
ACATCTTTAATGTGGAATTCTTGGACAAGAAACTACAGATTATCCATGGTTCAGA
AGATATGATGAATTAGTAACTATTTTTCCGTACATAAAAGTCAATCTTCCTAACC
AGTTTGTTGTTTTAGCTAAATGAATCGGTCAAATATTTTAGTTATATTAGCAATAT
TTTGTCTCTAAAATATCTTGACATAAAC

SEQ ID NO 26 (cDNA sequence of CLT encoding CLT Antigen 4)

TTTGCATTTCTTTCATTTTCCACCATGATTGTGAGGCCTCCCCAGCCATGTGGAA
CTAAATGCCTAAGAATTACATCAGGAGTAAAAGTGAAGAAAAGACTGTAAACT
GCGTGCTAACTGCTTTGAAGAATGAGAAGTCATGCCAAGCAAAATAGTAGATAA
TTGCAAGAAGGGGACTAATGGTCCATGGATGGAGCTGGAGGCCATTATCTTTA
GCAAACTAACAGAGGAACAGAAACCAAGTGAGAACCACATGTTCTCACTTATA
AGTGGGAGCTAAATGATGAGAACACATGGACACATAGAGGGAAACAACAGACA
CTGG

SEQ ID NO 27 (cDNA sequence of CLT encoding CLT Antigen 5)

GGTAGTCACATGTTTAGCTTTTAAAGAAATTGCCAAACTGTCAGAGTGGCTGTC
CCATTTTACTTTCCACCAGCAATGTATGAGTGATTTGTCTTTTCTTCATCCTTAGC
AGCATTTGGGGGCTATTTTTTATTTCAGCCGTTCTTACAAGTCCACGCTGCTTTT
ATGAGCTGTAACACTCACTGCGAAGATCTGCAGCTTCACTCCTGAGCCCAGCG
AGACCATGAGCCCACCGGGAGGAAGGAACAACTCCAGACATGCTGCCTTAAGA
GCTGTAACACTCACTGCGAAGGTCTGCAGCTTCACTCCTGAGCCAGTGAGACC
ACGAACCCGCCACAAGGAAGAAACTCTGAACACATCATCTGAACATCAGAAGG
AACAAACTCCAGACGCGCCACCTTAACAGCTGTAACACTCACCGCGAGGGTCC
GCGGCTTCATTCTTGAAGTCAGTGAGACCAAGAACCCACCAATTCCGGACACA
CCAGGACCTTCCGCTGCTCGCTCCTCCTCGTCCCTTCTACCCGCTGGGGTCAG
CCTCATCGACCCCGTCTCAACTCTGACACAGTCTCTCTCTCTCTCTCTCTCTA
TATATATATATATATATTTTTTTTTTAGATGGAGTCTCGCTTTGCTCCCCAGGCTG
GAGTGCAGTGGTGCAATCTCTGCTCACTGCAAGCTCCACCTCCTGGTTTCACG
CCATTCTCCTGCCTCAGCGTCCCAAGTAGCTGGGACTACAGGCGCCCGCCACC
ACACCCTGCTAA

SEQ ID NO 28 (cDNA sequence of CLT encoding CLT Antigen 6)

GGGTCCCTGGCTCGGCTCTACCCCCATGGATCTAGGTGAGGACAGGCGCTCC
TGCTTTCCCGCCCAAATGTTGTATTTTCCAAGCCTACCCTTGCCGGCCACGCCC
CCAACCTGGGCCTATAAAAACCGCCCCCGCAGGCCCTAGCGGGCAGACACA
CTGAAGTCGCTAGACATTTGAGGAACACATCCGGGGAAGAAGACACAGGTGGC
TGGTCATGGAGAGCCCGCTGGGGGAAGAGCACACAGACAGGCACCGGCAGGC
CATTGACCAGCGGGACAAGGTGGAGTTTGGCTGGGGCAGTGGGAGGAGAGCT
GGGGCCGCCGAGCTGCCCTACTCCAGGGGAAAACCACCTCCCTTCTCGCTCC
CCCATCACCGGAGAGCTACTTCCACTCAATACAACTTTGCACTCATTCTCCAAG
CCACGTGTGACCAATTTTCCGGTACACCAAGGCGAGAAATCCGGGGATACAG
AAAGCCCTCTGTCCTTGCGATAAGGTAGAGGGTCCAATTGAGCTAACACAAGCT
GCCTATAGACGGCAAACTAAGAGAGCACCCAGTAACACACGCCTGCTGGGGCT
TCAGGAGCACACGTCCACTGGGGCTTCGGGAGCTGTAAACAGTCAGCCCTA
GACACTGTCGTGGGATCGGAGCCCCACAGCCTGCCTGTCTGTATGCTCCTCTA
GAGGTCTGAGCAGCGGGGCGCTGAAGAAACGAGCCACACTCCCATCACACGC
CCTGAGAGGAGGACAAGGGAACCCGTACCGTTTCACTTGTGATAGAGATAAAG
TTATTATTGTTGTAATTTTAACTTATAGAACTATAATATAATGGTACAATGATATAT
ATTGACAATATTCCTCTTTCACCCACATTTTGTATCTGTGTTCAAGATTAAATGAA
AAAGGATATTCTCAAAAAGCTAGCAAAACCAAAAGCAAGACGTTATGCCAAATG
TAAACATATTTCTGTTTAGCAAATAGCAGGAGTGTATAAAACATTTCTCTTTTCAC
ATAACAGAATGTTCTATGCTTACTGTATTAGTTAACAAATTCAAGTCTGTTTATTT
TGTTTGAAATTCCACTTCATCCATAAATTACAGCATTACACAATAACACCAGAAG
GACAATATCACCATTGTTTGCTTTTACAGTCATCTCAGCCCACAAATGCTTCCC
ATTGTCAGCTTGCTATCATCAAGCTATTGTTGTTTTCCTCTTTCTGGGGTCTTGT
ATTAATATCATTCAAATTATTTAGACACTCAACAGTGTTTTTGCTATCAGTGCAAA
CCTCTAAAGAGCTGGAGCCCCAGCGTGATGACCAAATAACCCTTGACTATTTAT
CTTGCCGTAAGTCATTATTTCCTGAGGCCCTGGAGAAGCTGTGTTGCCATGTGT
GCACTGCAGGGACGGGGAGCTTCTCCTGCCGGAGCTGGTTTGTTCCACTGGA
CAATGAGCCCTTTTCTGCTTGGCTCTCTGTATGGGCACATACACAATGAGGCGG
TTTAGGGAAGAGGGGTGATGTGGGTCATTGATAACAACAATCCCCGAACTTCTT
AAAGAATTGTTGAGCCCCCTAAAAATATGTTGTCTTTATGAATTATCCTTAACCC
TTTTTAATTGCATAAAAACTTCAGGCACTTGAAAAAAATTAAAAAACGAAAGTAA
GTCTGTCTCTAGTATCCCTCTTCTCCTTTGTAGAATTACATCCTTTATTCACTCTG
CCACTATTTATTATGTGCCTCCTGTGTGTAAGACATACTGTTAGTCATTGGAAAT

ACAGAAATGAATGGGACAGACACACTTCTTGCCCTCATGGAACTTAGGGCCTAA
TGGGAGATACAATGTTAAGGTTGTTTCTAGTGTTTTTTTTTTTTTTTAAGAATATG
TTCTACATGTATACATGTAATATGTATTCTCTCCCATTTTTAAAAAACATAAATGG
TGGTTTACTATGTGTACGCTTTTTGGTTTTGCATTATTCTTTTAACAGTATGTAGA
GGATGCATTTTCTACTGTATAGTGTTCTGTCCCCCTTAAATAGCACTCTGATTTT
ATTTTGGGGGGGAATCACGACTTTCTAATTTTGCATACTCCTTGTGGGATTGTAA
ATCAGGTCCCCTGTCCTCAAGTAGCCAATGGTTAGGTATGCAACCCAGCTTATC
TCTCTTTAGACTCAATCTCAAGCAGATCCAGAGTTACTCAGGATCAGAACAATAT
TTGAAAGGCATTACCAGAATCCAGACAAGATGATGGAGCAATACCTGATGCCCA
GTGGTCTAGGGTAGCCGATTCCTGTTCTGCTCTCCAGGCTCCTGTCCATTCTGT
GGATCAACTCATATTGCTCCAATTCATTTTGTTTTGCTTGCATAAGCCAGAATTA
ACTTCTGTTGCTTGTAAACAAAGAGAGTTAACCAAAGACATACAGTATATCAGAG
TATAGAGACCTACTTTACTCTCGGTCATTGCCTGCATGAGTCTTCTTTATATGGA
TGTATCACAGTTTATTTAACCACTCCCTTGTAAGTGGACATTTAAGTCTAGTGTT
CTGTAAATAAAAGGTCAGAATATACGTCTGTATACAGTATATATTCTTGCATATC
CACCTTTGGACAGATGTGTGAATGTGTTTTGTAGAAATACATTTGTAGAAATGCA
ACTGCTGGGTCAAAGAATTAGTAGATTTTTAATAACATCAAACAGCGTTGAAGG
CCCCCATATAAGAATAACAACTACTGACTGAAGACATAACTAATTAAAAAAATTA
ATTACAGCTTATTTGTAATAACTTCTTATTGTCACTGAGTGAAAAGGTAATTCTCG
TTGAATTTACGAAAGTGACTAATAGGAAATTTAGAAAACTCAGAGAATATATAA
AAACACAAGAAAGCCAGCCACCAAGTCGCTTATAATTCTCTCACCAACAGTG
GCAGAATTACATTTAGTCATCATCATTATTCTTACATCCAGTTTTATAGTTATTTTT
GAAGAGGATTATTATCAACTATCAACTCTGTCATAGCTGGAAGTAGAGGCCACT
AAAACAGATTTCTTAAACTCCAAGTACTGTATATGGATTTTCTAC

SEQ ID NO 29 (cDNA sequence of CLT encoding CLT Antigen 7)

AGAGTACTTCTCAGAAGTGAATGTCAACTTCCTGATGTTTATACTCAAGGAGAAT
GAGGTTTGGAGATCTGAGTAGTTTACTGAAGGCACTCACATTGAAAGATTTTAT
GAAGCTGAAATTCAAATCTAGATGTGCCCCATCCAAAGACCACCCTCTTTATAC
AGCAACATGCTGCTTCCCCTGGATCAAGAAACACAAGGAGCCTTCATCTACCAC
ATAAGAATTGCAGCAAACCCTGCAGCTATCCTGAAGCTGCCATGCTGAAAAGGC
CAATTGGGAGACCACATAGAGACCGAGAGAGACTTCCAAGGACTCCAGCCAAT
CCTGGGCCCCAGCAGTTTGAATCTCCAGCAATGCCACCATACAGGAGAGGGA
GCAAATACTCAGAAGATTCAAGTGCCAGCTGCATGGGTTGATACCTACATAAAA

GGCATTGGCATTATTCACAAGAGCCAAGATATGGAAATAACCTGTGTCCATTGA
CAGACGAATAGATGAGGGAAACGTGGCATATACACAGTGGAATATTATTCGG
AATTAAAAAAGAAGGAAATCCTGAATCCTGCTATTTCTGACAACATGAGACTGC
AGGACGTTATGAAAATGGCCACATCATGCTCTTCTAGAACTTTCTCATCCCTCA
GTCAAGAGGTGGAGCCTATTTACCCTTGACCTTGAAATTGATCAGGACTTTGTC
ACTGCCTTGCTCTTAACATCACCATGCTGGAGAAATAGCATGGCAAGGCCAGCT
GGTGATAGAGAAAGATGCCTAAGGAGCTCCAGCTGCTATTTTCCCTGGATTTTT
GAGTCTTCCCAGTCCAGCCAGACATATGACTGGGCAAAACCCTCAGGTAACTA
CAGCCTTATCCACTGTCTGACTATCACAAATGAGAGACCTTGGGTGAGGACCA
CCTACCTGAGCCCAGTCATCCTGCAGAGATGCGAGAAAATAAGAATGTCATTA
TTGTTATTTTAAAATCACTGAGTTTGGGTTAATTTGTATGCAGCAATAGATAACTG
GGACACAAGATAGACTGTAAAAAATAAAGAGGGAATGGTTAAGCTAAAAACA
TTGTGATCAAATCAATGTTTAGTAAGTAAGCAAGAAAGATGGCTATCCAGGGAA
GAGAGATTGCACAACAAATGAAAATGAAACAAGCAAAGAAACAAAGCATGGAT
TATAACTTGTAAGAGAGAAACCATATTAAGGGGAGTTATTTGAATATTTTGTAT
GAAAATACAGAAATCAGATATAAGTGAGCTGACACTGACTTTGGAGGCAGGAG
GAAAACTGATGGGGAATTCTGGGAAAACAGAATGATACAAAGCAGAATCCTCAA
TGTCCCTACCCAAAACCTAATGAAATAAACAGAAATCAGCAACATTATTTTAAAA
ATTAATTCTCCAAGAACAATACAAAGTAAATGGAGCAAAAATTATTATTCATTTT
GAAAAGATAATCTAGAAACAGAGAGACAGAGGAATCTAGAAAATAGTACACAA
ATTCTTGTCTGAGAAGTGTCTGAGAAGACATCTATAGGCAAAACTGCATAAAATT
CTCTCCAGTTTGGAAGGCCATCCTGGGTAATTTTATAAAATGTCTCTTTCATAAT
AGAAGCCTCTTCCCAACATCATAGATGAAAAATTTTTCAACGAAGGGAAAATCTG
TTAGTGCTCATCAATCTTCCCCTACACCTCAAGGATGAGGGAGTAGAGGCATAG
ATTGAGCACAACTAATTATTTCCAGTAATAAACAACATCAAGATAAGACAAAGTC
TTGAGGCAATTTGGTTGTCCCTTGACAAAGGGAATAATGTGAAAGGAGGT

SEQ ID NO 30 (cDNA sequence of CLT encoding CLT Antigen 8)

TGGTCACCTGACATTCCTGTTAGGAAGAGGCCTCTCCTGCCCAGCTCATGTCTG
CAGCTACCCACTGTAACAAACCACATGGAAAAATGAGCTTGAACTGGACCCCT
ACCTTTTACCAGAGAAAAAAGTCAATTTGAGATGAAACAAGACTTTAATATAA
GACCTAAAACTATAAAATGTCATATAAGAAACCTAGAGAATACCCTTCTTGACG
TCAGCTTTGGCAAATAATTCATAACTAAGTCCTCAAAAGCAATTTCAACCAAAAG
AAAAATGGATAAGTTGGACCTGATTAAATTAAAGGGTTTCTGCACAATAAAAGAA

ACTATCAACAGGGTAAACAGACAGAGAGAAAATATTTGCAAACTATGCATCCAA
CAGAGATCTAATATCCACTATCTCTAAGAAACTTGAACAAATCAACAAGCAAAAG
ATGAACAGCCCTGTTAAGAAGTGGGCAAAAACATGAACAGGCTCTTCTCAGAA
GACATACATGTCGCCAACAAACATATGAAAACTACTCAAAATCACAAGTCAGG
AAAGAAATGCGAATGAAAGCCATAATGAAGTACCATCACACACCAGTCAGAATG
GCTACTATTAAAAAGTAAAAAAATAACAGATGTTGACAGGGTTGTGGAGAAAAG
GGAATGCGTGTTCATTATTGGTGGGAATGTAAACTATTTCAGTGCCTGTGGAAC
GAAACTTGGAGATATCTCAAAGAACTTACAAAGAACGACGATTCGACCCAGCA
ATTCCATTACTGGCTTTATATCCAAAGGAAAATAAATTATTCTACCAAAAAGACA
CATACATTCATATGTTCATCAAAACACTATTCACATTAATGAGGACATGGAATCA
ATGTAGATGTCCATCAGCTATGGACTGGATAAAGAAAATGTGGTACAAACATAA
CATGGAATACTATGCAGCCATAAAATGAAAGAAATTATGTACTTTGCATCAATAT
GGATCCAGCTGGAGACCATTATCTTAAGTGAATTAATGTGGGCACAGAAAGCCC
AATACTGCATGCTCTCACTTATAAGTGGGAACTAAACATTGGTTATGCATGGACA
TAAAGATGGGCACAATAGACACTGGGATCTACCATGTGGGGGAGGGCAGGATG
GAGAAAGGGCCTGAAGAACTGCCTATTGGATATTGTGTTCTCTACATTGGTGTT
GGGATTATTTGTACCCCATACATGAGTGTCACACAATATACTCATGCAACAAACT
CACACATGTACCCTCTGGACCTAAAATAAAAGTTGAAATGAAAAGAGTTGGTA
AATCAGTAGGTGGG

SEQ ID NO. 31 (cDNA sequence encoding CLT Antigen 1)

ATGAAACAGACAGAAATATTGATCCAGACAACTGGAATTTTCAAGTGTATTGCAATGATAA
TGACGACTGGAAAGGAAAGAGAGGAAGGTAACAAAGAAAAGCCAAGAAAACTATTTGGAG
TGAGAGACTATGGTAATATAAAAGGCTATAATGTGGTAAAATCGTGGGGACTTAATTCCAT
CATATGGAGGTTACTGAATGTATCAGAGACAGCCTGCAGTTCATCACCACACAAATTAGGA
GCAACGTAA

SEQ ID NO. 32 (cDNA sequence encoding CLT Antigen 2)

ATGTGGAACTCTCTGGAGGCCAGAAGTCCAAAATCAAGATGTTGCAGCACTGGTCCCTGG
GAGGCTGTGCAGGAGAATCTGCTCTGGGCGTCTCTCCTGGCTCCTGGTGGTGCCACAATC
TTCCCTGATCCTTGGCTTTTGAAGGCATCCCCCAGCTCTCTGCCTCATCTTCACAGGACTTC
TCCCTGTGCCTGTCTGTGCCCAAATTTCCCCTTCTATAAGGACACAGTCCTACTGCATCAG
GGCCCACGCTAA

SEQ ID NO. 33 (cDNA sequence encoding CLT Antigen 3)

ATGGCCATGGGACAAAGAACCCCGTCTTTAGCTGAATTAAGGAAAAGTTCTGCAACATTTT
TGACGTGCAACTTGGGGGCTCAAGCGGAGAAGCGAAGCAGAGCCCCTGGAAAACTCACAT
ATGTGTCAACAATAGTCCTTGATGCCCCTGTAACAAAACTTGAGCAAGGCCTGGTGATGAA
GAGATACAAGATTGTCACCCAAGGATTTGATTATACCTCTGTTGAAAGTTAA

SEQ ID NO. 34 (cDNA sequence encoding CLT Antigen 4)

ATGATTGTGAGGCCTCCCCAGCCATGTGGAACTAAATGCCTAAGAATTACATCA
GGAGTAAAAGTGAAGAAAAGACTGTAAACTGCGTGCTAACTGCTTTGAAGAAT
GAGAAGTCATGCCAAGCAAAATAG

SEQ ID NO 35 (cDNA sequence encoding CLT Antigen 5)

ATGAGCCCACCGGGAGGAAGGAACAACTCCAGACATGCTGCCTTAAGAGCTGT
AACACTCACTGCGAAGGTCTGCAGCTTCACTCCTGAGCCAGTGAGACCACGAA
CCCGCCACAAGGAAGAAACTCTGAACACATCATCTGAACATCAGAAGGAACAAA
CTCCAGACGCGCCACCTTAA

SEQ ID NO 36 (cDNA sequence encoding CLT Antigen 6)

ATGTGTGCACTGCAGGGACGGGGAGCTTCTCCTGCCGGAGCTGGTTTGTTCCA
CTGGACAATGAGCCCTTTTCTGCTTGGCTCTCTGTATGGGCACATACACAATGA
GGCGGTTTAG

SEQ ID NO 37 (cDNA sequence encoding CLT Antigen 7)

ATGAGGTTTGGAGATCTGAGTAGTTTACTGAAGGCACTCACATTGAAAGATTTTA
TGAAGCTGAAATTCAAATCTAGATGTGCCCCATCCAAAGACCACCCTCTTTATAC
AGCAACATGCTGCTTCCCCTGGATCAAGAAACACAAGGAGCCTTCATCTACCAC
ATAA

SEQ ID NO 38 (cDNA sequence encoding CLT Antigen 8)

ATGAACAGCCCTGTTAAGAAGTGGGCAAAAAACATGAACAGGCTCTTCTCAGAA
GACATACATGTCGCCAACAAACATATGAAAAACTACTCAAAATCACAAGTCAGG
AAAGAAATGCGAATGAAAGCCATAATGAAGTACCATCACACACCAGTCAGAATG
GCTACTATTAAAAAGTAA

SEQ ID NO 39 (peptide sequence derived from CLT Antigen 6)
SLYGHIHNEA

**Claims**

1. An isolated polypeptide comprising a sequence selected from:

   (a) the sequence of SEQ ID NO. 2; and
   (b) an immunogenic variant of the sequence of (a) which is at least 80% identical to SEQ ID NO. 2; and
   (c) an immunogenic fragment of the sequence of (a) comprising at least 9 contiguous amino acids of SEQ ID NO. 2.

2. The isolated polypeptide according to claim 1 comprising or consisting of the sequence selected from SEQ ID NOs. 11 to 14, or consisting of the sequence of SEQ ID NO: 2.

3. A fusion protein comprising the isolated polypeptide according to claim 1 or claim 2 fused to a second or further polypeptide selected from

   (i) one or more other polypeptides selected from

      (x) polypeptides having the sequences of SEQ ID NOs. 1 and 3-8; immunogenic variants of the polypeptides of (x) which are at least 80% identical thereto; immunogenic fragments of the polypeptides of (x) which comprise at least 9 contiguous amino acids of the sequence of the said polypeptide; and
      (y) polypeptides having the sequences of SEQ ID NOs. 9, 10, 15-22 and 39;

   (ii) other polypeptides which are melanoma associated antigens; and
   (iii) polypeptide sequences which are capable of enhancing an immune response.

4. An isolated nucleic acid encoding the polypeptide or fusion protein according to any one of claims 1 to 3 or vector comprising said nucleic acid.

5. An immunogenic pharmaceutical composition or vaccine composition comprising a polypeptide, nucleic acid, fusion protein or vector according to any one of claims 1 to 4 together with a pharmaceutically acceptable carrier; optionally wherein the composition comprises one or more immunostimulants.

6. A polypeptide, nucleic acid, fusion protein, vector or composition according to any one of claims 1 to 5 for use in medicine.

7. A polypeptide, nucleic acid, fusion protein, vector or composition according to any one of claims 1 to 5 for use in treating or preventing cancer in a human, wherein the cells of the cancer express a sequence selected from SEQ ID NO. 2, an immunogenic variant of SEQ ID NO. 2 which is at least 80% identical to SEQ ID NO. 2, and an immunogenic fragment of SEQ ID NO. 2 comprising at least 9 contiguous amino acids of SEQ ID NO. 2.

8. A polypeptide, nucleic acid, fusion protein, vector or composition according to any one of claims 1 to 5 for use in the *ex vivo* stimulation and/or amplification of T-cells derived from a human suffering from cancer, for subsequent reintroduction of said stimulated and/or amplified T cells into the said human for the treatment of the said cancer in the said human.

9. An isolated antigen-binding polypeptide which is immunospecific for a polypeptide having the sequence of SEQ ID NO. 2 or an immunogenic fragment of SEQ ID NO. 2 comprising at least 9 contiguous amino acids of SEQ ID NO. 2.

10. An isolated antigen-binding polypeptide which is immunospecific for an HLA-bound polypeptide that is or is part of the polypeptide having a sequence selected from the sequence of SEQ ID NO. 2, an immunogenic fragment of SEQ ID NO. 2 comprising at least 9 contiguous amino acids of SEQ ID NO. 2, and an immunogenic variant of SEQ ID NO. 2 which is at least 80% identical to SEQ ID NO. 2, optionally wherein said antigen-binding polypeptide is a T-cell receptor or a fragment thereof.

11. A cytotoxic cell expressing the antigen-binding polypeptide of claim 10 on its surface, for example, wherein the cytotoxic cell is a T-cell.

12. A cytotoxic cell according to claim 11 for use in treating or preventing cancer in a human, wherein the cells of the cancer express a sequence selected from SEQ ID NO. 2, an immunogenic fragment of SEQ ID NO. 2 comprising at least 9 contiguous amino acids of SEQ ID NO. 2, and an immunogenic variant of SEQ ID NO. 2 which is at least 80% identical to SEQ ID NO. 2.

13. A pharmaceutical composition comprising the antigen-binding polypeptide according to claim 9 or the cell according to claim 11 together with a pharmaceutically acceptable carrier.

14. A composition comprising said T-cell population according to claim 13 for use in treating or preventing cancer in a human, wherein the cells of the cancer express a sequence selected from SEQ ID NO. 2 and an immunogenic fragment of SEQ ID NO. 2 comprising at least 9 contiguous amino acids of SEQ ID NO. 2.

15. An antigen-binding polypeptide according to claim 10 or a composition comprising said antigen-binding polypeptide according to claim 13 for use in treating or preventing cancer in a human, wherein the cells of the cancer express a sequence selected from SEQ ID NO. 2, an immunogenic fragment of SEQ ID NO. 2 comprising at least 9 contiguous amino acids of SEQ ID NO. 2, and an immunogenic variant of SEQ ID NO. 2 which is at least 80% identical to SEQ ID NO. 2.

**Patentansprüche**

1. Isoliertes Polypeptid, umfassend eine Sequenz, ausgewählt aus:

   (a) der Sequenz von SEQ ID NO. 2; und
   (b) einer immunogenen Variante der Sequenz von (a), die zu mindestens 80 % mit SEQ ID NO. 2 identisch ist; und
   (c) einem immunogenen Fragment der Sequenz von (a), das mindestens 9 aufeinanderfolgende Aminosäuren von SEQ ID NO. 2 umfasst.

2. Isoliertes Polypeptid nach Anspruch 1, umfassend die Sequenz, ausgewählt aus SEQ ID NO. 11 bis 14, oder bestehend daraus oder bestehend aus der Sequenz von SEQ ID NO: 2.

3. Fusionsprotein, umfassend das isolierte Polypeptid nach Anspruch 1 oder Anspruch 2, das mit einem zweiten oder weiteren Polypeptid fusioniert ist, ausgewählt aus

   (i) einem oder mehreren anderen Polypeptiden, ausgewählt aus (x) Polypeptiden mit den Sequenzen von SEQ ID NO. 1 und 3 bis 8;

   immunogenen Varianten der Polypeptide von (x), die zu mindestens 80 % damit identisch sind;
   immunogenen Fragmenten der Polypeptide von (x), die mindestens 9 aufeinanderfolgende Aminosäuren der Sequenz des Polypeptids umfassen; und
   (y) Polypeptiden mit den Sequenzen von SEQ ID NO. 9, 10, 15 bis 22 und 39;

   (ii) anderen Polypeptiden, die Melanom-assoziierte Antigene sind; und
   (iii) Polypeptidsequenzen, die in der Lage sind, eine Immunantwort zu verstärken.

4. Isolierte Nukleinsäure, die das Polypeptid oder Fusionsprotein nach einem der Ansprüche 1 bis 3 codiert, oder ein Vektor, umfassend die Nukleinsäure.

5. Immunogene pharmazeutische Zusammensetzung oder Impfstoffzusammensetzung, umfassend ein Polypeptid, eine Nukleinsäure, ein Fusionsprotein oder einen Vektor nach einem der Ansprüche 1 bis 4 zusammen mit einem pharmazeutisch unbedenklichen Träger; wobei die Zusammensetzung gegebenenfalls ein oder mehrere Immunstimulanzien umfasst.

6. Polypeptid, Nukleinsäure, Fusionsprotein, Vektor oder Zusammensetzung nach einem der Ansprüche 1 bis 5 zur Verwendung in der Medizin.

7. Polypeptid, Nukleinsäure, Fusionsprotein, Vektor oder Zusammensetzung nach einem der Ansprüche 1 bis 5 zur Verwendung bei der Behandlung oder Vorbeugung einer Krebserkrankung bei einem Menschen, wobei die Zellen der Krebserkrankung eine Sequenz exprimieren, ausgewählt aus SEQ ID NO. 2, einer immunogenen Variante von SEQ ID NO. 2, die zu mindestens 80 % mit SEQ ID NO. 2 identisch ist, und einem immunogenen Fragment von SEQ ID NO. 2, das mindestens 9 aufeinanderfolgende Aminosäuren von SEQ ID NO. 2 umfasst.

8. Polypeptid, Nukleinsäure, Fusionsprotein, Vektor oder Zusammensetzung nach einem der Ansprüche 1 bis 5 zur Verwendung bei der Ex-vivo-Stimulation und/oder -Amplifikation von T-Zellen, die von einem an einer Krebserkrankung leidenden Menschen stammen, zur anschließenden Wiedereinführung der stimulierten und/oder amplifizierten T-Zellen in den Menschen zur Behandlung der Krebserkrankung bei diesem Menschen.

9. Isoliertes Antigen-bindendes Polypeptid, das für ein Polypeptid mit der Sequenz von SEQ ID NO. 2 oder einem immunogenen Fragment von SEQ ID NO. 2, das mindestens 9 aufeinanderfolgende Aminosäuren von SEQ ID NO. 2 umfasst, immunospezifisch ist.

10. Isoliertes, Antigen-bindendes Polypeptid, das für ein HLAgebundenes Polypeptid immunospezifisch ist, das das Polypeptid mit einer Sequenz ist, ausgewählt aus SEQ ID NO. 2, einem immunogenen Fragment von SEQ ID NO. 2, das mindestens 9 aufeinanderfolgende Aminosäuren von SEQ ID NO. 2 umfasst, und einer immunogenen Variante von SEQ ID NO. 2, die zu mindestens 80 % mit SEQ ID NO. 2 identisch ist, oder ein Teil davon ist, wobei das Antigen-bindende Polypeptid gegebenenfalls ein T-Zell-Rezeptor oder ein Fragment davon ist.

11. Zytotoxische Zelle, die das Antigen-bindende Polypeptid nach Anspruch 10 auf ihrer Oberfläche exprimiert, wobei die zytotoxische Zelle eine T-Zelle ist.

12. Zytotoxische Zelle nach Anspruch 11 zur Verwendung bei der Behandlung oder Vorbeugung einer Krebserkrankung bei einem Menschen, wobei die Zellen der Krebserkrankung eine Sequenz exprimieren, ausgewählt aus SEQ ID NO. 2, einem immunogenen Fragment von SEQ ID NO. 2, das mindestens 9 aufeinanderfolgende Aminosäuren von SEQ ID NO. 2 umfasst, und einer immunogenen Variante von SEQ ID NO. 2, die zu mindestens 80 % mit SEQ ID NO. 2 identisch ist.

13. Pharmazeutische Zusammensetzung, umfassend das Antigen-bindende Polypeptid nach Anspruch 9 oder die Zelle nach Anspruch 11 zusammen mit einem pharmazeutisch unbedenklichen Träger.

14. Zusammensetzung, umfassend die T-Zell-Population nach Anspruch 13, zur Verwendung bei der Behandlung oder Vorbeugung einer Krebserkrankung bei einem Menschen, wobei die Zellen der Krebserkrankung eine Sequenz exprimieren, ausgewählt aus SEQ ID NO. 2 und einem immunogenen Fragment von SEQ ID NO. 2, das mindestens 9 aufeinanderfolgende Aminosäuren von SEQ ID NO. 2 umfasst.

15. Antigen-bindendes Polypeptid nach Anspruch 10 oder Zusammensetzung, umfassend das Antigen-bindende Polypeptid nach Anspruch 13, zur Verwendung bei der Behandlung oder Vorbeugung einer Krebserkrankung bei einem Menschen, wobei die Zellen der Krebserkrankung eine Sequenz exprimieren, ausgewählt aus SEQ ID NO. 2, einem immunogenen Fragment von SEQ ID NO. 2, das mindestens 9 aufeinanderfolgende Aminosäuren von SEQ ID NO. 2 umfasst, und einer immunogenen Variante von SEQ ID NO. 2, die zu mindestens 80 % mit SEQ ID NO. 2 identisch ist.

**Revendications**

1. Polypeptide isolé comprenant une séquence choisie parmi :

   (a) la séquence SEQ ID NO. 2 ; et
   (b) une variante immunogène de la séquence de (a) qui est identique à au moins 80 % à SEQ ID NO. 2 ; et
   (c) un fragment immunogène de la séquence de (a) comprenant au moins 9 acides aminés contigus de SEQ ID NO. 2.

2. Polypeptide isolé selon la revendication 1, comprenant ou constitué de la séquence choisie parmi les SEQ ID NO. 11 à 14, ou constitué de la séquence de SEQ ID NO : 2.

3. Protéine de fusion comprenant le polypeptide isolé selon la revendication 1 ou la revendication 2 fusionné à un second polypeptide ou à un autre polypeptide choisi parmi

(i) un ou plusieurs autres polypeptides choisis parmi (x) des polypeptides ayant les séquences des SEQ ID NOs. 1 et 3 à 8 ;

des variantes immunogènes des polypeptides de (x) qui sont identiques à au moins 80 % à ceux-ci ;
des fragments immunogènes des polypeptides de (x) qui comprennent au moins 9 acides aminés contigus de la séquence dudit polypeptide ; et
(y) des polypeptides ayant les séquences des SEQ ID NOs. 9, 10, 15 à 22 et 39 ;

(ii) d'autres polypeptides qui sont des antigènes associés au mélanome ; et
(iii) des séquences polypeptidiques capables de renforcer une réponse immunitaire.

4. Acide nucléique isolé codant pour le polypeptide ou la protéine de fusion selon l'une quelconque des revendications 1 à 3 ou un vecteur comprenant ledit acide nucléique.

5. Composition pharmaceutique immunogène ou composition vaccinale comprenant un polypeptide, un acide nucléique, une protéine de fusion ou un vecteur selon l'une quelconque des revendications 1 à 4, avec qu'un support pharmaceutiquement acceptable ; éventuellement dans laquelle la composition comprend un ou plusieurs immunostimulants.

6. Polypeptide, acide nucléique, protéine de fusion, vecteur ou composition selon l'une quelconque des revendications 1 à 5 pour une utilisation en médecine.

7. Polypeptide, acide nucléique, protéine de fusion, vecteur ou composition selon l'une quelconque des revendications 1 à 5 pour une utilisation dans le traitement ou la prévention du cancer chez l'homme, dans lequel les cellules du cancer expriment une séquence choisie parmi SEQ ID NO. 2, une variante immunogène de SEQ ID NO. 2 qui est identique à au moins 80 % à SEQ ID NO. 2, et un fragment immunogène de SEQ ID NO. 2 comprenant au moins 9 acides aminés contigus de SEQ ID NO. 2.

8. Polypeptide, acide nucléique, protéine de fusion, vecteur ou composition selon l'une quelconque des revendications 1 à 5, pour une utilisation dans la stimulation et/ou l'amplification *ex vivo* de lymphocytes T provenant d'un humain souffrant de cancer, en vue de la réintroduction ultérieure de ces lymphocytes T stimulés et/ou amplifiés chez ledit humain pour le traitement dudit cancer chez ledit humain.

9. Polypeptide isolé de liaison à l'antigène qui présente une spécificité immunologique pour un polypeptide ayant la séquence de SEQ ID NO. 2 ou un fragment immunogène de SEQ ID NO. 2 comprenant au moins 9 acides aminés contigus de SEQ ID NO. 2.

10. Polypeptide isolé de liaison à l'antigène qui présente une spécificité immunologique pour un polypeptide lié à l'HLA, qui est ou fait partie d'un polypeptide ayant une séquence choisie parmi la séquence de SEQ ID NO. 2, un fragment immunogène de SEQ ID NO. 2 comprenant au moins 9 acides aminés contigus de SEQ ID NO. 2, et une variante immunogène de SEQ ID NO. 2 identique à au moins 80 % à SEQ ID NO. 2, dans lequel ledit polypeptide de liaison à l'antigène est éventuellement un récepteur de lymphocytes T ou un fragment de celui-ci.

11. Cellule cytotoxique exprimant à sa surface le polypeptide de liaison à l'antigène selon la revendication 10, par exemple, dans laquelle la cellule cytotoxique est un lymphocyte T.

12. Cellule cytotoxique selon la revendication 11, pour une utilisation dans le traitement ou la prévention du cancer chez l'homme, dans laquelle les cellules du cancer expriment une séquence choisie parmi SEQ ID NO. 2, un fragment immunogène de SEQ ID NO. 2 comprenant au moins 9 acides aminés contigus de SEQ ID NO. 2 et une variante immunogène de SEQ ID NO. 2 qui est identique à au moins 80 % à SEQ ID NO. 2.

13. Composition pharmaceutique comprenant le polypeptide de liaison à l'antigène selon la revendication 9 ou la cellule selon la revendication 11 avec un support pharmaceutiquement acceptable.

14. Composition comprenant ladite population de lymphocytes T selon la revendication 13 pour une utilisation dans le traitement ou la prévention du cancer chez l'homme, dans laquelle les cellules du cancer expriment une séquence choisie parmi SEQ ID NO. 2 et un fragment immunogène de SEQ ID NO. 2 comprenant au moins 9 acides aminés contigus de SEQ ID NO. 2.

**15.** Polypeptide de liaison à l'antigène selon la revendication 10 ou composition comprenant ledit polypeptide de liaison à l'antigène selon la revendication 13, pour une utilisation dans le traitement ou la prévention du cancer chez l'homme, dans lequel les cellules du cancer expriment une séquence choisie parmi SEQ ID NO. 2, un fragment immunogène de SEQ ID NO. 2 comprenant au moins 9 acides aminés contigus de SEQ ID NO. 2, et une variante immunogène de SEQ ID NO. 2 qui est identique à au moins 80 % à SEQ ID NO. 2.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Tumor sample

587.371

143.119

299.064

474.286

901.533

787.488

700.457

415.038

415.034

299.062

700.449

787.480

901.525

474.281

143.118

587.366

Synthetic peptide

Figure 16

Tumor sample

Synthetic peptide

Figure 17

Tumor sample

656.417

268.167

868.498

397.226

1015.568

173.093

213.088

609.308

434.753

722.393

510.311

510.309

722.389

609.305

397.222

213.087

434.749

173.093

656.412

868.492

1015.559

268.165

Synthetic peptide

Figure 18

Tumor sample

Synthetic peptide

Figure 19

Tumor sample

120.082

217.135
245.188

468.765
408.192
998.508
851.444
899.435
542.294
754.381
316.150

316.150
754.377
899.423
542.297
851.442
408.191
468.763
998.513

217.133

245.186

120.082

Synthetic peptide

Figure 20

Tumor sample

187.145

656.417

868.497

1015.568

434.753

284.141

510.311

771.448

284.139

771.438

510.308

1015.563

434.749

868.490

656.412

187.144

Synthetic peptide

## Figure 21

Tumor sample / Synthetic peptide mass spectra comparison

## Figure 22

### Tumor sample

129.103

270.194

515.297

143.114

228.172

374.278

317.877

586.371

440.265

742.462

742.456

440.261

586.369

228.172

143.113

374.275

317.874

270.192

515.293

129.102

### Synthetic peptide

Figure 23

Tumor sample

Synthetic peptide

Figure 24

Tumor sample

Synthetic peptide

Figure 25

Tumor sample

Synthetic peptide

Figure 26

Tumor sample

pre[+2]

173.130

201.125

189.125

526.255

851.379

876.438

722.339

1039.503

722.341

876.430

189.125

851.377

1039.497

201.123

526.252

173.128

Synthetic peptide

Figure 27

Tumor sample

Synthetic peptide

Figure 28

Tumor sample

Synthetic peptide

Figure 29

Figure 30

Figure 31

Figure 32

Figure 33

Figure 34

Figure 35

Figure 36

Figure 37

Figure 38

Figure 39

# Panel A

# Panel B

Figure 39 (cont.)

# Panel C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005099750 A
- WO 0006598 A
- WO 2006119527 A
- WO 2007137279 A
- WO 2006103562 A
- WO 2007109583 A
- US 2010136518 A

**Non-patent literature cited in the description**

- **RIBAS, A.** ; **WOLCHOK, J. D.** *Science*, 2018, vol. 359, 1350-1355
- **HURST** ; **MAGIORKINIS**. *J. Gen. Virol*, 2015, vol. 96, 1207-1218
- **KASSIOTIS** ; **STOYE**. *Nat. Rev. Immunol.*, 2016, vol. 16, 207-219
- **RUPRECHT**. *Cell Mol Life Sci*, 2008, vol. 65, 3366-3382
- **WANG-JOHANNING**. *Cancer*, 2003, vol. 98, 187-197
- **ANDERSSON et al.** *Int. J. Oncol*, 1998, vol. 12, 309-313
- **MANGENEY et al.** *J. Gen. Virol.*, 2001, vol. 82, 2515-2518
- **BABAIAN** ; **MAGER**. *Mob. DNA*, 2016
- **LOCK et al.** *PNAS*, 2014, vol. 111, 3534-3543
- **WOLD et al.** Adenovirus Vectors for Gene Therapy, Vaccination and Cancer Gene Therapy. *Current Gene Therapy*, 2013, vol. 13, 421-433
- **YOSSEF et al.** *JCI Insight*, 04 October 2018, vol. 3 (19), 122467
- **KERSHAW et al.** *Cancer Res.*, 2001, vol. 61, 7920-7924
- **SLANSKY et al.** *Immunity*, 2000, vol. 13, 529-538
- **SACHA et al.** *J.Immunol*, 2012, vol. 189, 1467-1479
- **HUMER J et al.** *Canc. Res*, 2006, vol. 66, 1658-63
- **FERRUCCI et al.** *Curr. Top. Med. Chem.*, 2012, vol. 12 (1), 11-31
- **BANNERT et al.** *Front. Microbiol*, 2018, vol. 9, 178
- **ESCUDIER et al.** *J. Transl. Med*, 2005, vol. 3, 10
- **YIN et al.** *Inflammation*, 2013, vol. 36 (1), 232-40
- **CREIGHTON**. *Proteins*, 1984
- **PAUL**. Fundamental Immunology. 1993, 243-247
- **BEIβBARTH et al.** *Bioinformatics*, 2005, vol. 21 (1), i29-i37
- **BATZER et al.** *Nucleic Acid Res.*, 1991, vol. 19, 5081
- **OHTSUKA et al.** *J. Biol. Chem.*, 1985, vol. 260, 2605-2608
- **ROSSOLINI et al.** *Mol. Cell. Probes*, 1994, vol. 8, 91-98
- **SMITH** ; **WATERMAN**. *Adv. Appl. Math*, 1981, vol. 2, 482
- **NEEDLEMAN** ; **WUNSCH**. *J. Mol. Biol.*, 1970, vol. 48, 443
- **PEARSON** ; **LIPMAN**. *Proc. Nat'l. Acad. Sci. USA*, 1988, vol. 85, 2444
- GAP, BESTFIT, FASTA, and TFASTA in the Wisconsin Genetics Software Package. *Genetics Computer Group*
- Current Protocols in Molecular Biology. 1995
- **FENG** ; **DOOLITTLE**. *J. Mol. Evol.*, 1987, vol. 35, 351-360
- **HIGGINS** ; **SHARP**. *CABIOS*, 1989, vol. 5, 151-153
- **DEVEREAUX et al.** *Nuc. Acids Res.*, 1984, vol. 12, 387-395
- **ALTSCHUL et al.** *Nuc. Acids Res.*, 1977, vol. 25, 3389-3402
- **ALTSCHUL et al.** *J. Mol. Biol.*, 1990, vol. 215, 403-410
- **HENIKOFF** ; **HENIKOFF**. *Proc. Natl. Acad. Sci. USA*, 1989, vol. 89, 10915
- **KARLIN** ; **ALTSCHUL**. *Proc. Nat'l. Acad. Sci. USA*, 1993, vol. 90, 5873-5787
- **GREEN** ; **SAMBROOK**. Molecular Cloning: A Laboratory Manual. Cold Spring Harbour Laboratory Press, 2012
- **NAMBIAR et al.** *Science*, 1984, vol. 223, 1299-1301
- **SAKAMAR** ; **KHORANA**. *Nucl. Acids Res.*, 1988, vol. 14, 6361-6372
- **WELLS et al.** *Gene*, 1985, vol. 34, 315-323

- **GRUNDSTROM et al.** *Nucl. Acids Res.*, 1985, vol. 13, 3305-3316
- **VERMA** ; **ECKSTEIN**. *Annu. Rev. Biochem.*, 1998, vol. 67, 99-134
- *Methods Mol Biol.*, 2012, vol. 834, 93-109
- **ROLLAND**. *Crit. Rev. Therap. Drug Carrier Systems*, 1998, vol. 15, 143-198
- **ULMER et al.** *Science*, 1993, vol. 259, 1745-1749
- **COHEN**. *Science*, 1993, vol. 259, 1691-1692
- **ULMER et al.** *Vaccine*, 2012, vol. 30, 4414-4418
- **GEALL et al.** *PNAS*, 2012, vol. 109, 14604-14609
- **SCHLAKE et al.** *RNA Biology*, vol. 9, 1319-1330
- **BRITO et al.** *Molecular Therapy*, 2014, vol. 22, 2118-2129
- **KRANZ et al.** *Nature*, 2006, vol. 534, 396-401
- Vaccine Design. 1995
- **LAUSS et al.** *Nature Communications*, 2017, vol. 8 (1), 1738, http://doi.org/10.1038/s41467-017-01460-0
- **SMART et al.** *Nature Biotechnology*, 2018, http://doi.org/10.1038/nbt.4239
- **HOYOS et al.** *Cancer Cell*, vol. 34 (2), 181-183, http://doi.org/10.1016/j.ccell.2018.07.008
- **KAHLES et al.** *Cancer Cell*, 2018, vol. 34 (2), 211-224, 6, http://doi.org/10.1016/j.ccell.2018.07.001
- **GIGOUX, M.** ; **WOLCHOK, J.** *JEM*, 2018, vol. 215, 2233
- **MARIJT et al.** *JEM*, 2018, vol. 215, 2325
- **KÖHLER** ; **MILSTEIN**. *Nature*, 1975, vol. 256 (5517), 495-497
- **NELSON et al.** *Mol Pathol.*, June 2000, vol. 53 (3), 111-7
- **WENDELL** ; **JUNE**. *Cell*, 2017, vol. 168, 724-740
- **MARCEL M.** *EMBnet J*, 2011, vol. 17, 3
- **CRUSOE et al.** *F1000Res.*, 2015, vol. 4, 900
- **TRINITY** ; **GRABHERR, M.G. et al.** *Nat. Biotechnol.*, 2011, vol. 29, 644-52
- **PATRO, R. et al.** *Nat. Methods*, 2017, vol. 14, 417-419
- **WU et al.** *Bioinf.*, 2005, vol. 21, 1859-1875
- **TRAPNELL et al.** *Nat. Biotech.*, 2010, vol. 28, 511-515
- **LYER et al.** *Nat. Genet.*, 2015, vol. 47, 199-208
- **ATTIG et al.** *Front. In Microbiol.*, 2017, vol. 8, 2489
- **WHEELER et al.** *Bioinform*, 2013, vol. 29, 2487-2489
- **HUBLEY et al.** *Nuc. Acid. Res*, 2016, vol. 44, 81-89
- The Genotype-Tissue Expression Consortium. *Science*, 2015, vol. 348, 648-60
- **FREUDENMANN et al.** *Immunology*, 2018, vol. 154 (3), 331-345
- **NESVIZHSKII et al.** *Nat. Methods*, 2014, vol. 11, 1114-1125
- **LIEPE et al.** *Science*, 2016, vol. 354 (6310), 354-358
- **LI et al.** *BMC Genomics*, 2016, vol. 17 (13), 1031
- **BASSANI-STERNBERG et al.** *Nature Commun*, 2016, vol. 7, 13404, https:/www.ebi.ac.uk/pride/archive/projects/PXD004894
- **TERNETTE et al.** *Proteomics*, 2018, vol. 18, 1700465
- **BASSANI-STERNBERG et al.** *Nature Commun*, 2016, vol. 7, 13404
- **STERNBERG et al.** *Nature Commun*, 2016, vol. 7, 13404, https://www.ebi.ac.uk/pride/archive/projects/PXD004894
- **BASSANI-STERNBERG et al.** *Nature Comm.*, 2016, vol. 7, 13404
- **ANAGNOSTOU et al.** *Cancer Discovery*, 2017
- **LE et al.** *Science*, 2017
- **WANG et al.** *J Mol Diagn.*, 2012, vol. 14 (1), 22-29